# EUROPEAN PATENT APPLICATION

(11) **EP 4 582 563 A2**
(43) Date of publication of application: **09.07.2025**
(21) Application number: 25169725.6
(22) Date of filing: 04.03.2021
(51) Int. Cl.: C12Q 1/70

(54) **COMPOSITIONS AND METHODS FOR DETECTING SARS-COV -2 NUCLEIC ACID**

(30) Priority: 04.03.2020 US 202062985139 P; 15.04.2020 US 202063010186 P; 04.05.2020 US 202063020007 P; 15.09.2020 US 202063078790 P
(62) Divisional of application: 21716873.1
(71) Applicant: Gen-Probe Incorporated, San Diego, CA 92121 (US)
(72) Inventor: SHAH, Ankur H., San Diego, 92131 (US); EATON, Barbara Lynn, San Diego, 92131 (US); GETMAN, Damon Kittredge, Poway, 92064 (US); MOBERLY, Joshua Kalani, Poway, 92064 (US); DARBY, Paul M., San Diego, 92126 (US); PHAM, Jimmykim, San Diego, 92117 (US)
(74) Representative: Script Intellectual Property LLP

(57) **Abstract**

There is described a composition or kit for determining the presence or absence of SARS-CoV-2 in a sample, said composition or kit comprising: (a) a first amplification oligomer combination comprising first and second SARS-CoV-2 region 1-specific amplification oligomers capable of amplifying a first target region of a SARS-CoV-2 target nucleic acid, (i) wherein the first SARS-CoV-2 region 1-specific amplification oligomer comprises a first SARS-CoV-2 region 1-specific target-hybridizing sequence that is from 18 to 27 contiguous nucleotides in length, is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and (ii) wherein the second SARS-CoV-2 region 1-specific amplification oligomer comprises a second SARS-CoV-2 region 1-specific target-hybridizing sequence that is from 18 to 23 contiguous nucleotides in length, is contained within SEQ ID NO:85, and contains a nucleotide sequence differing by no more than 0, 1, 2, 3, 4, or 5 nucleotides from the nucleotide sequence of SEQ ID NO:86, SEQ ID NO:87, or SEQ ID NO:88, or (b) a second amplification oligomer combination comprising first and second SARS-CoV-2 region 2-specific amplification oligomers capable of amplifying a second target region of a SARS-CoV-2 target nucleic acid, (i) wherein the first SARS-CoV-2 region 2-specific amplification oligomer comprises a first SARS-CoV-2 region 2-specific target-hybridizing sequence that is from 20 to 23 contiguous nucleotides in length, is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and (ii) wherein the second SARS-CoV-2 region 2-specific amplification oligomer comprises a second SARS-CoV-2 region 2-specific target-hybridizing sequence that is from 16 to 27 contiguous nucleotides in length, is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113.

## Description

### CROSS-REFERENCE TO RELATED APPLICATIONS

This application claims the benefit of U.S. Provisional Application No. 62/985,139, filed March 4, 2020, U.S. Provisional Application No. 63/010,186, filed April 15, 2020, U.S. Provisional Application No. 63/020,007, filed May 4, 2020, and U.S. Provisional Application No. 63/078,790, filed September 15, 2020, each of which is incorporated herein by reference.

### SEQUENCE LISTING

The Sequence Listing written in file DIA-0116-05_SeqList.txt is 65 kilobytes in size, was created March 4, 2021, and is hereby incorporated by reference.

### BACKGROUND

Coronaviruses are a family of RNA viruses that infect avians and mammals, including humans. Coronaviruses belong to the family Coronaviridae, which has four main sub-groupings, known as alphacoronavirus, betacoronavirus, gammacoronavirus, and deltacoronavirus. Human coronaviruses include alphacoronaviruses 229E and NL63 and betacoronaviruses OC43, HKU1, SARS-CoV (the coronavirus that causes severe acute respiratory syndrome, or SARS), SARS-CoV-2 (previously 2019-nCoV or Wuhan CoV), and MERS-CoV (the coronavirus that causes Middle East Respiratory Syndrome, or MERS).

Human coronaviruses cause approximately 10-15% of all upper and lower respiratory tract infections. They account for significant hospitalizations of children under 18 years of age, the elderly, and immunocompromised individuals. Four human coronaviruses (229E, HKU1, NL63, and OC43) are associated with a range of respiratory outcomes, including bronchiolitis and pneumonia (Gaunt et al., Journal of Clinical Microbiology 48:2940-2947, 2010). Up to 10% of acute respiratory diseases are caused by human coronavirus NL63 (NL63) (Abdul-Rasool and Fielding, The Open Virology Journal 4:76-84, 2010). An important aspect of NL63 infection is the co-infection with other human coronaviruses, influenza A, respiratory syncytial virus (RSV), parainfluenza virus human metapneumovirus (*see* id). In children, coronaviruses are associated with acute respiratory tract illness, pneumonia, and croup leading in many cases to hospitalization. In one epidemiological study, out of 1471 hospitalized children (<2 years), 207 (14%) were positive for human coronavirus (Dijkman et al., Journal of Clinical Virology 53:135-139, 2012). In a large-scale survey on 11,661 diagnostic respiratory samples collected in Edinburgh, UK, between 2006 and 2009, 267 (2.30%) were positive for at least one coronavirus, accounting for 8.15% of all virus detections (*see* Gaunt et al., supra). 11 to 41% of coronaviruses detected were present in samples tested positive for other respiratory viruses (*e.g.,* RSV) (*see* id).

The emerging SARS-CoV-2 can cause severe lower respiratory tract infections (COVID-19) and was declared a global emergency by the World Health Organization. SARS-CoV-2 is responsible for the recent pneumonia outbreak that started in early December 2019 in Wuhan City, Hubei Province, China (Huang et al., Lancet (2020) v395, issue 10223, p.497). To date, thousands of human infections have been confirmed in China along with many exported cases across the globe (China CDC, 2020), including in the United States, Canada, Japan, Australia and Europe.

Several commercial assays based on nucleic acids detection are available for detection of human coronavirus OC43, HKU1, NL63, and 229E in clinical specimens. These include the NxTAG^{®} Respiratory Pathogen Panel (Luminex), the BIOFIRE^{®} FILMARRAY^{®} Respiratory Panel (bioMérieux), and the ePlex^{®} Respiratory Pathogen Panel (GenMark Diagnostics). The U.S. Center for Disease Control has received Emergency Use Authorization for its 2019-nCoV Real-Time RT-PCR Diagnostic Panel.

### SUMMARY

In some aspects, the present invention provides compositions and/or kits for determining the presence or absence of SARS-CoV-2 in a sample. The compositions and/or kits generally includes at least one amplification oligomer combination comprising first and second SARS-CoV-2-specific amplification oligomers capable of amplifying a target region of a SARS-CoV-2 nucleic acid. The described oligonucleotides, compositions, formulations, kits, and methods can be used for isothermal amplification of target nucleic acids in SARS-CoV-2. The isothermal reaction can be, but is not limited to, transcription-mediated amplification (TMA).

In another aspect, the present invention provides formulations for amplifying SARS-CoV-2 nucleic acid in a sample. The formulations generally includes (i) at least one amplification oligomer combination comprising first and second SARS-CoV-2-specific amplification oligomers capable of amplifying a target region of a SARS-CoV-2 target nucleic acid, and (ii) a buffer. In some embodiments, the formulations are dried compositions. In some embodiments, the kits are described comprising such dried compositions for amplifying SARS-CoV-2 nucleic acid. In some embodiments, methods are described for preparing aqueous reaction mixtures for determining the presence or absence of SARS-CoV-2 in a sample, wherein the methods generally include the step of combining a described dried composition with an aqueous reconstitution reagent.

In some aspects, methods for determining the presence or absence of SARS-CoV-2 in a sample are described. In some embodiments, the methods comprise performing an *in vitro* nucleic acid amplification reaction utilizing at least one amplification oligomer combination comprising first and second SARS-CoV-2-specific amplification oligomers to generate one or more amplicons corresponding to at least one SARS-CoV-2 target region, and detecting the presence or absence of the one or more amplicons. In some embodiments, the methods are multiplex methods for determining the presence of SARS-CoV-2 and at least one other pathogen in a sample.

Representative embodiments of these aspects are further set forth below.

Embodiment 1. A composition or kit for determining the presence or absence of SARS-CoV-2 in a sample, said composition or kit comprising:
(a) a first amplification oligomer combination comprising first and second SARS-CoV-2 region 1-specific amplification oligomers capable of amplifying a first target region of a SARS-CoV-2 target nucleic acid,
   (i) wherein the first SARS-CoV-2 region 1-specific amplification oligomer comprises a first SARS-CoV-2 region 1-specific target-hybridizing sequence that is from 18 to 27 contiguous nucleotides in length, is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
   (ii) wherein the second SARS-CoV-2 region 1-specific amplification oligomer comprises a second SARS-CoV-2 region 1-specific target-hybridizing sequence that is from 18 to 23 contiguous nucleotides in length, is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88; and
(b) a second amplification oligomer combination comprising first and second SARS-CoV-2 region 2-specific amplification oligomers capable of amplifying a second target region of the SARS-CoV-2 target nucleic acid,
   (i) wherein the first SARS-CoV-2 region 2-specific amplification oligomer comprises a first SARS-CoV-2 region 2-specific target-hybridizing sequence that is from 20 to 23 contiguous nucleotides in length, is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
   (ii) wherein the second SARS-CoV-2 region 2-specific amplification oligomer comprises a second SARS-CoV-2 region 2-specific target-hybridizing sequence that is 16 to 27 contiguous nucleotides in length, is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113.

Embodiment 2. The composition or kit of embodiment 1, further comprising a first detection probe oligomer configured to hybridize to a target sequence contained within an amplicon amplifiable by the first amplification oligomer combination of (a), and wherein the first detection probe oligomer comprises a SARS-CoV-2-specific detection probe target-hybridizing sequence that is from 19 to 27 contiguous nucleotides in length.

Embodiment 3. The composition or kit of embodiment 1 or 2, further comprising a second detection probe oligomer configured to hybridize to a target sequence contained within an amplicon amplifiable by the second amplification oligomer combination of (b), and wherein the second detection probe oligomer comprises a SARS-CoV-2-specific detection probe target-hybridizing sequence that is from 25 to 29 contiguous nucleotides in length.

Embodiment 4. The composition or kit of embodiment 1, 2, or 3, wherein the first SARS-CoV-2 region 1-specific target-hybridizing sequence of (a)(i) contains or consists of a nucleotide sequence differing by no more than 0, 1, 2, 3, 4, or 5 nucleotides from the nucleotide sequence of SEQ ID NO:12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2 region 1-specific target-hybridizing sequence of (a)(ii) contains or consists of a nucleotide sequence differing by no more than 0, 1, 2, 3, 4, or 5 nucleotides from the nucleotide sequence of SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.

Embodiment 5. The composition or kit of embodiment 1, 2, 3, or 4, wherein the first SARS-CoV-2 region 2-specific target-hybridizing sequence of (b)(i) contains or consists of a nucleotide sequence differing by no more than 0, 1, 2, 3, 4, or 5 nucleotides from the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2 region 2-specific target-hybridizing sequence of (b)(ii) contains or consists of a nucleotide sequence differing by no more than 0, 1, 2, 3, 4, or 5 nucleotides from the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.

Embodiment 5.1. The composition or kit of embodiments 4 or 5, wherein the first SARS-CoV-2 region 1-specific target-hybridizing sequence of (a)(i) contains or consists of the nucleotide sequence of SEQ ID NO:12, SEQ ID NO:18, SEQ ID NO:19, SEQ ID NO:20, or SEQ ID NO:21, or an RNA equivalent or a DNA/RNA chimeric thereof and including from 0-7 nucleotide analogs; the second SARS-CoV-2 region 1-specific target-hybridizing sequence of (a)(ii) contains or consists of the nucleotide sequence of SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs; first SARS-CoV-2 region 2-specific target-hybridizing sequence of (b)(i) contains or consists of the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs; and/or the second SARS-CoV-2 region 2-specific target-hybridizing sequence of (b)(ii) contains or consists of the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.

Embodiment 6. The composition or kit of any one of embodiments 2, 4, or 5a, wherein the SARS-CoV-2-specific detection probe target-hybridizing sequence contains or consists of a nucleotide sequence differing by no more than 0, 1, 2, 3, 4, or 5 nucleotides from the nucleotide sequence of SEQ ID NO:14, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:107, or SEQ ID NO:108, including a DNA equivalent, an RNA equivalent, or a DNA/RNA chimeric thereof and including from 0-7 nucleotide analogs.

Embodiment 7. The composition or kit of any one of embodiments 3, 5 or 5a, wherein the SARS-CoV-2-specific detection probe target-hybridizing sequence contains or consists of a nucleotide sequence differing by no more than 0, 1, 2, 3, 4, or 5 nucleotides from the nucleotide sequence of SEQ ID NO:17, SEQ ID NO:43, SEQ ID NO:44, SEQ ID NO:45, SEQ ID NO:46, SEQ ID NO:47, SEQ ID NO:48, SEQ ID NO:109, or SEQ ID NO:110, including a DNA equivalent, an RNA equivalent, or a DNA/RNA chimeric thereof and including from 0-7 nucleotide analogs.

Embodiment 8. The composition or kit of any one of embodiments 2, 3, 6, and 7, wherein the first and/or second detection probe oligomer further comprises a detectable label.

Embodiment 9. The composition or kit of embodiment 8, wherein the detectable label is a fluorescent or chemiluminescent label.

Embodiment 10. The composition or kit of embodiment 9, wherein the detectable label is a fluorescent label and the first and/or second detection probe oligomer further comprises a non-fluorescent quencher.

Embodiment 11. The composition or kit of any one of embodiments 2, 3, 6, 7, 8, 9, and 10, wherein the first and/or second detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 12. The composition or kit of embodiment 11, wherein the self-complementary sequence is joined to the SARS-CoV-2-specific detection probe target-hybridizing sequence via a non-nucleotide linker.

Embodiment 13. The composition or kit of any one of embodiments 2-3 and 6-12, wherein the first and/or second detection probe oligomer is a molecular beacon or a molecular torch.

Embodiment 14. The composition or kit of any one of embodiments 1 to 13 wherein the second SARS-CoV-2-specific target-hybridizing sequence of (a)(ii) is joined at its 5' end to a promoter sequence.

Embodiment 15. The composition or kit of embodiment 14, wherein the promoter sequence is substantially identical to SEQ ID NO:11.

Embodiment 16. The composition or kit of embodiment 14, wherein the second SARS-CoV-2-specific amplification oligomer of (a)(ii) comprises or consists of a contiguous nucleotide sequence differing by no more than 0, 1, 2, 3, 4, or 5 nucleotides from the nucleotide sequence of SEQ ID NO:57, SEQ ID NO:58, SEQ ID NO:59, SEQ ID NO:60, SEQ ID NO:61, or SEQ ID NO:62, or an RNA equivalent or a DNA/RNA chimeric thereof and including from 0-7 nucleotide analogs.

Embodiment 17. The composition or kit of any one of embodiments 1 to 16, wherein the second SARS-CoV-2-specific target-hybridizing sequence of (b)(ii) is joined at its 5' end to a promoter sequence.

Embodiment 18. The composition or kit of embodiment 17, wherein the promoter sequence is substantially identical to SEQ ID NO:11.

Embodiment 19. The composition or kit of embodiment 17, wherein the second SARS-CoV-2-specific amplification oligomer of (b)(ii) comprises or consists of a contiguous nucleotide sequence differing by no more than 0, 1, 2, 3, 4, or 5 nucleotides from the nucleotide sequence of SEQ ID NO:63, SEQ ID NO:64, SEQ ID NO:65, SEQ ID NO:66, SEQ ID NO:67; SEQ ID NO:68; SEQ ID NO:69; SEQ ID NO:70, or SEQ ID NO:78, or an RNA equivalent or a DNA/RNA chimeric thereof and including from 0-7 nucleotide analogs.

Embodiment 20. A composition or kit for determining the presence or absence of SARS-CoV-2 in a sample, said composition or kit comprising an amplification oligomer combination comprising first and second SARS-CoV-2-specific amplification oligomers capable of amplifying a target region of a SARS-CoV-2 target nucleic acid,
(a) wherein the first SARS-CoV-2-specific amplification oligomer comprises a first SARS-CoV-2-specific target-hybridizing sequence that is from 18 to 27 contiguous nucleotides in length, is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
(b) wherein the second SARS-CoV-2-specific amplification oligomer comprises a second SARS-CoV-2-specific target-hybridizing sequence that is from 18 to 23 contiguous nucleotides in length, is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO: 88.

Embodiment 21. The composition or kit of embodiment 20, wherein the first SARS-CoV-2-specific target-hybridizing sequence contains or consists of a nucleotide sequence differing by no more than 0, 1, 2, 3, 4, or 5 nucleotides from the nucleotide sequence of SEQ ID NO:12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence contains or consists of a nucleotide sequence differing by no more than 0, 1, 2, 3, 4, or 5 nucleotides from the nucleotide sequence of SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.

Embodiment 22. The composition or kit of embodiment 20 or 21, wherein the second SARS-CoV-2-specific target-hybridizing sequence is joined at its 5' end to a promoter sequence.

Embodiment 23. The composition or kit of embodiment 22, wherein the promoter sequence is substantially identical to SEQ ID NO:11.

Embodiment 24. The composition or kit of embodiment 22, wherein the second SARS-CoV-2-specific amplification oligomer comprises a contiguous nucleotide sequence comprising or consisting of a nucleotide sequence differing by no more than 0, 1, 2, 3, 4, or 5 nucleotides from the nucleotide sequence of SEQ ID NO:57, SEQ ID NO:58, SEQ ID NO:59, SEQ ID NO:60, SEQ ID NO:61, or SEQ ID NO:62, or an RNA equivalent or a DNA/RNA chimeric thereof and including from 0-7 nucleotide analogs.

Embodiment 25. The composition or kit of any one of embodiments 20 to 24, further comprising a SARS-CoV-2-specific detection probe oligomer, wherein the detection probe oligomer comprises a SARS-CoV-2-specific detection probe target-hybridizing sequence that is from 19 to 27 contiguous nucleotides in length, is contained within SEQ ID NO:50, and contains a nucleotide sequence differing by no more than 0, 1, 2, 3, 4, or 5 nucleotides from the nucleotide sequence of SEQ ID NO:51, SEQ ID NO:52, SEQ ID NO:53, or SEQ ID NO:114.

Embodiment 26. The composition or kit of embodiment 25, wherein the SARS-CoV-2-specific detection probe target-hybridizing sequence contains or consists of a nucleotide sequence differing by no more than 0, 1, 2, 3, 4, or 5 nucleotides from the nucleotide sequence of SEQ ID NO:14, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:107, or SEQ ID NO:108, including a DNA equivalent, an RNA equivalent, or a DNA/RNA chimeric thereof and including from 0-7 nucleotide analogs.

Embodiment 27. The composition or kit of embodiment 25, wherein the SARS-CoV-2-specific detection probe oligomer target-hybridizing sequence contains or consists of a nucleotide sequence differing by no more than 0, 1, 2, 3, 4, or 5 nucleotides from the nucleotide sequence of SEQ ID NO:14, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:103, SEQ ID NO:104, SEQ ID NO:107, or SEQ ID NO:108, including a DNA equivalent, an RNA equivalent, or a DNA/RNA chimeric thereof and including from 0-7 nucleotide analogs.

Embodiment 28. The composition or kit of any one of embodiments 25 to 27, wherein the SARS-CoV-2-specific detection probe oligomer further comprises a detectable label.

Embodiment 29. The composition or kit of embodiment 28, wherein the detectable label is a fluorescent or chemiluminescent label.

Embodiment 30. The composition or kit of embodiment 29, wherein the detectable label is a fluorescent label and the SARS-CoV-2-specific detection probe oligomer further comprises a non-fluorescent quencher.

Embodiment 31. The composition or kit of any one of embodiments 25, 26, and 28 to 30, wherein the SARS-CoV-2-specific detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 32. The composition or kit of embodiment 31, wherein the self-complementary sequence is joined to the SARS-CoV-2-specific detection probe target-hybridizing sequence via a non-nucleotide linker.

Embodiment 33. The composition or kit of any one of embodiments 26, and 28 to 32, wherein the SARS-CoV-2-specific detection probe oligomer is a molecular beacon or a molecular torch.

Embodiment 34. A composition or kit for determining the presence or absence of SARS-CoV-2 in a sample, said composition or kit comprising an amplification oligomer combination comprising first and second SARS-CoV-2-specific amplification oligomers capable of amplifying a target region of a SARS-CoV-2 target nucleic acid,
(a) wherein the first SARS-CoV-2-specific amplification oligomer comprises a first SARS-CoV-2-specific target-hybridizing sequence that is from 20 to 23 contiguous nucleotides in length, is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
(b) wherein the second SARS-CoV-2-specific amplification oligomer comprises a second SARS-CoV-2-specific target-hybridizing sequence that is 16 to 27 contiguous nucleotides in length, is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113.

Embodiment 35. The composition or kit of embodiment 34, wherein the first SARS-CoV-2-specific target-hybridizing sequence contains or consists of a nucleotide sequence differing by no more than 0, 1, 2, 3, 4, or 5 nucleotides from the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence contains or consists of a nucleotide sequence differing by no more than 0, 1, 2, 3, 4, or 5 nucleotides from the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.

Embodiment 36. The composition or kit of embodiment 34 or 35, wherein the second SARS-CoV-2-specific target-hybridizing sequence is joined at its 5' end to a promoter sequence.

Embodiment 37. The composition or kit of embodiment 36, wherein the promoter sequence is substantially identical to SEQ ID NO:11.

Embodiment 38. The composition or kit of embodiment 36, wherein the second SARS-CoV-2-specific amplification oligomer comprises or consists of a contiguous nucleotide sequence differing by no more than 0, 1, 2, 3, 4, or 5 nucleotides from the nucleotide sequence of SEQ ID NO: 63, SEQ ID NO:64, SEQ ID NO:65, SEQ ID NO:66, SEQ ID NO:67; SEQ ID NO:68; SEQ ID NO:69; SEQ ID NO:70, or SEQ ID NO:78, or an RNA equivalent or a DNA/RNA chimeric thereof and including from 0-7 nucleotide analogs.

Embodiment 39. The composition or kit of any one of embodiments 34 to 38, further comprising a SARS-CoV-2-specific detection probe oligomer, wherein the detection probe oligomer comprises a SARS-CoV-2-specific detection probe target-hybridizing sequence that is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO:115.

Embodiment 40. The composition or kit of embodiment 39, wherein the SARS-CoV-2-specific detection probe target-hybridizing sequence contains or consists of a nucleotide sequence differing by no more than 0, 1, 2, 3, 4, or 5 nucleotides from the nucleotide sequence of SEQ ID NO:17, SEQ ID NO:43, SEQ ID NO:44, SEQ ID NO:45, SEQ ID NO:46, SEQ ID NO:47, SEQ ID NO:48, SEQ ID NO:109 or SEQ ID NO:110, including a DNA equivalent, an RNA equivalent, or a DNA/RNA chimeric thereof and including from 0-7 nucleotide analogs.

Embodiment 41. The composition or kit of embodiment 39, wherein the second detection probe target-hybridizing sequence contains or consists of a nucleotide sequence differing by no more than 0, 1, 2, 3, 4, or 5 nucleotides from the nucleotide sequence of SEQ ID NO:17, SEQ ID NO:43, SEQ ID NO:44, SEQ ID NO:45, SEQ ID NO:46, SEQ ID NO:47, SEQ ID NO:48, SEQ ID NO:105, SEQ ID NO:106, SEQ ID NO:109 or SEQ ID NO:110, including a DNA equivalent, an RNA equivalent, or a DNA/RNA chimeric thereof and including from 0-7 nucleotide analogs.

Embodiment 42. The composition or kit of any one of embodiments 39 to 41, wherein the SARS-CoV-2-specific detection probe oligomer further comprises a detectable label.

Embodiment 43. The composition or kit of embodiment 42, wherein the detectable label is a fluorescent or chemiluminescent label.

Embodiment 44. The composition or kit of embodiment 43, wherein the detectable label is a fluorescent label and the SARS-CoV-2-specific detection probe oligomer further comprises a non-fluorescent quencher.

Embodiment 45. The composition or kit of any one of embodiments 39, 40, and 42 to 44, wherein the SARS-CoV-2-specific detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 46. The composition or kit of embodiment 45, wherein the self-complementary sequence is joined to the SARS-CoV-2-specific detection probe target-hybridizing sequence via a non-nucleotide linker.

Embodiment 47. The composition or kit of any one of embodiments 40 and 42 to 46, wherein the SARS-CoV-2-specific detection probe oligomer is a molecular beacon or a molecular torch.

Embodiment 48. A composition or kit comprising one or more SARS-CoV-2-specific-detection probe oligomers selected from the group consisting of: (a) a first detection probe oligomer comprising a SARS-CoV-2-specific detection probe target-hybridizing sequence that is from 19 to 27 contiguous nucleotides in length, is contained within SEQ ID NO:50, and contains a nucleotide sequence differing by no more than 0, 1, 2, 3, 4, or 5 nucleotides from the nucleotide sequence of SEQ ID NO:51, SEQ ID NO:52, SEQ ID NO:53, or SEQ ID NO:114; and (b) a second detection probe oligomer comprising a SARS-CoV-2-specific detection probe target-hybridizing sequence that is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO:115.

Embodiment 49. The composition or kit of embodiment 48, wherein the first and/or second detection probe oligomer comprises a SARS-CoV-2-specific detection probe target hybridizing sequence differing by no more than 0, 1, 2, 3, 4, or 5 nucleotides from the nucleotide sequence of SEQ ID NO:14, SEQ ID NO:17, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:44, SEQ ID NO:45, SEQ ID NO:46, SEQ ID NO:47, SEQ ID NO:48, SEQ ID NO:107, SEQ ID NO: 108, SEQ ID NO:109, or SEQ ID NO:110, including a DNA equivalent, an RNA equivalent, or a DNA/RNA chimeric thereof and including from 0-7 nucleotide analogs.

Embodiment 50. The composition or kit of embodiment 48, wherein the one or more SARS-CoV-2-specific detection probe oligomers independently comprises a sequence selected from the group consisting of: SEQ ID NO:14, SEQ ID NO: 17, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:44, SEQ ID NO:45, SEQ ID NO:46, SEQ ID NO:47, SEQ ID NO:48, SEQ ID NO:103, SEQ ID NO:104, SEQ ID NO:105, SEQ ID NO:106, SEQ ID NO:107, SEQ ID NO:108, SEQ ID NO:109 and SEQ ID NO:110, including a DNA equivalent, an RNA equivalent, or a DNA/RNA chimeric thereof and including from 0-7 nucleotide analogs.

Embodiment 51. The composition or kit of embodiment 48 or 49, wherein the SARS-CoV-2-specific detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 52. The composition or kit of embodiment 51, wherein the self-complementary sequence is joined to the SARS-CoV-2-specific detection probe target-hybridizing sequence via a non-nucleotide linker.

Embodiment 53. The composition or kit of any one of embodiments 48 to 52, wherein the SARS-CoV-2-specific detection probe oligomer further comprises a detectable label.

Embodiment 54. The composition or kit of embodiment 54, wherein the detectable label is a fluorescent or chemiluminescent label.

Embodiment 55. The composition or kit of embodiment 55, wherein the detectable label is a fluorescent label and the SARS-CoV-2-specific detection probe oligomer further comprises a non-fluorescent quencher.

Embodiment 56. The composition or kit of any one of embodiments 48-49 and 51-55, wherein the SARS-CoV-2-specific detection probe oligomer is a molecular beacon or a molecular torch.

Embodiment 57. A formulation for amplifying a SARS-CoV-2 nucleic acid in a sample, the formulation comprising:
(a) a first amplification oligomer combination comprising first and second SARS-CoV-2 region 1-specific amplification oligomers capable of amplifying a first target region of a SARS-CoV-2 target nucleic acid,
   (i) wherein the first SARS-CoV-2 region 1-specific amplification oligomer comprises a first SARS-CoV-2 region 1-specific target-hybridizing sequence that is from 18 to 27 contiguous nucleotides in length, is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
   (ii) wherein the second SARS-CoV-2 region 1-specific amplification oligomer comprises a second SARS-CoV-2 region 1-specific target-hybridizing sequence that is from 18 to 23 contiguous nucleotides in length, is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88;
(b) a second amplification oligomer combination comprising first and second SARS-CoV-2 region 2-specific amplification oligomers capable of amplifying a second target region of a SARS-CoV-2 target nucleic acid,
   (i) wherein the first SARS-CoV-2 region 2-specific amplification oligomer comprises a first SARS-CoV-2 region 2-specific target-hybridizing sequence that is from 20 to 23 contiguous nucleotides in length, is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
   (ii) wherein the second SARS-CoV-2 region 2-specific amplification oligomer comprises a second SARS-CoV-2 region 2-specific target-hybridizing sequence that is 16 to 27 contiguous nucleotides in length, is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113; and
(c) a buffer.

Embodiment 58. The formulation of embodiment 57, wherein the first SARS-CoV-2 region 1-specific target-hybridizing sequence of (a)(i) contains or consists of a nucleotide sequence differing by no more than 0, 1, 2, 3, 4, or 5 nucleotides from the nucleotide sequence of SEQ ID NO:12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2 region 1-specific target-hybridizing sequence of (a)(ii) contains or consists of a nucleotide sequence differing by no more than 0, 1, 2, 3, 4, or 5 nucleotides from the nucleotide sequence of SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.

Embodiment 59. The formulation of embodiment 57 or 58, wherein the first SARS-CoV-2 region 2-specific target-hybridizing sequence of (b)(i) contains or consists of a nucleotide sequence differing by no more than 0, 1, 2, 3, 4, or 5 nucleotides from the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2 region 2-specific target-hybridizing sequence of (b)(ii) contains or consists of a nucleotide sequence differing by no more than 0, 1, 2, 3, 4, or 5 nucleotides from the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.

Embodiment 60. The formulation of embodiment 57 or 58, wherein the second SARS-CoV-2-specific target-hybridizing sequence of (a)(ii) is joined at its 5' end to a promoter sequence.

Embodiment 61. The formulation of embodiment 60, wherein the promoter sequence is substantially identical to SEQ ID NO:11.

Embodiment 62. The formulation of embodiment 61, wherein the second SARS-CoV-2-specific amplification oligomer of (a)(ii) comprises a contiguous nucleotide sequence containing or consisting of a nucleotide sequence differing by no more than 0, 1, 2, 3, 4, or 5 nucleotides from the nucleotide sequence of SEQ ID NO:57, SEQ ID NO:58, SEQ ID NO:59, SEQ ID NO:60, SEQ ID NO:61, or SEQ ID NO:62, or an RNA equivalent or a DNA/RNA chimeric thereof and including from 0-7 nucleotide analogs.

Embodiment 63. The formulation of any one of embodiments 57-62, wherein the second SARS-CoV-2-specific target-hybridizing sequence of (b)(ii) is joined at its 5' end to a promoter sequence.

Embodiment 64. The formulation of embodiment 63, wherein the promoter sequence is substantially identical to SEQ ID NO:11.

Embodiment 65. The formulation of embodiment 64, wherein the second SARS-CoV-2-specific amplification oligomer of (b)(ii) comprises or consists of a contiguous nucleotide sequence differing by no more than 0, 1, 2, 3, 4, or 5 nucleotides from the nucleotide sequence of f SEQ ID NO:63, SEQ ID NO:64, SEQ ID NO:65, SEQ ID NO:66, SEQ ID NO:67; SEQ ID NO:68; SEQ ID NO:69; SEQ ID NO:70, or SEQ ID NO:78, or an RNA equivalent or a DNA/RNA chimeric thereof and including from 0-7 nucleotide analogs.

Embodiment 66. The formulation of any one of embodiments 57 to 65, wherein the formulation further comprises a first detection probe oligomer configured to hybridize to a target sequence contained within an amplicon amplifiable by the first amplification oligomer combination of (a), and wherein the first detection probe oligomer comprises a SARS-CoV-2-specific detection probe target-hybridizing sequence that is from 19 to 27 contiguous nucleotides in length.

Embodiment 67. The formulation of any one of embodiments 57 to 66, wherein the formulation further comprises a second detection probe oligomer configured to hybridize to a target sequence contained within an amplicon amplifiable by the second amplification oligomer combination of (b), and wherein the detection probe oligomer comprises a SARS-CoV-2-specific detection probe target-hybridizing sequence that is from 25 to 29 contiguous nucleotides in length.

Embodiment 68. The formulation of embodiment 66, wherein the SARS-CoV-2-specific detection probe target-hybridizing sequence contains or consists of a nucleotide sequence differing by no more than 0, 1, 2, 3, 4, or 5 nucleotides from the nucleotide sequence of SEQ ID NO:14, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:107, or SEQ ID NO:108, including a DNA equivalent, an RNA equivalent, or a DNA/RNA chimeric thereof and including from 0-7 nucleotide analogs.

Embodiment 69. The formulation of embodiment 68, wherein the first detection probe oligomer target-hybridizing sequence contains or consists of a nucleotide sequence differing by no more than 0, 1, 2, 3, 4, or 5 nucleotides from the nucleotide sequence of SEQ ID NO:14, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:103, SEQ ID NO:104, SEQ ID NO:107, or SEQ ID NO:108, including a DNA equivalent, an RNA equivalent, or a DNA/RNA chimeric thereof and including from 0-7 nucleotide analogs.

Embodiment 70. The formulation of embodiment 67, wherein the SARS-CoV-2-specific detection probe target-hybridizing sequence contains or consists of a nucleotide sequence differing by no more than 0, 1, 2, 3, 4, or 5 nucleotides from the nucleotide sequence of SEQ ID NO:17, SEQ ID NO:43, SEQ ID NO:44, SEQ ID NO:45, SEQ ID NO:46, SEQ ID NO:47, SEQ ID NO:48, SEQ ID NO:109 or SEQ ID NO:110, including a DNA equivalent, an RNA equivalent, or a DNA/RNA chimeric thereof and including from 0-7 nucleotide analogs.

Embodiment 71. The formulation of embodiment 70, wherein the second detection probe oligomer contains or consists of a nucleotide sequence differing by no more than 0, 1, 2, 3, 4, or 5 nucleotides from the nucleotide sequence of SEQ ID NO: 17, SEQ ID NO:43, SEQ ID NO:44, SEQ ID NO:45, SEQ ID NO:46, SEQ ID NO:47, SEQ ID NO:48, SEQ ID NO:105, SEQ ID NO:106, SEQ ID NO:109, or SEQ ID NO:110, including a DNA equivalent, an RNA equivalent, or a DNA/RNA chimeric thereof and including from 0-7 nucleotide analogs.

Embodiment 72. The formulation of any one of embodiments 66 to 71, wherein the SARS-CoV-2-specific detection probe oligomer further comprises a detectable label.

Embodiment 73. The formulation of embodiment 72, wherein the detectable label is a fluorescent or chemiluminescent label.

Embodiment 74. The formulation of embodiment 73, wherein the detectable label is a fluorescent label and the SARS-CoV-2-specific detection probe oligomer further comprises a non-fluorescent quencher.

Embodiment 75. The formulation of any one of embodiments 57 to 74, wherein the formulation further comprises one or more of, a bulking agent, a surfactant, a nucleotide triphosphate, an enzyme, and an inorganic salt.

Embodiment 76. The formulation of any one of embodiments 57 to 75, wherein the formulation is a dried composition.

Embodiment 77. A formulation for amplifying a SARS-CoV-2 nucleic acid in a sample, the formulation comprising:
(a) an amplification oligomer combination comprising first and second SARS-CoV-2-specific amplification oligomers capable of amplifying a target region of a SARS-CoV-2 target nucleic acid, wherein the first SARS-CoV-2-specific amplification oligomer comprises a first SARS-CoV-2-specific target-hybridizing sequence that is from 18 to 27 contiguous nucleotides in length, is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and wherein the second SARS-CoV-2-specific amplification oligomer comprises a second SARS-CoV-2-specific target-hybridizing sequence that is from 18 to 23 contiguous nucleotides in length, is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88; and
(b) a buffer.

Embodiment 78. The formulation of embodiment 77, wherein the first SARS-CoV-2-specific target-hybridizing sequence contains or consists of a nucleotide sequence differing by no more than 0, 1, 2, 3, 4, or 5 nucleotides from the nucleotide sequence of SEQ ID NO:12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence contains or consists of a nucleotide sequence differing by no more than 0, 1, 2, 3, 4, or 5 nucleotides from the nucleotide sequence of SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.

Embodiment 79. The formulation of embodiment 77 or 78, wherein the second SARS-CoV-2-specific target-hybridizing sequence is joined at its 5' end to a promoter sequence.

Embodiment 80. The formulation of embodiment 79, wherein the promoter sequence is substantially identical to SEQ ID NO:11.

Embodiment 81. The formulation of embodiment 79, wherein the second SARS-CoV-2-specific amplification oligomer comprises or consists of a contiguous nucleotide sequence differing by no more than 0, 1, 2, 3, 4, or 5 nucleotides from the nucleotide sequence of SEQ ID NO:57, SEQ ID NO:58, SEQ ID NO:59, SEQ ID NO:60, SEQ ID NO:61, or SEQ ID NO:62, or an RNA equivalent or a DNA/RNA chimeric thereof and including from 0-7 nucleotide analogs.

Embodiment 82. The formulation of any one of embodiments 77 to 81, further comprising a SARS-CoV-2-specific detection probe oligomer, wherein the detection probe oligomer comprises a SARS-CoV-2-specific detection probe target-hybridizing sequence that is from 19 to 27 contiguous nucleotides in length, is contained within SEQ ID NO:50, and contains a nucleotide sequence differing by no more than 0, 1, 2, 3, 4, or 5 nucleotides from the nucleotide sequence of SEQ ID NO:51, SEQ ID NO:52, SEQ ID NO:53, or SEQ ID NO:114.

Embodiment 83. The formulation of embodiment 82, wherein the SARS-CoV-2-specific detection probe target-hybridizing sequence contains or consists of a nucleotide sequence differing by no more than 0, 1, 2, 3, 4, or 5 nucleotides from the nucleotide sequence of SEQ ID NO:14, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:107, or SEQ ID NO:108, including a DNA equivalent, an RNA equivalent, or a DNA/RNA chimeric thereof and including from 0-7 nucleotide analogs.

Embodiment 83a. The formulation of embodiment 83, wherein the SARS-CoV-2-specific detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 84. The formulation of embodiment 82, wherein the SARS-CoV-2-specific detection probe oligomer target-hybridizing sequence contains or consists of a nucleotide sequence differing by no more than 0, 1, 2, 3, 4, or 5 nucleotides from the nucleotide sequence of SEQ ID NO:14, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:103, SEQ ID NO:104, SEQ ID NO:107, or SEQ ID NO:108, including a DNA equivalent, an RNA equivalent, or a DNA/RNA chimeric thereof and including from 0-7 nucleotide analogs.

Embodiment 85. The formulation of any one of embodiments 82 to 84, wherein the SARS-CoV-2-specific detection probe oligomer further comprises a detectable label.

Embodiment 86. The formulation of embodiment 85, wherein the detectable label is a fluorescent or chemiluminescent label.

Embodiment 87. The formulation of embodiment 86, wherein the detectable label is a fluorescent label and the SARS-CoV-2-specific detection probe oligomer further comprises a non-fluorescent quencher.

Embodiment 88. The formulation of any one of embodiments 77 to 87, wherein the formulation further comprises one or more of, a bulking agent, a surfactant, a nucleotide triphosphate, an enzyme, and an inorganic salt.

Embodiment 89. The formulation of any one of embodiments 77 to 88, wherein the formulation is a dried composition.

Embodiment 90. A formulation for amplifying a SARS-CoV-2 nucleic acid in a sample, the formulation comprising:
(a) an amplification oligomer combination comprising first and second SARS-CoV-2-specific amplification oligomers capable of amplifying a target region of a SARS-CoV-2 target nucleic acid, wherein the first SARS-CoV-2-specific amplification oligomer comprises a first SARS-CoV-2-specific target-hybridizing sequence that is from 20 to 23 contiguous nucleotides in length, is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and wherein the second SARS-CoV-2-specific amplification oligomer comprises a second SARS-CoV-2-specific target-hybridizing sequence that is from 16 to 27 contiguous nucleotides in length, is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113; and
(b) a buffer.

Embodiment 91. The formulation of embodiment 90, wherein the first SARS-CoV-2-specific target-hybridizing sequence contains or consists of a contiguous nucleotide sequence differing by no more than 0, 1, 2, 3, 4, or 5 nucleotides from the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence contains or consists of a contiguous nucleotide sequence differing by no more than 0, 1, 2, 3, 4, or 5 nucleotides from the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.

Embodiment 92. The formulation of embodiment 90 or 91, wherein the second SARS-CoV-2-specific target-hybridizing sequence is joined at its 5' end to a promoter sequence.

Embodiment 93. The formulation of embodiment 92, wherein the promoter sequence is substantially identical to SEQ ID NO:11.

Embodiment 94. The formulation of embodiment 92, wherein the second SARS-CoV-2-specific amplification oligomer comprises or consists of a contiguous nucleotide sequence differing by no more than 0, 1, 2, 3, 4, or 5 nucleotides from the nucleotide sequence of SEQ ID NO: 63, SEQ ID NO:64, SEQ ID NO:65, SEQ ID NO:66, SEQ ID NO:67; SEQ ID NO:68; SEQ ID NO:69; SEQ ID NO:70, or SEQ ID NO:78, or an RNA equivalent or a DNA/RNA chimeric thereof and including from 0-7 nucleotide analogs.

Embodiment 95. The formulation of any one of embodiments 90 to 94, further comprising a SARS-CoV-2-specific detection probe oligomer, wherein the detection probe oligomer comprises a SARS-CoV-2-specific detection probe target-hybridizing sequence that is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO:115.

Embodiment 96. The formulation of embodiment 95, wherein the SARS-CoV-2-specific detection probe target-hybridizing sequence contains or consists of a contiguous nucleotide sequence differing by no more than 0, 1, 2, 3, 4, or 5 nucleotides from the nucleotide sequence of SEQ ID NO:17, SEQ ID NO:43, SEQ ID NO:44, SEQ ID NO:45, SEQ ID NO:46, SEQ ID NO:47, SEQ ID NO:48, SEQ ID NO:109 or SEQ ID NO:110, including a DNA equivalent, an RNA equivalent, or a DNA/RNA chimeric thereof and including from 0-7 nucleotide analogs.

Embodiment 96. The formulation of embodiment 96, wherein the SARS-CoV-2-specific detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 97. The formulation of embodiment 95, wherein the SARS-CoV-2-specific detection probe oligomer contains or consists of a nucleotide sequence differing by no more than 0, 1, 2, 3, 4, or 5 nucleotides from the nucleotide sequence of SEQ ID NO:17, SEQ ID NO:43, SEQ ID NO:44, SEQ ID NO:45, SEQ ID NO:46, SEQ ID NO:47, SEQ ID NO:48, SEQ ID NO:105, SEQ ID NO:106, SEQ ID NO:109, or SEQ ID NO:110, including a DNA equivalent, an RNA equivalent, or a DNA/RNA chimeric thereof and including from 0-7 nucleotide analogs.

Embodiment 98. The formulation of any one of embodiments 95 to 97, wherein the SARS-CoV-2-specific detection probe oligomer further comprises a detectable label.

Embodiment 99. The formulation of embodiment 98, wherein the detectable label is a fluorescent or chemiluminescent label.

Embodiment 100. The formulation of embodiment 99, wherein the detectable label is a fluorescent label and the SARS-CoV-2-specific detection probe oligomer further comprises a non-fluorescent quencher.

Embodiment 101. The formulation of any one of embodiments 90 to 100, wherein the formulation further comprises one or more of, a bulking agent, a surfactant, a nucleotide triphosphate, an enzyme, and an inorganic salt.

Embodiment 102. The formulation of any one of embodiments 90 to 101, wherein the formulation is a dried composition.

Embodiment 103. A formulation for detecting SARS-CoV-2 in a sample, the formulation comprising:
(a) a buffer; and
(b) one or more SARS-CoV-2-specific-detection probe oligomers selected from the group consisting of: (i) a first detection probe oligomer comprising a SARS-CoV-2-specific detection probe target-hybridizing sequence that is from 19 to 27 contiguous nucleotides in length, is contained within SEQ ID NO:50, and contains a nucleotide sequence differing by no more than 0, 1, 2, 3, 4, or 5 nucleotides from the nucleotide sequence of SEQ ID NO:51, SEQ ID NO:52, SEQ ID NO:53, or SEQ ID NO:114, and (ii) a first detection probe oligomer comprising a SARS-CoV-2-specific detection probe target-hybridizing sequence that is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO:115.

Embodiment 104. The formulation of embodiment 103, wherein the first and/or second detection probe oligomer comprises a SARS-CoV-2-specific detection probe target hybridizing sequence differs by no more than 0, 1, 2, 3, 4, or 5 nucleotides from the nucleotide sequence of SEQ ID NO:14, SEQ ID NO: 17, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:44, SEQ ID NO:45, SEQ ID NO:46, SEQ ID NO:47, SEQ ID NO:48, SEQ ID NO:107, SEQ ID NO:108, SEQ ID NO:109, or SEQ ID NO:110, including a DNA equivalent, an RNA equivalent, or a DNA/RNA chimeric thereof and including from 0-7 nucleotide analogs.

Embodiment 105. The formulation of embodiment 103, wherein the at least one SARS-CoV-2-specific detection probe oligomer target-hybridizing sequence contains or consists of a nucleotide sequence differing by no more than 0, 1, 2, 3, 4, or 5 nucleotides from the nucleotide sequence of SEQ ID NO: 14, SEQ ID NO:17, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:44, SEQ ID NO:45, SEQ ID NO:46, SEQ ID NO:47, SEQ ID NO:48, SEQ ID NO:103, SEQ ID NO:104, SEQ ID NO:105, SEQ ID NO:106, SEQ ID NO:107, SEQ ID NO:108, SEQ ID NO:109, or SEQ ID NO:110, including a DNA equivalent, an RNA equivalent, or a DNA/RNA chimeric thereof and including from 0-7 nucleotide analogs.

Embodiment 106. The formulation of embodiment 103 or 104, wherein the SARS-CoV-2-specific detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 107. The formulation of embodiment 106, wherein the self-complementary sequence is joined to the SARS-CoV-2-specific detection probe target-hybridizing sequence via a non-nucleotide linker.

Embodiment 108. The formulation of any one of embodiments 103 to 107, wherein the SARS-CoV-2-specific detection probe oligomer further comprises a detectable label.

Embodiment 109. The formulation of embodiment 108, wherein the detectable label is a fluorescent or chemiluminescent label.

Embodiment 110. The formulation of embodiment 109, wherein the detectable label is a fluorescent label and the SARS-CoV-2-specific detection probe oligomer further comprises a non-fluorescent quencher.

Embodiment 111. The formulation of any one of embodiments 103 to 110, wherein the formulation further comprises one or more of, a bulking agent, a surfactant, and an inorganic salt.

Embodiment 112. The formulation of any one of embodiments 103 to 111, wherein the formulation is a dried composition.

Embodiment 113. A kit comprising a dried composition as described in any one of embodiments 76, 89, 102 and 112.

Embodiment 114. The kit of embodiment 114, further comprising a reconstitution reagent, wherein the dried composition is in a first vial within the kit and the reconstitution reagent is in a second vial within the kit.

Embodiment 115. The kit of embodiment 113 or 114, wherein the dried composition comprises a bulking agent selected from the group consisting of trehalose, raffinose, mannitol, and a combination thereof.

Embodiment 116. The kit of any one of embodiments 113 to 115, wherein the dried composition comprises a non-linear surfactant.

Embodiment 117. The kit of embodiment 116, wherein the non-linear surfactant is polyoxyethylene sorbitan fatty acid ester or digitonin.

Embodiment 118. The kit of any one of embodiments 113 to 117, wherein the reconstitution reagent comprises one of more of: magnesium chloride, potassium chloride, and ethyl alcohol.

Embodiment 119. A method for preparing an aqueous reaction mixture for determining the presence or absence of a SARS-CoV-2 in a sample, the method comprising the step of combining a dried composition as described in any one of embodiments 76, 89, 102 or 112 with a reconstitution reagent to make an aqueous reaction mixture.

Embodiment 120. The method of embodiment 119, wherein the reconstitution reagent comprises one of more of magnesium chloride, potassium chloride, and ethyl alcohol.

Embodiment 121. The method of embodiment 119, wherein the combining step is performed using an automated system comprising a first holder for the dried composition, a second holder for the reconstitution reagent, a programmable controller and a pipetting device in communication with the programmable controller, and wherein the programmable controller is configured by software instructions to cause the pipetting device to transfer an aliquot of the reconstitution reagent from the second holder location to the first holder location thereby making the aqueous reaction mixture.

Embodiment 122. A method for determining the presence or absence of SARS-CoV-2 in a sample, the method comprising:
(a) contacting a sample containing or suspected of containing a SARS-CoV-2 target nucleic acid with at least a first amplification oligomer combination and a second amplification oligomer combination, wherein;
   (i) the first amplification oligomer combination comprises first and second SARS-CoV-2 region 1-specific amplification oligomers capable of amplifying a first target region of the SARS-CoV-2 target nucleic acid,
      (1) wherein the first SARS-CoV-2 region 1-specific amplification oligomer comprises a first SARS-CoV-2- region 1specific target-hybridizing sequence that is from 18 to 27 contiguous nucleotides in length, is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
      (2) wherein the second SARS-CoV-2 region 1-specific amplification oligomer comprises a second SARS-CoV-2 region 1-specific target-hybridizing sequence that is from 18 to 23 contiguous nucleotides in length, is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88; and
   (ii) the second amplification oligomer combination comprises first and second SARS-CoV-2 region 2-specific amplification oligomers capable of amplifying a second target region of the SARS-CoV-2 target nucleic acid,
      (1) wherein the first SARS-CoV-2 region 2-specific amplification oligomer comprises a first SARS-CoV-2 region 2-specific target-hybridizing sequence that is from 20 to 23 contiguous nucleotides in length, is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
      (2) wherein the second SARS-CoV-2 region 2-specific amplification oligomer comprises a second SARS-CoV-2 region 2-specific target-hybridizing sequence that is 16 to 27 contiguous nucleotides in length, is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113;
(b) incubating the sample in conditions suitable for nucleic acid amplification, wherein any SARS-CoV-2 target nucleic acid, if present in the sample, is used as a template for generating one or more amplicons corresponding to the first and/or second target regions of the SARS-CoV-2 target nucleic acid; and
(c) detecting the presence or absence of the one or more amplicons, thereby determining the presence or absence of SARS-CoV-2 in the sample.

Embodiment 123. The method of embodiment 122, wherein the first SARS-CoV-2-specific target-hybridizing sequence of (a)(i)(1) contains or consists of a contiguous nucleotide sequence differing by no more than 0, 1, 2, 3, 4, or 5 nucleotides from the nucleotide sequence of SEQ ID NO:12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence of (a)(i)(2) contains or consists of a contiguous nucleotide sequence differing by no more than 0, 1, 2, 3, 4, or 5 nucleotides from the nucleotide sequence of SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.

Embodiment 124. The method of embodiment 122 or 123, wherein the first SARS-CoV-2-specific target-hybridizing sequence of (a)(ii)(1) contains or consists of a contiguous nucleotide sequence differing by no more than 0, 1, 2, 3, 4, or 5 nucleotides from the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence of (a)(ii)(2) contains or consists of a contiguous nucleotide sequence differing by no more than 0, 1, 2, 3, 4, or 5 nucleotides from the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.

Embodiment 125. The method of any one of embodiments 122 to 124 wherein the second SARS-CoV-2-specific target-hybridizing sequence of (a)(i)(2) is joined at its 5' end to a promoter sequence.

Embodiment 126. The method of embodiment 125, wherein the promoter sequence is substantially identical to SEQ ID NO:11.

Embodiment 127. The method of embodiment 125, wherein the second SARS-CoV-2-specific amplification oligomer of (a)(i)(2) comprises or consists of a contiguous nucleotide sequence differing by no more than 0, 1, 2, 3, 4, or 5 nucleotides from the nucleotide sequence of SEQ ID NO:57, SEQ ID NO:58, SEQ ID NO:59, SEQ ID NO:60, SEQ ID NO:61, or SEQ ID NO:62, or an RNA equivalent or a DNA/RNA chimeric thereof and including from 0-7 nucleotide analogs.

Embodiment 128. The method of any one of embodiments 122 to 127, wherein the second SARS-CoV-2-specific target-hybridizing sequence of (a)(ii)(2) is joined at its 5' end to a promoter sequence.

Embodiment 129. The method of embodiment 128, wherein the promoter sequence is substantially identical to SEQ ID NO:11.

Embodiment 130. The method of embodiment 128, wherein the second SARS-CoV-2-specific amplification oligomer of (a)(ii)(2) comprises or consists of a contiguous nucleotide sequence differing by no more than 0, 1, 2, 3, 4, or 5 nucleotides from the nucleotide sequence of f SEQ ID NO:63, SEQ ID NO:64, SEQ ID NO:65, SEQ ID NO:66, SEQ ID NO:67; SEQ ID NO:68; SEQ ID NO:69; SEQ ID NO:70, or SEQ ID NO:78, or an RNA equivalent or a DNA/RNA chimeric thereof and including from 0-7 nucleotide analogs.

Embodiment 131. The method of any one of embodiments 122 to 130, wherein the detecting step (c) comprising contacting the one or more amplicons, if present, with one or more SARS-CoV-2-specific-detection probe oligomers selected from the group consisting of: (a) a first detection probe oligomer comprising a SARS-CoV-2-specific detection probe target-hybridizing sequence that is from 19 to 27 contiguous nucleotides in length, is contained within SEQ ID NO:50, and contains a nucleotide sequence differing by no more than 0, 1, 2, 3, 4, or 5 nucleotides from the nucleotide sequence of SEQ ID NO:51, SEQ ID NO:52, SEQ ID NO:53, or SEQ ID NO:114, or (ii) a second detection probe oligomer comprising a SARS-CoV-2-specific detection probe target-hybridizing sequence that is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO:115.

Embodiment 132. The method of embodiment 131, wherein the first and/or second detection probe oligomer comprises a SARS-CoV-2-specific detection probe target hybridizing sequence differing by no more than 0, 1, 2, 3, 4, or 5 nucleotides from the nucleotide sequence of: SEQ ID NO:14, SEQ ID NO: 17, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:44, SEQ ID NO:45, SEQ ID NO:46, SEQ ID NO:47, SEQ ID NO:48, SEQ ID NO:107, SEQ ID NO:108, SEQ ID NO:109 and SEQ ID NO:110, including a DNA equivalent, an RNA equivalent, or a DNA/RNA chimeric thereof and including from 0-7 nucleotide analogs.

Embodiment 133. The method of embodiment 131, wherein the one or more SARS-CoV-2-specific detection probe oligomers independently comprise a sequence selected from the group consisting of: SEQ ID NO:14, SEQ ID NO: 17, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:44, SEQ ID NO:45, SEQ ID NO:46, SEQ ID NO:47, SEQ ID NO:48, SEQ ID NO:103, SEQ ID NO:104, SEQ ID NO: 105, SEQ ID NO:106, SEQ ID NO:107, SEQ ID NO:108, SEQ ID NO:109 and SEQ ID NO:110, including a DNA equivalent, an RNA equivalent, or a DNA/RNA chimeric thereof and including from 0-7 nucleotide analogs.

Embodiment 134. The method of embodiment 131 or 132, wherein the SARS-CoV-2-specific detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 135. The method of embodiment 134, wherein the self-complementary sequence is joined to the SARS-CoV-2-specific detection probe target-hybridizing sequence via a non-nucleotide linker.

Embodiment 136. The method of any one of embodiments 131 to 135, wherein the SARS-CoV-2-specific detection probe oligomer further comprises a detectable label.

Embodiment 137. The method of embodiment 136, wherein the detectable label is a fluorescent or chemiluminescent label.

Embodiment 138. The method of embodiment 137, wherein the detectable label is a fluorescent label and the SARS-CoV-2-specific detection probe oligomer further comprises a non-fluorescent quencher.

Embodiment 139. The method of any one of embodiments 134-138, wherein the SARS-CoV-2-specific detection probe oligomer is a molecular beacon or a molecular torch.

Embodiment 140. The method of any one of embodiments 122 to 139, wherein the SARS-CoV-2 target nucleic acid is separated away from other components of the sample prior to the amplification step (b).

Embodiment 141. The method of embodiment 140, wherein the SARS-CoV-2 target nucleic acid is separated away from other components of the sample prior to the contacting step (a).

Embodiment 142. The method of embodiment 140 or 141, wherein the separating step is performed by directly or indirectly binding the SARS-CoV-2 target nucleic acid to a solid support followed by washing the SARS-CoV-2 target nucleic acid-solid support complex to remove other components of the sample.

Embodiment 143. The method of embodiment 142, wherein the SARS-CoV-2 target nucleic acid is hybridized to a target capture oligonucleotide, and wherein the target capture oligonucleotide directly binds the solid support.

Embodiment 144. The method of embodiment 142, wherein the SARS-CoV-2 target nucleic acid is hybridized to a target capture oligonucleotide, and wherein the target capture oligonucleotide directly binds an immobilized probe that is attached to the solid support.

Embodiment 145. The method of embodiment 143 or 144, wherein the target capture oligonucleotide comprises (i) a target hybridizing sequence containing or consisting of a nucleotide sequence differing by no more than 0, 1, 2, 3, 4, or 5 nucleotides from the nucleotide sequence of SEQ ID NO:97, SEQ ID NO:98, SEQ ID NO:99, SEQ ID NO:100, SEQ ID NO:101, or SEQ ID NO: 102, including a DNA equivalent, an RNA equivalent, or a DNA/RNA chimeric thereof and including from 0-24 nucleotide analogs, and (ii) a solid support binding region that is d(T)₀₋₃d(A)₁₄₋₃₀.

Embodiment 146. The method of any one of embodiments 143 to 145, wherein the target capture oligonucleotide comprises or consists of a nucleotides sequence differing by no more than 0, 1, 2, 3, 4, or 5 nucleotides from the nucleotide sequence of SEQ ID NO:91, SEQ ID NO:92, SEQ ID NO:93, SEQ ID NO:94, SEQ ID NO:95, or SEQ ID NO:96.

Embodiment 147. The method of any one of embodiments 122 to 146, wherein the *in vitro* nucleic acid amplification reaction is an isothermal amplification reaction.

Embodiment 148. The method of embodiment 147, wherein the isothermal amplification reaction is a transcription-mediated amplification (TMA) reaction or a nucleic acid sequence-based amplification (NASBA) reaction.

Embodiment 149. The method of any one of embodiments 122 to 146, wherein the *in vitro* nucleic acid amplification reaction is a PCR reaction.

Embodiment 150. The method of any one of embodiments 122 to 149, wherein the *in vitro* nucleic acid amplification reaction is a real-time reaction.

Embodiment 151. The method of any one of embodiments 122 to 150, wherein the *in vitro* nucleic acid amplification reaction is a quantitative reaction.

Embodiment 152. The method of any one of embodiments 122 to 151, wherein the *in vitro* nucleic acid amplification reaction is a multiplex reaction.

Embodiment 153. A method for determining the presence or absence of SARS-CoV-2 in a sample, the method comprising:
(a) contacting a sample containing or suspected of containing a SARS-CoV-2 target nucleic acid with an amplification oligomer combination comprising first and second SARS-CoV-2-specific amplification oligomers capable of amplifying a target region of the SARS-CoV-2 target nucleic acid,
   (i) wherein the first SARS-CoV-2-specific amplification oligomer comprises a first SARS-CoV-2-specific target-hybridizing sequence that is from 18 to 27 contiguous nucleotides in length, is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
   (ii) wherein the second SARS-CoV-2-specific amplification oligomer comprises a second SARS-CoV-2-specific target-hybridizing sequence that is from 18 to 23 contiguous nucleotides in length, is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO: 88;
(b) incubating the sample under conditions suitable for nucleic acid amplification, wherein any SARS-CoV-2 target nucleic acid, if present in the sample, is used as a template for generating an amplicon corresponding to the target region of the SARS-CoV-2 target nucleic acid; and
(c) detecting the presence or absence of the amplicon, thereby determining the presence or absence of SARS-CoV-2 in the sample.

Embodiment 154. The method of embodiment 153, wherein the first SARS-CoV-2-specific target-hybridizing sequence contains or consists of a nucleotide sequence differing by no more than 0, 1, 2, 3, 4, or 5 nucleotides from the nucleotide sequence of SEQ ID NO: 12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence contains or consists of a nucleotide sequence differing by no more than 0, 1, 2, 3, 4, or 5 nucleotides from the nucleotide sequence of SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.

Embodiment 155. The method of embodiment 153 or 154, wherein the second SARS-CoV-2-specific target-hybridizing sequence is joined at its 5' end to a promoter sequence.

Embodiment 156. The method of embodiment 155, wherein the promoter sequence is substantially identical to SEQ ID NO:11.

Embodiment 157. The method of embodiment 155, wherein the second SARS-CoV-2-specific amplification oligomer comprises or consists of a contiguous nucleotide sequence differing by no more than 0, 1, 2, 3, 4, or 5 nucleotides from the nucleotide sequence of SEQ ID NO:57, SEQ ID NO:58, SEQ ID NO:59, SEQ ID NO:60, SEQ ID NO:61, or SEQ ID NO:62, or an RNA equivalent or a DNA/RNA chimeric thereof and including from 0-7 nucleotide analogs.

Embodiment 158. The method of any one of embodiments 153 to 157, wherein the detecting step (c) comprising contacting the amplicon, if present, with a SARS-CoV-2-specific-detection probe oligomer comprising a SARS-CoV-2-specific detection probe target-hybridizing sequence that is from 19 to 27 contiguous nucleotides in length, is contained within SEQ ID NO:50, and contains of a nucleotide sequence differing by no more than 0, 1, 2, 3, 4, or 5 nucleotides from the nucleotide sequence of SEQ ID NO:51, SEQ ID NO:52, SEQ ID NO:53, or SEQ ID NO:114.

Embodiment 159. The method of embodiment 158, wherein the SARS-CoV-2-specific detection probe target-hybridizing sequence contains or consists of a nucleotide sequence differing by no more than 0, 1, 2, 3, 4, or 5 nucleotides from the nucleotide sequence of SEQ ID NO:14, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:107, or SEQ ID NO: 108, including a DNA equivalent, an RNA equivalent, or a DNA/RNA chimeric thereof and including from 0-7 nucleotide analogs.

Embodiment 160. The method of embodiment 158, wherein the SARS-CoV-2-specific detection probe oligomer target-hybridizing sequence contains or consists of a nucleotide sequence differing by no more than 0, 1, 2, 3, 4, or 5 nucleotides from the nucleotide sequence of SEQ ID NO:14, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:103, SEQ ID NO:104, SEQ ID NO:107, or SEQ ID NO:108, including a DNA equivalent, an RNA equivalent, or a DNA/RNA chimeric thereof and including from 0-7 nucleotide analogs.

Embodiment 161. The method of embodiment 158 or 159, wherein the SARS-CoV-2-specific detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 162. The method of embodiment 161, wherein the self-complementary sequence is joined to the SARS-CoV-2-specific detection probe target-hybridizing sequence via a non-nucleotide linker.

Embodiment 163. The method of any one of embodiments 158 to 162, wherein the SARS-CoV-2-specific detection probe oligomer further comprises a detectable label.

Embodiment 164. The method of embodiment 163, wherein the detectable label is a fluorescent or chemiluminescent label.

Embodiment 165. The method of embodiment 164, wherein the detectable label is a fluorescent label and the SARS-CoV-2-specific detection probe oligomer further comprises a non-fluorescent quencher.

Embodiment 166. The method of embodiment 164, wherein the SARS-CoV-2-specific detection probe oligomer is a molecular beacon or a molecular torch.

Embodiment 167. The method of any one of embodiments 153 to 166, wherein the SARS-CoV-2 target nucleic acid is separated away from other components of the sample prior to the amplification step (b).

Embodiment 168. The method of embodiment 167, wherein the SARS-CoV-2 target nucleic acid is separated away from other components of the sample prior to the contacting step (a).

Embodiment 169. The method of embodiment 167 or 168, wherein the separating step is performed by directly or indirectly binding the SARS-CoV-2 target nucleic acid to a solid support followed by washing the SARS-CoV-2 target nucleic acid-solid support complex to remove other components of the sample.

Embodiment 170. The method of embodiment 169, wherein the SARS-CoV-2 target nucleic acid is hybridized to a target capture oligonucleotide, and wherein the target capture oligonucleotide directly binds the solid support.

Embodiment 171. The method of embodiment 169, wherein the SARS-CoV-2 target nucleic acid is hybridized to a target capture oligonucleotide, and wherein the target capture oligonucleotide directly binds an immobilized probe that is attached to the solid support.

Embodiment 172. The method of embodiment 170 or 171, wherein the target capture oligonucleotide comprises (i) a target hybridizing sequence containing or consisting of a nucleotide sequence differing by no more than 0, 1, 2, 3, 4, or 5 nucleotides from the nucleotide sequence of SEQ ID NO:97, SEQ ID NO:98, SEQ ID NO:99, SEQ ID NO:100, SEQ ID NO:101, or SEQ ID NO: 102, including a DNA equivalent, an RNA equivalent, or a DNA/RNA chimeric thereof and including from 0-24 nucleotide analogs, and (ii) a solid support binding region that is d(T)₀₋₃d(A)₁₄₋₃₀.

Embodiment 173. The method of any one of embodiments 170 to 172, wherein the target capture oligonucleotide comprises a sequence containing or consisting of a nucleotide sequence differing by no more than 0, 1, 2, 3, 4, or 5 nucleotides from the nucleotide sequence of SEQ ID NO:91, SEQ ID NO:92, SEQ ID NO:93, SEQ ID NO:94, SEQ ID NO:95, or SEQ ID NO:96.

Embodiment 174. The method of any one of embodiments 153 to 173, wherein the *in vitro* nucleic acid amplification reaction is an isothermal amplification reaction.

Embodiment 175. The method of embodiment 174, wherein the isothermal amplification reaction is a TMA reaction or a NASBA reaction.

Embodiment 176. The method of any one of embodiments 153 to 173, wherein the *in vitro* nucleic acid amplification reaction is a PCR reaction.

Embodiment 177. The method of any one of embodiments 153 to 176, wherein the *in vitro* nucleic acid amplification reaction is a quantitative reaction.

Embodiment 178. The method of any one of embodiments 153 to 177, wherein the *in vitro* nucleic acid amplification reaction is a real-time reaction.

Embodiment 179. A method for determining the presence or absence of SARS-CoV-2 in a sample, the method comprising:
(a) contacting a sample containing or suspected of containing a SARS-CoV-2 target nucleic acid with an amplification oligomer combination comprising first and second SARS-CoV-2-specific amplification oligomers capable of amplifying a target region of the SARS-CoV-2 target nucleic acid,
   (i) wherein the first SARS-CoV-2-specific amplification oligomer comprises a first SARS-CoV-2-specific target-hybridizing sequence that is from 20 to 23 contiguous nucleotides in length, is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
   (ii) wherein the second SARS-CoV-2-specific amplification oligomer comprises a second SARS-CoV-2-specific target-hybridizing sequence that is 16 to 27 contiguous nucleotides in length, is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113;
(b) incubating the sample under conditions suitable for nucleic acid amplification, wherein any SARS-CoV-2 target nucleic acid, if present in the sample, is used as a template for generating an amplicon corresponding to the target region of the SARS-CoV-2 target nucleic acid; and
(c) detecting the presence or absence of the amplicon, thereby determining the presence or absence of SARS-CoV-2 in the sample.

Embodiment 180. The method of embodiment 179, wherein the first SARS-CoV-2-specific target-hybridizing sequence contains or consists of a nucleotide sequence differing by no more than 0, 1, 2, 3, 4, or 5 nucleotides from the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence contains or consists of a nucleotide sequence differing by no more than 0, 1, 2, 3, 4, or 5 nucleotides from the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.

Embodiment 181. The method of embodiment 179 or 180, wherein the second SARS-CoV-2-specific target-hybridizing sequence is joined at its 5' end to a promoter sequence.

Embodiment 182. The method of embodiment 181, wherein the promoter sequence is substantially identical to SEQ ID NO:11.

Embodiment 183. The method of embodiment 181, wherein the second SARS-CoV-2-specific amplification oligomer comprises or consists of a contiguous nucleotide sequence differing by no more than 0, 1, 2, 3, 4, or 5 nucleotides from the nucleotide sequence of SEQ ID NO: 63, SEQ ID NO:64, SEQ ID NO:65, SEQ ID NO:66, SEQ ID NO:67; SEQ ID NO:68; SEQ ID NO:69; SEQ ID NO:70, or SEQ ID NO:78, or an RNA equivalent or a DNA/RNA chimeric thereof and including from 0-7 nucleotide analogs.

Embodiment 184. The method of any one of embodiments 179 to 183, wherein the detecting step (c) comprising contacting the amplicon with a SARS-CoV-2-specific detection probe oligomer comprising a SARS-CoV-2-specific detection probe target-hybridizing sequence that that is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO:115.

Embodiment 185. The method of embodiment 184, wherein the SARS-CoV-2-specific detection probe target-hybridizing sequence contains or consists of a nucleotide sequence differing by no more than 0, 1, 2, 3, 4, or 5 nucleotides from the nucleotide sequence of SEQ ID NO:17, SEQ ID NO:43, SEQ ID NO:44, SEQ ID NO:45, SEQ ID NO:46, SEQ ID NO:47, SEQ ID NO:48, SEQ ID NO:109 or SEQ ID NO:110, including a DNA equivalent, an RNA equivalent, or a DNA/RNA chimeric thereof and including from 0-7 nucleotide analogs.

Embodiment 186. The method of embodiment 184, wherein the SARS-CoV-2-specific detection probe oligomer target-hybridizing sequence contains or consists of a nucleotide sequence differing by no more than 0, 1, 2, 3, 4, or 5 nucleotides from the nucleotide sequence of SEQ ID NO:17, SEQ ID NO:43, SEQ ID NO:44, SEQ ID NO:45, SEQ ID NO:46, SEQ ID NO:47, SEQ ID NO:48, SEQ ID NO:105, SEQ ID NO:106, SEQ ID NO:109, or SEQ ID NO:110, including a DNA equivalent, an RNA equivalent, or a DNA/RNA chimeric thereof and including from 0-7 nucleotide analogs.

Embodiment 187. The method of embodiment 184 or 185, wherein the SARS-CoV-2-specific detection probe oligomer further comprises a self-complementary sequence joined to the SARS-CoV-2-specific detection probe target-hybridizing sequence, optionally wherein the self-complementary sequence joined to the 5' end of the SARS-CoV-2-specific detection probe target-hybridizing sequence.

Embodiment 188. The method of embodiment 187, wherein the self-complementary sequence is joined to the SARS-CoV-2-specific detection probe target-hybridizing sequence via a non-nucleotide linker.

Embodiment 189. The method of any one of embodiments 184 to 188, wherein the SARS-CoV-2-specific detection probe oligomer further comprises a detectable label.

Embodiment 190. The method of embodiment 189, wherein the detectable label is a fluorescent or chemiluminescent label.

Embodiment 191. The method of embodiment 190, wherein the detectable label is a fluorescent label and the SARS-CoV-2-specific detection probe oligomer further comprises a non-fluorescent quencher.

Embodiment 192. The method of embodiment 190, wherein the SARS-CoV-2-specific detection probe oligomer is a molecular beacon or a molecular torch.

Embodiment 193. The method of any one of embodiments 179 to 192, wherein the SARS-CoV-2 target nucleic acid is separated away from other components of the sample prior to the amplification step (b).

Embodiment 194. The method of embodiment 193, wherein the SARS-CoV-2 target nucleic acid is separated away from other components of the sample prior to the contacting step (a).

Embodiment 195. The method of embodiment 193 or 194, wherein the separating step is performed by directly or indirectly binding the SARS-CoV-2 target nucleic acid to a solid support followed by washing the SARS-CoV-2 target nucleic acid-solid support complex to remove other components of the sample.

Embodiment 196. The method of embodiment 195, wherein the SARS-CoV-2 target nucleic acid is hybridized to a target capture oligonucleotide, and wherein the target capture oligonucleotide directly binds the solid support.

Embodiment 197. The method of embodiment 195, wherein the SARS-CoV-2 target nucleic acid is hybridized to a target capture oligonucleotide, and wherein the target capture oligonucleotide directly binds an immobilized probe that is attached to the solid support.

Embodiment 198. The method of embodiment 196 or 197, wherein the target capture oligonucleotide comprises (i) a target hybridizing sequence containing or consisting of a nucleotide sequence differing by no more than 0, 1, 2, 3, 4, or 5 nucleotides from the nucleotide sequence of SEQ ID NO:97, SEQ ID NO:98, SEQ ID NO:99, SEQ ID NO:100, SEQ ID NO:101, or SEQ ID NO: 102, including a DNA equivalent, an RNA equivalent, or a DNA/RNA chimeric thereof and including from 0-24 nucleotide analogs, and (ii) a solid support binding region that is d(T)₀₋₃d(A)₁₄₋₃₀.

Embodiment 199. The method of any one of embodiments 196 to 198, wherein the target capture oligonucleotide comprises or consists of a nucleotide sequence differing by no more than 0, 1, 2, 3, 4, or 5 nucleotides from the nucleotide sequence of SEQ ID NO:91, SEQ ID NO:92, SEQ ID NO:93, SEQ ID NO:94, SEQ ID NO:95, or SEQ ID NO:96.

Embodiment 200. The method of any one of embodiments 179 to 199, wherein the *in vitro* nucleic acid amplification reaction is an isothermal amplification reaction.

Embodiment 201. The method of embodiment 200, wherein the isothermal amplification reaction is a TMA reaction or a NASBA reaction.

Embodiment 202. The method of any one of embodiments 179 to 199, wherein the *in vitro* nucleic acid amplification reaction is a PCR reaction.

Embodiment 203. The method of any one of embodiments 179 to 202, wherein the *in vitro* nucleic acid amplification reaction is a quantitative reaction.

Embodiment 204. The method of any one of embodiments 179 to 203, wherein the *in vitro* nucleic acid amplification reaction is a real-time reaction.

Embodiment 205. A multiplex method for determining the presence or absence of SARS-CoV-2 and at least one other pathogen in a sample, wherein the presence or absence of SARS-CoV-2 is determined using the method of any one of embodiments 153 to 178.

Embodiment 206. The multiplex method of embodiment 205, wherein the method determines the presence or absence of SARS-CoV-2 and at least one respiratory pathogen in a sample.

Embodiment 207. The multiplex method of embodiment 205, wherein the method determines the presence or absence of SARS-CoV-2 and one or more pathogens selected from the group consisting of: influenza A, influenza B, respiratory syncytial virus A, respiratory syncytial virus B, parainfluenza virus 1, parainfluenza virus 2, parainfluenza virus 3, parainfluenza virus 4, adenovirus, metapneumovirus, rhino virus, coronavirus 229E, coronavirus NL63, coronavirus HKU1, coronavirus OC43, SARS-CoV (SARS), and MERS-CoV.

Embodiment 208. A multiplex method for determining the presence or absence of SARS-CoV-2 and at least one other pathogen in a sample, wherein the presence or absence of SARS-CoV-2 is determined using the method of any one of embodiments 179 to 204.

Embodiment 209. The multiplex method of embodiment 208, wherein the method determines the presence or absence of SARS-CoV-2 and at least one respiratory pathogen in a sample.

Embodiment 210. The multiplex method of embodiment 208, wherein the method determines the presence or absence of SARS-CoV-2 and one or more pathogens selected from the group consisting of: influenza virus A, influenza virus B, respiratory syncytial virus A, respiratory syncytial virus B, parainfluenza virus 1, parainfluenza virus 2, parainfluenza virus 3, parainfluenza virus 4, adenovirus, metapneumovirus, rhino virus, coronavirus 229E, coronavirus NL63, coronavirus HKU1, coronavirus OC43, SARS-CoV (SARS), and MERS-CoV.

Embodiment 211. A multiplex method for determining the presence or absence of SARS-CoV-2 and at least one other pathogen in a sample, wherein the presence or absence of SARS-CoV-2 is determined using the method of any one of embodiments 122-152.

Embodiment 212. The multiplex method of embodiment 211, wherein the method determines the presence or absence of SARS-CoV-2 and at least one respiratory pathogen in a sample.

Embodiment 213. The multiplex method of embodiment 211, wherein the method determines the presence or absence of SARS-CoV-2 and one or more pathogens selected from the group consisting of: influenza A, influenza B, respiratory syncytial virus A, respiratory syncytial virus B, parainfluenza virus 1, parainfluenza virus 2, parainfluenza virus 3, parainfluenza virus 4, adenovirus, metapneumovirus, rhino virus, coronavirus 229E, coronavirus NL63, coronavirus HKU1, coronavirus OC43, SARS-CoV (SARS), and MERS-CoV.

Embodiment 214. The method of any one of embodiments 122 to 223, wherein the sample is a pooled sample.

These and other aspects and embodiments will become evident upon reference to the following detailed description and the attached drawings.

### BRIEF DESCRIPTION OF THE FIGURES

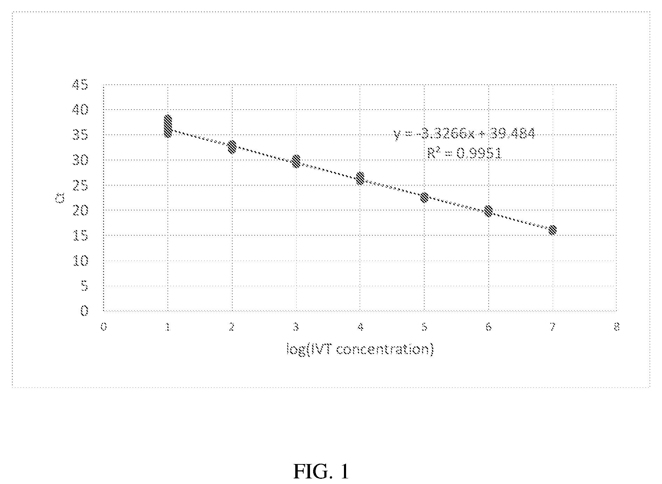

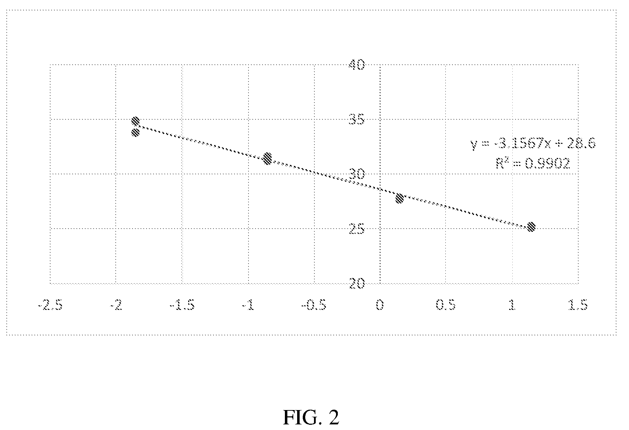

Figures 1 and 2 are linearity plots showing performance of primer and probe (PPR) mixtures for the amplification and detection of SARS-CoV-2 organisms.
FIG. 1 shows linearity data for reactions against dilutions of *in vitro* transcript (IVT) using a combined PPR mix, which contained each of SARS-CoV-2 PPR Mix 1 and SARS-CoV-2 PPR Mix 5 as shown in Table 1. See Example 2, *infra.*
FIG. 2 shows linearity data for reactions against dilutions of a specific virus strain using the same combined PPR mix. *See* Example 3, *infra.*

### DEFINITIONS

Unless defined otherwise, all technical and scientific terms used herein have the same meaning as commonly understood by one of ordinary skill in the art pertinent to the methods and compositions described. As used herein, the following terms and phrases have the meanings ascribed to them unless specified otherwise.

The terms "a," "an," and "the" include plural referents, unless the context clearly indicates otherwise. For example, "a nucleic acid" as used herein is understood to represent one or more nucleic acids. As such, the terms "a" (or "an"), "one or more," and "at least one" can be used interchangeably herein.

When a value is expressed as "about" X or "approximately" X, the stated value of X will be understood to be accurate to ±10%.

All ranges are to be interpreted as encompassing the endpoints in the absence of express exclusions such as "not including the endpoints"; thus, for example, "within 10-15" includes the values 10 and 15. One skilled in the art will understand that the recited ranges include the end values, as whole numbers in between the end values, and where practical, rational numbers within the range (*e.g.,* the range 5-10 includes 5, 6, 7, 8, 9, and 10, and where practical, values such as 6.8, 9.35, etc.).

"Sample" includes any specimen that contains or is suspected of containing a coronavirus causing COVID-19, such as SARS-CoV-2, including components thereof, such as nucleic acids or fragments of nucleic acids. Samples include "biological samples" which include any tissue or material derived from a living or dead mammal (such as a human) or organism. Biological samples include, but are not limited to, nasopharyngeal swab, nasal swab, mid-turbinate swab, oropharyngeal swab, throat swab, nasal wash, bronchial wash, nasal aspirate, sputum, blood, plasma, serum, blood cells, saliva, mucous, respiratory tissue, exudates (e.g., bronchoalveolar lavage), sputum, tracheal aspirates, lymph node, gastrointestinal tissue, feces, urine, genitourinary fluid, and biopsy cells or tissue. A sample may be treated or processed by sample preparation. A sample may be an individual sample (*i.e.,* a sample derived from a single subject) or a pooled sample (*i.e.,* a sample prepared by pooling a plurality of individual samples).

"Sample preparation" refers to any steps or methods required to prepare a sample for amplification and/or detection. A sample may be treated to chemically, physically and/or mechanically disrupt tissue, cells, or cellular components to release intracellular components into an aqueous or organic solution which may further contain enzymes, buffers, salts, detergents and the like, which are used to prepare a biological sample for analysis. A sample may also be treated chemically, physically, and/or mechanically to remove cellular components or debris. A sample may be processed by passing the samples over or through a filtering device, centrifugation, or by adherence to a medium, matrix, or support. Sample preparation includes knowns method of concentrating components, such as polynucleotides, from a larger sample volume, such as by filtration from larger volume sample, centrifugation, or by isolation of microbes from a sample by using standard microbiology methods. Sample preparation may also include use of a polynucleotide to specifically or non-specifically capture a target nucleic acid and separate it from other sample components.

The term "target capture" refers to selectively separating or isolating a target nucleic acid from other components of a sample mixture, such as cellular fragments, organelles, proteins, lipids, carbohydrates, or other nucleic acids. A target capture system may be specific and selectively separate a predetermined target nucleic acid from other sample components (*e.g.,* by using a sequence specific to the intended target nucleic acid, such as a TCO TS sequence). Target capture methods and compositions have been previously described in detail (U.S. Pat. Nos. 6,110,678 and 6,534,273; and US Pub. No. 2008/0286775 A1). In some embodiments, target capture utilizes a TCO in solution phase and an immobilized capture probe attached to a support to form a complex with the target nucleic acid and separate the captured target from other components.

A "Target capture oligonucleotide" (TCO) is a nucleic acid oligonucleotide that specifically hybridizes to a sequence in a target nucleic acid by standard base pairing and joins to a binding partner on an immobilized probe to capture the target nucleic acid to a support. TCOs can be used to capture or isolate the target nucleic acid from a sample. The TCO comprises a target specific (TS) nucleotide sequence that hybridizes to (*i.e.,* is complementary to) a region of a target nucleic acid. In some embodiments, the TCO TS sequence comprises a 10-35 nucleotide sequence having at least 90%, at least 95%, or 100% complementarity to a nucleotide sequence present in the target nucleic acid and hybridizes to a region in the target nucleic acid sequence (a TCO binding site). A TCO includes an immobilized capture probe-binding region that binds to an immobilized capture probe (*e.g.,* by specific binding pair interaction). In some embodiments, the TCO TS sequence is linked to the capture probe-binding region. In some embodiments, the TCO TS sequence and capture probe-binding region are present on two different oligonucleotides joined together by one or more linkers. In some embodiments, the capture probe-binding region comprises: a poly A sequence, a poly T sequence, or a polyT-polyA sequence. In some embodiments, a polyT-polyA sequence comprises (dT)₀₋₃(dA)₁₄₋₃₀ or (dT)₃(dA)₃₀.

An "immobilized capture probe" provides a means for joining a TCO to a solid support. In some embodiments, an immobilized capture probe contains a base sequence recognition molecule joined to the solid support, which facilitates separation of bound target polynucleotide from unbound material. Any known solid support may be used, such as matrices and particles free in solution. For example, solid supports may be nitrocellulose, nylon, glass, polyacrylate, mixed polymers, polystyrene, silane polypropylene and magnetically attractable particles. In some embodiments, the supports include magnetic spheres that are monodisperse (*i.e.,* uniform in size ± about 5%). The immobilized capture probe may be joined directly (*e.g.,* via a covalent linkage or ionic interaction), or indirectly to the solid support. Common examples of useful solid supports include magnetic particles or beads.

A "nucleotide" is a subunit of a nucleic acid consisting of a phosphate group, a 5-carbon sugar, and a nitrogenous base (also referred to herein as "nucleobase"). The 5-carbon sugar found in RNA is ribose. In DNA, the 5-carbon sugar is 2'-deoxyribose.

"Nucleic acid" and "polynucleotide" refer to a multimeric compound comprising nucleotides and/or nucleotide analogs linked together to form a biopolymer. The biopolymers include conventional RNA, conventional DNA, mixed RNA-DNA, and nucleotide analog containing versions thereof. A nucleic acid "backbone" may be made up of a variety of linkages, including one or more of sugar-phosphodiester linkages, peptide-nucleic acid bonds ("peptide nucleic acids" or PNA), phosphorothioate linkages, methylphosphonate linkages, or combinations thereof. Sugar moieties of a nucleic acid may be ribose, deoxyribose, or similar compounds with substitutions or modifications, *e.g.,* analogs with a methoxy, fluoro or halide group at the 2' position of the ribose (also referred to herein as "2'-O-Me" or "2'-methoxy" or 2'-fluoro, or "2'-halide"). Nitrogenous bases may be conventional bases, adenine (A), uracil (U), guanine (G), thymine (T), and cytosine (C), and analogs thereof (*e.g.,* inosine, 5-methyl-2'-deoyxcytosine ("5-Me-dC" or "5MeC"), propyne dU, and isoguanine). Nucleic acids may include one or more "abasic" residues where the backbone includes no nitrogenous base for position(s) of the polymer.

Sequence identity can be determined by aligning sequences using algorithms, such as BESTFIT, FASTA, and TFASTA in the Wisconsin Genetics Software Package Release 7.0, Genetics Computer Group, 575 Science Dr., Madison, Wis.), using default gap parameters, or by inspection, and the best alignment (*i.e.,* resulting in the highest percentage of sequence similarity over a comparison window). Percentage of sequence identity is calculated by comparing two optimally aligned sequences over a window of comparison, determining the number of positions at which the identical residues occurs in both sequences to yield the number of matched positions, dividing the number of matched positions by the total number of matched and mismatched positions not counting gaps in the window of comparison *(i.e.,* the window size), and multiplying the result by 100 to yield the percentage of sequence identity. Unless otherwise indicated the window of comparison between two sequences is defined by the entire length of the shorter of the two sequences.

The term "complementarity" refers to the ability of a polynucleotide to form hydrogen bond(s) (hybridize) with another polynucleotide sequence by either traditional Watson-Crick base pairing or other non-traditional types of base paring. The two complementary polynucleotide strands are antiparallel one another. A percent complementarity indicates the percentage of bases, in a contiguous strand, in a first nucleic acid sequence which can form hydrogen bonds (*e.g.,* Watson-Crick base pairing) with a second nucleic acid sequence (*e.g*., 5, 6, 7, 8, 9, 10 out of 10 being 50%, 60%, 70%, 80%, 90%, and 100% complementary). Percent complementarity is calculated in a similar manner to percent identify.

By "RNA equivalents" and "DNA equivalents" is meant RNA and DNA molecules having essentially the same nucleic acid sequence or complementary base pair hybridization properties. RNA and DNA equivalents have different sugar moieties (*i.e.,* ribose versus deoxyribose) and may differ by the presence of uracil in RNA and thymine in DNA. The differences between RNA and DNA equivalents do not contribute to differences in homology because the equivalents have the same degree of complementarity to a particular sequence. By "DNA/RNA chimeric" is meant a nucleic acid comprising both DNA and RNA nucleotides. Unless the context clearly dictates otherwise, reference to a nucleic acid of coronavirus includes the RNA and DNA equivalents and DNA/RNA chimerics thereof.

An "oligomer," "oligonucleotide," or "oligo" refers to a nucleic acid of generally less than 1,000 nucleotides (nt), including those in a size range having a lower limit of about 5 nt and an upper limit of about 900 nt. In some embodiments, the oligomers are in a size range having a 5 to 15 nt lower limit and a 50 to 500 nt upper limit. In some embodiments, the oligomers are in a size range of 10-100 nucleobases, 10-90 nucleobases, 10-80 nucleobases, 10-70 nucleobases, or 10-60 nucleobases. The oligonucleotide may be DNA and/or RNA and/or analogs thereof. The term oligonucleotide does not denote any particular function to the reagent; rather, it is used generically to cover all such reagents described herein. Oligomers may be referred to by a functional name (*e.g.,* capture probe, primer, or promoter primer) but those skilled in the art will understand that such terms refer to oligomers. Described are oligomers that include RNA polymerase promoter-containing oligomers (also termed promoter primers; *e.g.,* T7 primers), non-RNA polymerase promoter-containing oligomers (also termed non-T7 primers, NT7 primers, or non-promoter primers), probe oligomers (also termed detection oligomers or detection probes, probes, or Torches), target capture oligonucleotides (TCOs), forward primers (non-T7 primer or NT7 primer), and reverse primers (including T7 primers). Oligomers can be made synthetically using any well-known *in vitro* chemical or enzymatic method, and may be purified after synthesis by using standard methods, e.g., high-performance liquid chromatography (HPLC).

Any of the described oligonucleotides can contain at least one modified nucleotide (*i.e*., at least one nucleotide analog). The modified nucleotide can be, but is not limited to, 2'-O-methyl modified nucleotide, 2'-fluoro modified nucleotide, or a 5-methyl cytosine. In some embodiments, an amplification oligonucleotide comprises two or more modified nucleotides. The two or more modified nucleotides may be the same or different. In some embodiments, thymidine nucleotides can be substituted for uridine nucleotides. In some embodiments, all thymidine nucleotides can be substituted for uridine nucleotides. In some embodiments, 5-methyl-2-deoxycytosine bases can be used to increase the stability of the duplex by raising the Tm by about 0.5°-1.3°C for each 5'methyl-2'deoxycytosine incorporated in an oligonucleotide (relative to the corresponding unmethylated amplification oligonucleotides).

A "target nucleic acid" is a nucleic acid comprising a target sequence to be amplified and/or detected. Target nucleic acids may be DNA or RNA and may be either single-stranded or double-stranded. A target nucleic acid can be, but is not limited to, a genomic nucleic acid and a transcribed nucleic acid, such as an mRNA. For a single stranded target nucleic acid, such as a single stranded RNA virus or an mRNA, the target nucleic acid includes the complement thereof. A target nucleic acid can also be a nucleic acid derived from a genomic or transcribed nucleic acid. A target nucleic acid (including where appropriate its complement) contains sequences that hybridize to capture oligonucleotides, primers, and/or probes used to amplify and/or detect the target nucleic acid. The target nucleic acid may include other sequences besides the target sequence, which may not be amplified.

A "target sequence" or "target nucleic acid sequence" is the particular nucleotide sequence of the target nucleic acid that is to be amplified and/or detected. The target sequence, which includes a complement thereof, contains sequences that hybridize to primers and probes used to amplify and/or detect the target nucleic acid during an amplification processes (*e.g.,* PCR, TMA). Unless the context clearly dictates otherwise, where the target nucleic acid is originally single-stranded, the term "target sequence" will also refer to the sequence complementary to the "target sequence" as present in the target nucleic acid, and where the target nucleic acid is originally double-stranded, the term "target sequence" refers to both the sense (+) and antisense (-) strands.

A "target-hybridizing sequence," "target hybridizing region," or "target-specific sequence" is a sequence in an oligomer that hybridizes to a region in a target nucleic acid sequence. The target hybridizing region is a contiguous sequence of nucleotides that hybridizes to a complementary contiguous sequence of nucleotides in the target nucleic acid sequence. Target hybridizing sequences are configured to specifically hybridize with a target nucleic acid. Target-hybridizing sequences are configured to specifically hybridize with a target nucleic acid. Target-hybridizing sequences may be 100% complementary to the portion of the target nucleic acid sequence to which they are configured to hybridize, but not necessarily. Target-hybridizing sequences may also include inserted, deleted, and/or substituted nucleotide residues relative to a target sequence provided the target hybridizing sequence specifically hybridizes with a target nucleic acid. A primer or probe can contain both target specific sequence and non-target specific sequence. The target specific sequence (or target hybridizing sequence or target hybridizing region) is the portion of the oligonucleotide that is configured to hybridize with a target nucleic acid. Reference to an oligonucleotide (such as a primer or probe) comprising a target hybridizing sequence consisting of SEQ ID NO: X indicates the portion of the oligonucleotide that is complementary to the target nucleic acid consists only of the indicated SEQ ID NO. The oligonucleotide may contain other non-target hybridizing sequences or other components (such as a label), but the target hybridizing sequence consists of the sequence in the indicated SEQ ID NO.

"Non-target-specific sequence" or "non-target-hybridizing sequence" refers to a region of an oligomer sequence, wherein said region does not stably hybridize with a target sequence under standard hybridization conditions. Oligomers with non-target specific sequences include, but are not limited to, promoter primers, promoter providers, target capture oligonucleotides, torches, and molecular beacons.

"Target a sequence," is used in reference to a region of a SARS-CoV-2 nucleic acid whereby an oligonucleotide hybridizes to a target sequence in a manner that allows for amplification and/or detection as described herein. In some embodiments, the oligonucleotide is complementary with the targeted SARS-CoV-2 nucleic acid sequence and contains no mismatches. In some embodiments, the oligonucleotide is complementary but contains 1, 2, 3, 4, or 5 mismatches with the targeted SARS-CoV-2 nucleic acid sequence.

The term "configured to" denotes an actual arrangement of the polynucleotide sequence configuration of a referenced oligonucleotide target-hybridizing sequence. For example, amplification oligomers that are configured to generate a specified amplicon from a target sequence have polynucleotide sequences that hybridize to the target sequence and can be used in an amplification reaction to generate the amplicon. Also, as an example, oligonucleotides that are configured to specifically hybridize to a target sequence have a polynucleotide sequence that specifically hybridizes to the referenced sequence under stringent hybridization conditions.

The term "configured to specifically hybridize to" indicates that that the target-hybridizing region of an amplification oligonucleotide, detection probe, or other oligonucleotide is designed to have a polynucleotide sequence that can target a sequence of the referenced SARS-CoV-2 target sequence. The oligonucleotide is designed to function as a component of an assay for amplification and/or detection of a SARS-CoV-2 target nucleic acid in a sample, and therefore is designed to target SARS-CoV-2 in the presence of other nucleic acids commonly found in testing samples. "Specifically hybridize to" does not mean exclusively hybridize to, as some small level of hybridization to non-target nucleic acids may occur, as is understood in the art. Rather, "specifically hybridize to" means that the oligonucleotide is configured to function in an assay to primarily hybridize the target so that an accurate amplification and/or detection of target nucleic acid in a sample can be performed.

An "amplification oligonucleotide," "amplification oligomer," or "primer" is an oligonucleotide that hybridizes to a target nucleic acid and participates in a nucleic acid amplification reaction, *e.g.,* serving as a primer. Amplification oligomers can have 3' ends that are extended by polymerization as part of the nucleic acid amplification reaction. Amplification oligomers can alternatively have 3' ends that are not extended by polymerization, but provide a component that facilitates nucleic acid amplification, e.g., a promoter sequence joined 5' to the target hybridizing sequence of the amplification oligomer. Such an amplification oligomer is referred to as a promoter provider. Amplification oligomers that provide both a 3' target hybridizing region that is extendable by polymerization and a 5' promoter sequence are referred to as promoter primers. Amplification oligomers may be optionally modified to include 5' non-target hybridizing regions such as tags, promoters (as mentioned), or other sequences used or useful for manipulating or amplifying the primer or target oligonucleotide.

"Nucleic acid amplification" refers to any *in vitro* procedure that produces multiple copies of a target nucleic acid sequence, or its complementary sequence, or fragments thereof (*i.e.,* an amplified sequence containing less than the complete target nucleic acid). Examples of nucleic acid amplification procedures include transcription associated methods, such as transcription-mediated amplification (TMA), nucleic acid sequence-based amplification (NASBA) and others (*e.g.,* U.S. Patent Nos. 5,399,491, 5,554,516, 5,437,990, 5,130,238, 4,868,105, and 5,124,246), and polymerase chain reaction (PCR) *(e.g.,* U.S. Patent Nos. 4,683,195, 4,683,202, and 4,800,159).

"Transcription-mediated amplification" uses a DNA polymerase (*e.g.,* reverse transcriptase), an RNA polymerase, deoxyribonucleoside triphosphates, ribonucleoside triphosphates, primers, including a promoter primer, and optionally may include other oligonucleotides, to produce multiple RNA transcripts from a nucleic acid template (described in detail in US Pat. Nos. 5,399,491 and 5,554,516, Kacian et al., US Pat. No. 5,437,990, Burg et al., PCT Nos. WO 88/01302 and WO 88/10315, Gingeras et al., US Pat. No. 5,130,238, Malek et al., US Pat. Nos. 4,868,105 and 5,124,246, Urdea et al., PCT No. WO 94/03472, McDonough et al., PCT No. WO 95/03430, and Ryder et al., each of which is incorporated herein by reference). Methods that use TMA are described in detail previously (US Pat. Nos. 5,399,491 and 5,554,516, each of which is incorporated herein by reference). TMA can be a substantially isothermal amplification. TMA can also be run as a biphasic amplification reaction.

The term "substantially isothermal amplification" refers to an amplification reaction that is conducted at a substantially constant temperature. The isothermal portion of the reaction may be preceded or followed by one or more steps at a variable temperature, for example, a first denaturation step and a final heat inactivation step or cooling step. It will be understood that this definition does not exclude small variations in temperature but is rather used to differentiate the isothermal amplification techniques from other amplification techniques known in the art that basically rely on "cycling temperatures" in order to generate the amplified products.

An "amplicon" or "amplification product" is a nucleic acid molecule generated in a nucleic acid amplification reaction and which is derived (amplified) from a target nucleic acid. An amplicon or amplification product contains a target nucleic acid sequence that may be of the same or opposite sense as the target nucleic acid.

"Relative fluorescence unit" ("RFU") is a unit of measurement of fluorescence intensity. RFU varies with the characteristics of the detection means used for the measurement and can be used as a measurement to compare relative intensities between samples and controls.

A "detection probe oligomer," "detection probe," or "probe" is an oligomer that hybridizes specifically to a target sequence, including an amplified product, under conditions that promote nucleic acid hybridization, for detection of the target nucleic acid. Detection may either be direct (*i.e.,* probe hybridized directly to the target) or indirect *(i.e.,* a probe hybridized to an intermediate structure that links the probe to the target). A probe's target sequence generally refers to the specific sequence within a larger sequence which the probe hybridizes specifically. A detection probe may include target specific sequence(s) and non-target specific sequence(s). Such non-target specific sequences can include sequences which will confer a desired secondary or tertiary structure, such as a hairpin structure, which can be used to facilitate detection and/or amplification. A probe can have a detectable label. The detectable label can be joined directly or indirectly to the probe.

A "Molecular torch" or "Torch" is a type of probe and can be used to indicate whether an amplicon is present in the sample. Molecular torches include distinct regions of self-complementarity. When exposed to the target, the two self-complementary regions (fully or partially complementary) of the molecular torch melt, thus allowing for the individual nucleotides (comprising the target binding domain) to hybridize to the complementary contiguous nucleotides on the target nucleic acid sequence. Molecular torches are designed so that the target binding domain favors hybridization to the target nucleic acid sequence over the target closing domain (region of self-complementarity). The target binding domain and the target closing domain of a molecular torch include interacting labels (*e.g.,* fluorescent dye and quencher, FRET pair), so that a different signal is produced when the molecular torch is self-hybridized, as opposed to when the molecular torch is hybridized to a target nucleic acid sequence (thereby permitting detection of probe:target duplexes in a test sample in the presence of unhybridized probe). Methods of synthesizing labels, attaching labels to nucleic acid, and detecting signals from labels are well known in the art (*e.g.,* Sambrook et al., supra, at Chapter 10, and US Pat. Nos. 5,658,737, 5,656,207, 5,547,842, 5,283,174, and 4,581,333, and EP Pat. App. 0747706).

"Stringent hybridization conditions," or "stringent conditions" are conditions permitting an oligomer to preferentially hybridize to a target sequence and not to nucleic acid derived from a closely related non-target nucleic acid (*i.e.,* conditions permitting an oligomer to hybridize to its target sequence to form a stable oligomer:target hybrid, but not form a sufficient number of stable oligomer:non-target hybrids, so as to allow for amplification and/or detection of target nucleic acids but not non-targeted organisms). While the definition of stringent hybridization conditions does not vary, the actual reaction environment that can be used for stringent hybridization may vary depending upon factors including the GC content and length of the oligomer, the degree of similarity between the oligomer sequence and sequences of non-target nucleic acids that may be present in the test sample, and the target sequence. Hybridization conditions include the temperature and the composition of the hybridization reagents or solutions. Stringent hybridization conditions are readily ascertained by those having ordinary skill in the art.

A "label" or "detectable label" is a moiety or compound joined directly or indirectly to a probe that is detected or leads to a detectable signal. Direct joining may use covalent bonds or non-covalent interactions (*e.g.,* hydrogen bonding, hydrophobic or ionic interactions, and chelate or coordination complex formation) whereas indirect joining may use a bridging moiety or linker (*e.g*., via an antibody or additional oligonucleotide(s), which amplify a detectable signal. Any detectable moiety may be used, *e.g.,* radionuclide, ligand such as biotin or avidin, enzyme, enzyme substrate, reactive group, chromophore such as a dye or particle (*e.g.,* latex or metal bead) that imparts a detectable color, luminescent compound (*e.g.,* bioluminescent, phosphorescent, or chemiluminescent compound such as an acridinium ester ("AE") compound), and fluorescent compound (*i.e.,* fluorophore). A fluorophore may be used in combination with a quencher molecule that absorbs light emitted by the fluorophore when in close proximity to the fluorophore. Detectably labeled probes include, but are not limited to, TaqMan^{™} probes, AE-labeled probes, molecular torches, and molecular beacons.

A "quencher" is a molecule that absorbs light. Quenchers are commonly used in combination with a light emitting label such as a fluorophore to absorb emitted light when in close proximity to the fluorophore. Quenchers are well-known in the art and include, but are not limited to, Black Hole Quencher^{™} (or BHQ^{™}, BHQ-1^{™}, or BHQ-2^{™}), Blackberry Quencher, Dabcyl, QSY, and Tamra^{™} compounds, to name a few.

A "non-extendable" oligomer includes a blocking moiety at or near its 3'-terminus to prevent extension. A blocking group near the 3' end is in some embodiments within five residues of the 3' end and is sufficiently large to limit binding of a polymerase to the oligomer. In other embodiments, a blocking group is covalently attached to the 3' terminus. Suitable blocking groups include, *e.g.,* alkyl groups, non-nucleotide linkers, alkane-diol dideoxynucleotide residues, cordycepin, 3'-deoxy nucleotides, 3'-phosphorylated nucleotides, inverted nucleotides, proteins, peptides, and labels such as fluorophores or quenchers.

Reference to "the sequence of SEQ ID NO:X" refer to the sequence of nucleobases, nucleotides, and/or nucleotide analogs linked together to form a biopolymer. Reference to a sequence by SEQ ID NO: does not connote the identity of the backbone (*e.g.,* RNA, 2'-O-Me RNA, or DNA) or any nucleobase modifications (*e.g.,* methylation of cytosine residues ("5MeC")) unless the context clearly dictates otherwise. In some instances, the sequence of a SEQ ID NO: is followed by the statement "including from [x-y] nucleotide analogs"; it is understood that the nucleotide analogs may be substitutions within the sequence of the SEQ ID NO:, additions to the SEQ ID NO:, or both. Unless the context clearly dictates otherwise, reference to a sequence by SEQ ID NO: includes reference to its complementary sequence (*e.g.,* reference to the sequence 5'-ttagc-3' includes reference to the sequence 5'-gctaa-3').

"Separating" or "purifying" refers to removal of one or more components or a mixture, such as a sample, from one or more other components in the mixture. Sample components include nucleic acids, including target nucleic acids, cellular fragments, proteins, carbohydrates, lipids, and other compounds.. Separating or purifying does not connote any degree of purification. In some embodiments, at least 70%, at least 75%, at least 80%, at least 85%, at least 90%, or at least 95%, of the target nucleic acid or amplified product is separated or removed from other components in the mixture.

"Specificity," in the context of an amplification and/or detection system, refers to the characteristic of the system which describes its ability to distinguish between target and non-target sequences dependent on sequence and assay conditions. In terms of nucleic acid amplification, specificity generally refers to the ratio of the number of specific amplicons produced to the number of side-products (*e.g.,* the signal-to-noise ratio). In terms of detection, specificity generally refers to the ratio of signal produced from target nucleic acids to signal produced from non-target nucleic acids.

"Sensitivity" refers to the precision with which a nucleic acid amplification reaction can be detected or quantitated. The sensitivity of an amplification reaction is generally a measure of the smallest copy number of the target nucleic acid that can be reliably detected in the amplification system, and will depend, for example, on the detection assay being employed, and the specificity of the amplification reaction, *e.g.,* the ratio of specific amplicons to side-products.

### DETAILED DESCRIPTION

Provided herein are compositions (including amplification oligomer combinations), kits, formulations, and methods for amplifying and/or detecting SARS-CoV-2 nucleic acid from a sample. In some embodiments, the sample is a biological sample. The compositions, kits, and methods provide oligonucleotides and oligonucleotide sequences that target the ORF1ab gene sequence of SARS-CoV-2 or their complementary sequences. The described oligonucleotides include amplification oligonucleotides, which may include primers, promoter primers, blocked oligonucleotides, and promoter provider oligonucleotides, whose functions have been described previously (*see, e.g.,* US Patent Nos. 4,683,195; 4,683,202; 4,800,159; 5,399,491; 5,554,516; 5,824,518; and 7,374,885; each incorporated by reference herein). The described oligonucleotides as also include probes for detecting amplified sequences of SARS-CoV-2, and target capture oligonucleotides for capture of SARS-CoV-2 target nucleic acid.

The methods provide for sensitive and specific detection of SARS-CoV-2 nucleic acids. The methods include performing a nucleic acid amplification of a SARS-CoV-2 target region, and detecting the amplified product by, for example, specifically hybridizing the amplified product with a nucleic acid detection probe that provides a signal to indicate the presence of SARS-CoV-2 in the sample. The amplification step includes contacting the sample with one or more amplification oligomers specific for a target sequence in a SARS-CoV-2 target nucleic acid to produce an amplified product if SARS-CoV-2 nucleic acid is present in the sample. Amplification synthesizes additional copies of the target sequence or its complement by using at least one nucleic acid polymerase and at least one amplification oligomer to produce the copies from a template strand (*e.g.,* by extending the sequence from a primer using the template strand). In some embodiments, detecting the amplified product comprises a hybridizing step that includes contacting the amplified product with at least one detection probe oligomer specific for a sequence amplified by the selected amplification oligomers, e.g., a sequence contained in the target sequence and flanked target sequences to which the amplification oligomers hybridize.

Described are amplification oligomer combinations and probes for amplifying and detecting SARS-CoV-2. In some embodiments, an amplification oligomer combination includes at least two primers configured for amplifying a target nucleic acid sequence. In some embodiments, an amplification oligomer combination includes a first primer (*e.g.,* NT7 primer or forward primer) and a second primer (*e.g.,* promoter primer or T7 primer or reverse primer). One skilled in the art will understand that at least one primer comprises a target hybridizing sequence in the sense orientation and at least one primer comprises a target hybridizing sequence in the antisense orientation relative to the target nucleic acid. The primers are configured such that the antisense primer is situated downstream of the sense primer and the sense primer is situated downstream of the antisense primer *(i.e.,* the at least two primers are configured such that they flank the target region to be amplified and prime polymerization in the direction of the other primer, thereby amplifying the region between the two primers). In some embodiments, compositions, formulations, reaction mixtures, and kits comprising amplification oligomer combinations and corresponding probes for amplifying and detecting at least two regions of SARS-CoV-2 are described.

An amplification and/or detection methods in accordance with the present disclosure can further include the step of obtaining the sample to be subjected to subsequent steps of the method. In some embodiments, "obtaining" a sample to be used includes, for example, receiving the sample at a testing facility or other location where one or more steps of the method are performed, and/or retrieving the sample from a location (*e.g.,* from storage or other depository) within a facility where one or more steps of the method are performed.

In some embodiments, the compositions, formulations, kits, and methods provide for exponential amplification of a SARS-CoV-2 target sequence in an *in vitro* amplification reaction that utilizes at least two amplification oligomers that flank a target region to be amplified. The amplification reaction can be cycled or isothermal.

In some aspects, the compositions are configured to specifically hybridize to SARS-CoV-2 nucleic acid with minimal cross-reactivity to one or more non-SARS-CoV-2 pathogens. In some aspects, the compositions are configured to specifically hybridize to SARS-CoV-2 nucleic acid with minimal cross-reactivity to one or more non-SARS-CoV-2 pathogens listed in Table 5 *(see* Example 4, *infra*)*.* In other, non-mutually exclusive aspects, the compositions are configured to specifically hybridize to SARS-CoV-2 nucleic acid with minimal cross-reactivity to one or more coronavirus pathogens selected from OC43, HKU1, NL63, and 229E. In some aspects, the compositions are part of a multiplex system that further includes components and methods for detecting one of more pathogens that are not SARS-CoV-2 (*e.g.,* one or more non-SARS-CoV-2 pathogens such as, for example, one or more of the pathogens listed in Table 5).

In some aspects, there are provided methods for utilizing an oligomer or oligomer combination as described herein. Any method disclosed herein is also to be understood as a disclosure of corresponding uses of materials involved in the method directed to the purpose of the method. Any method disclosed herein is also understood to be a disclosure of the oligonucleotides and compositions used in the method. Any of the oligomers comprising a SARS-CoV-2 target-hybridizing sequence and any combinations (*e.g.,* kits and compositions) comprising such an oligomer are to be understood as also disclosed for use in detecting or quantifying SARS-CoV-2 and for use in the preparation of a composition for detecting or quantifying SARS-CoV-2.

Broadly speaking, the methods may comprise one or more of the following components: target capture, in which SARS-CoV-2 nucleic acid (*e.g.,* from a sample, such as a clinical sample), if present, is annealed to a target capture oligonucleotide; isolation, *e.g.,* washing, to remove material not associated with the target capture oligonucleotide; amplification; and amplicon detection, *e.g.,* amplicon quantification, which may be performed in real time with amplification. In some embodiments, the methods involve each of the foregoing steps. In some embodiments, the amplification comprises exponential amplification, optionally with a preceding linear amplification step (e.g., biphasic amplification). In some embodiments, amplification comprises exponential amplification and amplicon detection. In some embodiments, the methods involve any two of the components listed above. In some embodiments, the method involve any two components listed adjacently above, e.g., washing and amplification, or amplification and detection.

In some embodiments, amplifying a SARS-CoV-2 target sequence utilizes an *in vitro* amplification reaction using at least two amplification oligomers that flank a target region to be amplified. Particularly suitable oligomer combinations for amplification of SARS-CoV-2 target regions are described herein. In some embodiments, an oligomer combination includes at least two amplification oligomers for amplifying a first target region of SARS-CoV-2 target nucleic acid corresponding to a region of SEQ ID NO:1 from about nucleotide position 1141 to about nucleotide position 1235. The at least two amplification oligomers for amplifying the first target region of SARS-CoV-2 target nucleic acid can include a first SARS-CoV-2-specific amplification oligomer and a second first SARS-CoV-2-specific amplification oligomer. In some embodiments, the first SARS-CoV-2-specific amplification oligomer comprises a first SARS-CoV-2-specific target-hybridizing sequence that is from 18 to 27 contiguous nucleotides in length, is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and a second SARS-CoV-2-specific amplification oligomer comprises a second SARS-CoV-2-specific target-hybridizing sequence that is from 18 to 23 contiguous nucleotides in length, is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88. In some embodiments, an oligomer combination includes at least two amplification oligomers for amplifying a second target region of SARS-CoV-2 target nucleic acid corresponding to a region of SEQ ID NO:1 from about nucleotide position 4642 to about nucleotide position 4744. The least two amplification oligomers for amplifying the second target region of SARS-CoV-2 target nucleic acid can include a first SARS-CoV-2-specific amplification oligomer and a second first SARS-CoV-2-specific amplification oligomer. In some such embodiments, the first SARS-CoV-2-specific amplification oligomer comprises a first SARS-CoV-2-specific target-hybridizing sequence that is from 20 to 23 contiguous nucleotides in length, is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and the second SARS-CoV-2-specific amplification oligomer comprises a second SARS-CoV-2-specific target-hybridizing sequence that is 16 to 27 contiguous nucleotides in length, is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113. In some embodiments, both the first and second SARS-CoV-2 target regions are amplified in a multiplex format. In some embodiments, one or both of the first and second SARS-CoV-2 target regions and an internal control target region are amplified in a multiplex format. Exemplary amplification oligomers for amplifying the first and/or second SARS-CoV-2 target regions are listed in Table 39, *infra,* and particular combinations of first and second amplification oligomers for each of the first and second target regions are set forth herein (*see* Embodiments section, *supra,* and Examples 2-13, *infra*)*.*

### SARS-CoV-2 region 1 primers and probes

In some embodiments, a first SARS-CoV-2 region 1-specific primer (amplification oligomer; *e.g.,* forward primer, non-T7 primer, or NT7 primer) comprises a target hybridizing region 18-27 nucleobases in length, wherein the target hybridizing region is contained within SEQ ID NO: 26 or an RNA equivalent or a DNA/RNA chimeric thereof and contains the nucleotide sequence of SEQ ID NO: 27 or 28 or an RNA equivalent or a DNA/RNA chimeric thereof. In some embodiments, a first SARS-CoV-2 region 1-specific primer comprises a target hybridizing region 18-27 nucleobases in length, wherein the target hybridizing region is contained within SEQ ID NO: 26 or an RNA equivalent or a DNA/RNA chimeric thereof and contains a nucleotide sequence differing by no more than 0, 1, 2, 3, 4, or 5 nucleotides from the nucleotide sequence of SEQ ID NO: 27 or 28 or an RNA equivalent or a DNA/RNA chimeric thereof. In some embodiments, the first SARS-CoV-2 region 1-specific primer comprises the nucleotide sequence of SEQ ID NO: 12, 18, 19, 20, or 21, or an RNA equivalent or a DNA/RNA chimeric thereof. In some embodiments, the first SARS-CoV-2 region 1-specific primer consists essentially of the nucleotide sequence of SEQ ID NO: 12, 18, 19, 20, or 21 or an RNA equivalent or a DNA/RNA chimeric thereof. In some embodiments, the first SARS-CoV-2 region 1-specific primer consists of the nucleotide sequence of SEQ ID NO: 12, 18, 19, 20, or 21 or an RNA equivalent or a DNA/RNA chimeric thereof. In some embodiments, the first SARS-CoV-2 region 1-specificprimer comprises a nucleotide sequence having no more than 0, 1, 2, 3, 4, or 5 mismatches from the nucleotide sequence of SEQ ID NO: 12, 18, 19, 20, or 21 or an RNA equivalent or a DNA/RNA chimeric thereof. In some embodiments, the first SARS-CoV-2 region 1-specific primer comprises a nucleotide sequence having at least 80%, at least 85% at least 90%, or at least 95% homology to the nucleotide sequence of SEQ ID NO: 12, 18, 19, 20, or 21 or an RNA equivalent or a DNA/RNA chimeric thereof. A first SARS-CoV-2 region 1-specific primer can have 1 or more modified nucleotides or nucleotide analogs. In some embodiments, the first SARS-CoV-2 region 1-specific primer is an NT7 primer.

In some embodiments, a second SARS-CoV-2 region 1-specific primer (reverse primer, promoter primer or T7 primer) comprises a target hybridizing region 18-23 nucleobases in length, wherein the target hybridizing region is contained within SEQ ID NO: 85 or an RNA equivalent or a DNA/RNA chimeric thereof and contains the nucleotide sequence of SEQ ID NO: 86, 87, or 88 or an RNA equivalent or a DNA/RNA chimeric thereof. In some embodiments, a second SARS-CoV-2 region 1-specific primer comprises a target hybridizing region 18-23 nucleobases in length, wherein the target hybridizing region is contained within SEQ ID NO: 85 or an RNA equivalent or a DNA/RNA chimeric thereof and contains a nucleotide sequence differing by no more than 0, 1, 2, 3, 4, or 5 nucleotides from the nucleotide sequence of SEQ ID NO: 86, 87, 88 or an RNA equivalent or a DNA/RNA chimeric thereof. In some embodiments, the second SARS-CoV-2 region 1-specific primer comprises the nucleotide sequence of SEQ ID NO: 13, 71, 72, 73, 74, or 75 an RNA equivalent or a DNA/RNA chimeric thereof. In some embodiments, the second SARS-CoV-2 region 1-specific primer consists essentially of the nucleotide sequence of SEQ ID NO: 13, 71, 72, 73, 74, or 75 or an RNA equivalent or a DNA/RNA chimeric thereof. In some embodiments, the second SARS-CoV-2 region 1-specific primer consists of the nucleotide sequence of SEQ ID NO: 13, 71, 72, 73, 74, or 75 or an RNA equivalent or a DNA/RNA chimeric thereof. In some embodiments, the second SARS-CoV-2 region 1-specific primer comprises a nucleotide sequence having no more than 0, 1, 2, 3, 4, or 5 mismatches from the nucleotide sequence of SEQ ID NO: 13, 71, 72, 73, 74, or 75 or an RNA equivalent or a DNA/RNA chimeric thereof. In some embodiments, the second SARS-CoV-2 region 1-specific primer comprises a nucleotide sequence having at least 80%, at least 85% at least 90%, or at least 95% homology to the nucleotide sequence of SEQ ID NO: 13, 71, 72, 73, 74, or 75 or an RNA equivalent or a DNA/RNA chimeric thereof. A second SARS-CoV-2 region 1-specific primer can have 1 or more modified nucleotides or nucleotide analogs. In some embodiments, two different second SARS-CoV-2 region 1 primers are used in a TMA reaction.

In some embodiments, a second SARS-CoV-2 region 1 primers comprises a SARS-CoV-2 region 1 promoter primer. In some embodiments, the SARS-CoV-2 region 1 promoter primer comprises the nucleotide sequence of SEQ ID NO: 57, 58, 59, 60, 61, or 62 or an RNA equivalent or a DNA/RNA chimeric thereof. In some embodiments, the SARS-CoV-2 region 1 promoter primer consists essentially of the nucleotide sequence of SEQ ID NO: 57, 58, 59, 60, 61, or 62 or an RNA equivalent or a DNA/RNA chimeric thereof. In some embodiments, the SARS-CoV-2 region 1 promoter primer consists of the nucleotide sequence of SEQ ID NO: 57, 58, 59, 60, 61, or 62 or an RNA equivalent or a DNA/RNA chimeric thereof. In some embodiments, the SARS-CoV-2 region 1 promoter primer comprises a nucleotide sequence having no more than 0, 1, 2, 3, 4, or 5 mismatches from the nucleotide sequence of SEQ ID NO: 57, 58, 59, 60, 61, or 62 or an RNA equivalent or a DNA/RNA chimeric thereof. In some embodiments, the SARS-CoV-2 region 1 promoter primer comprises a nucleotide sequence having at least 80%, at least 85% at least 90%, or at least 95% homology to the nucleotide sequence of SEQ ID NO: 57, 58, 59, 60, 61, or 62 or an RNA equivalent or a DNA/RNA chimeric thereof. A SARS-CoV-2 region 1 promoter primer can have 1 or more modified nucleotides or nucleotide analogs. In some embodiments, two different SARS-CoV-2 region 1 promoter primers are used in a TMA reaction.

In some embodiments, a SARS-CoV-2 region 1 probe comprises a target hybridizing region 19-27 nucleobases in length, wherein the target hybridizing region is contained within SEQ ID NO: 50 or a RNA equivalent, a DNA/RNA chimeric, and/or a complement thereof and contains the nucleotide sequence of SEQ ID NO: 51, 52, 53, or 114 or a RNA equivalent, a DNA/RNA chimeric, and/or a complement thereof. In some embodiments, a SARS-CoV-2 region 1 probe comprises a target hybridizing region 19-27 nucleobases in length, wherein the target hybridizing region is contained within SEQ ID NO: 50 or a RNA equivalent, a DNA/RNA chimeric, and/or a complement thereof and contains a nucleotide sequence differing by no more than 0, 1, 2, 3, 4, or 5 nucleotides from the nucleotide sequence of SEQ ID NO: 51, 52, 53, or 114 or a RNA equivalent, a DNA/RNA chimeric, and/or a complement thereof. In some embodiments, the SARS-CoV-2 region 1 probe comprises the nucleotide sequence of SEQ ID NO: 14, 32-41, 103, 104, 107, or 108 or a DNA or RNA equivalent, a DNA/RNA chimeric, and/or a complement thereof. In some embodiments, the SARS-CoV-2 region 1 probe consists essentially of the nucleotide sequence of SEQ ID NO: 14, 32-41, 103, 104, 107, or 108 or a DNA or RNA equivalent, a DNA/RNA chimeric, and/or a complement thereof. In some embodiments, the SARS-CoV-2 region 1 probe consists of the nucleotide sequence of SEQ ID NO: 14, 32-41, 103, 104, 107, or 108 or a DNA or RNA equivalent, a DNA/RNA chimeric, and/or a complement thereof. In some embodiments, the SARS-CoV-2 region 1 probe comprises a nucleotide sequence having no more than 0, 1, 2, 3, 4, or 5 mismatches from the nucleotide sequence of SEQ ID NO: 14, 32-41, 103, 104, 107, or 108 or a DNA or RNA equivalent, a DNA/RNA chimeric, and/or a complement thereof. In some embodiments, the SARS-CoV-2 region 1 probe comprises a nucleotide sequence having at least 80%, at least 85% at least 90%, or at least 95% homology to the nucleotide sequence of SEQ ID NO: 14, 32-41, 103, 104, 107, or 108 or a DNA or RNA equivalent, a DNA/RNA chimeric, and/or a complement thereof. A SARS-CoV-2 region 1 probe can have 1 or more modified nucleotides or nucleotide analogs.

In some embodiments, a SARS-CoV-2 region 1 probe comprises a SARS-CoV-2 region 1 torch. In some embodiments, the SARS-CoV-2 region 1 torch comprises the nucleotide sequence of SEQ ID NO: 103 or 104 or a DNA equivalent, a DNA/RNA chimeric, and/or a complement thereof. In some embodiments, the SARS-CoV-2 region 1 torch consists essentially of the nucleotide sequence of SEQ ID NO: 103 or 104 or a DNA equivalent, a DNA/RNA chimeric, and/or a complement thereof. In some embodiments, the SARS-CoV-2 region 1 torch consists of the nucleotide sequence of SEQ ID NO: 103 or 104 or a DNA equivalent, a DNA/RNA chimeric, and/or a complement thereof. In some embodiments, the SARS-CoV-2 region 1 torch comprises a nucleotide sequence having no more than 0, 1, 2, 3, 4, or 5 mismatches from the nucleotide sequence of SEQ ID NO: 103 or 104 or a DNA equivalent, a DNA/RNA chimeric, and/or a complement thereof. In some embodiments, the SARS-CoV-2 region 1 torch comprises a nucleotide sequence having at least 80%, at least 85% at least 90%, or at least 95% homology to the nucleotide sequence of SEQ ID NO: 103 or 104 or a DNA equivalent, a DNA/RNA chimeric, and/or a complement thereof. A SARS-CoV-2 region 1 torch can have 1 or more modified nucleotides or nucleotide analogs.

A SARS-CoV-2 first amplification oligomer combination includes at least one first primer and at least 1 second primer as described above. In some embodiments, a SARS-CoV-2 region 1 first amplification oligomer combination includes at least one first primer and at least 2 different second primers.

### SARS-CoV-2 Region 2 primers and probes

In some embodiments, a first SARS-CoV-2 region 2-specific primer ((amplification oligomer; *e.g.,* forward primer, non-T7 primer, or NT7 primer)) comprises a target hybridizing region 20-23 nucleobases in length, wherein the target hybridizing region is contained within SEQ ID NO: 29 or an RNA equivalent or a DNA/RNA chimeric thereof and contains the nucleotide sequence of SEQ ID NO: 30, 31, or 111 or an RNA equivalent or a DNA/RNA chimeric thereof. In some embodiments, a first SARS-CoV-2 region 2-specific primer comprises a target hybridizing region 20-23 nucleobases in length, wherein the target hybridizing region is contained within SEQ ID NO: 29 or an RNA equivalent or a DNA/RNA chimeric thereof and contains a nucleotide sequence differing by no more than 0, 1, 2, 3, 4, or 5 nucleotides from the nucleotide sequence of SEQ ID NO: 30, 31, or 111 or an RNA equivalent or a DNA/RNA chimeric thereof. In some embodiments, the first SARS-CoV-2 region 2-specific primer comprises the nucleotide sequence of SEQ ID NO: 15, 22, 23, 24, or 25 or an RNA equivalent or a DNA/RNA chimeric thereof. In some embodiments, the first SARS-CoV-2 region 2-specific primer consists essentially of the nucleotide sequence of SEQ ID NO: 15, 22, 23, 24, or 25or an RNA equivalent or a DNA/RNA chimeric thereof. In some embodiments, the first SARS-CoV-2 region 2-specific primer consists of the nucleotide sequence of SEQ ID NO: 15, 22, 23, 24, or 25 or an RNA equivalent or a DNA/RNA chimeric thereof. In some embodiments, the first SARS-CoV-2 region 2-specific primer comprises a nucleotide sequence having no more than 0, 1, 2, 3, 4, or 5 mismatches from the nucleotide sequence of SEQ ID NO: 15, 22, 23, 24, or 25 or an RNA equivalent or a DNA/RNA chimeric thereof. In some embodiments, the first SARS-CoV-2 region 2-specific primer comprises a nucleotide sequence having at least 80%, at least 85% at least 90%, or at least 95% homology to the nucleotide sequence of SEQ ID NO: 15, 22, 23, 24, or 25 or an RNA equivalent or a DNA/RNA chimeric thereof. A first SARS-CoV-2 region 2-specific primer can have 1 or more modified nucleotides or nucleotide analogs. In some embodiments, the SARS-CoV-2 region 2 first primer is an NT7 primer.

In some embodiments, a second SARS-CoV-2 region 2-specific primer (reverse primer, promoter primer or T7 primer) comprises a target hybridizing region 16-27 nucleobases in length, wherein the target hybridizing region is contained within SEQ ID NO: 89 or an RNA equivalent or a DNA/RNA chimeric thereof and contains the nucleotide sequence of SEQ ID NO: 90, 1112, or 113 or an RNA equivalent or a DNA/RNA chimeric thereof. In some embodiments, a second SARS-CoV-2 region 2-specific primer comprises a target hybridizing region 16-27 nucleobases in length, wherein the target hybridizing region is contained within SEQ ID NO: 89 or an RNA equivalent or a DNA/RNA chimeric thereof and contains a nucleotide sequence differing by no more than 0, 1, 2, 3, 4, or 5 nucleotides from the nucleotide sequence of SEQ ID NO: 90, 1112, or 113 or an RNA equivalent or a DNA/RNA chimeric thereof. In some embodiments, the second SARS-CoV-2 region 2-specific primer comprises the nucleotide sequence of SEQ ID NO: 16, 76, 77, 79, 80, 81, 82, 83, or 84 or an RNA equivalent or a DNA/RNA chimeric thereof. In some embodiments, the second SARS-CoV-2 region 2-specific primer consists essentially of the nucleotide sequence of SEQ ID NO: 16, 76, 77, 79, 80, 81, 82, 83, or 84 or an RNA equivalent or a DNA/RNA chimeric thereof. In some embodiments, the second SARS-CoV-2 region 2-specific primer consists of the nucleotide sequence of SEQ ID NO: 16, 76, 77, 79, 80, 81, 82, 83, or 84 or an RNA equivalent or a DNA/RNA chimeric thereof. In some embodiments, the second SARS-CoV-2 region 2-specific primer comprises a nucleotide sequence having no more than 0, 1, 2, 3, 4, or 5 mismatches from the nucleotide sequence of SEQ ID NO: 16, 76, 77, 79, 80, 81, 82, 83, or 84 or an RNA equivalent or a DNA/RNA chimeric thereof. In some embodiments, the second SARS-CoV-2 region 2-specific primer comprises a nucleotide sequence having at least 80%, at least 85% at least 90%, or at least 95% homology to the nucleotide sequence of SEQ ID NO: 16, 76, 77, 79, 80, 81, 82, 83, or 84 or an RNA equivalent or a DNA/RNA chimeric thereof. A second SARS-CoV-2 region 2-specific primer can have 1 or more modified nucleotides or nucleotide analogs. In some embodiments, two different second SARS-CoV-2 region 2-specific primer are used in a TMA reaction.

In some embodiments, a second SARS-CoV-2 region 2-specific primer comprises a SARS-CoV-2 region 2 promoter primer. In some embodiments, the SARS-CoV-2 region 2 promoter primer comprises the nucleotide sequence of SEQ ID NO: 63, 64, 65, 66, 67, 68, 69, 70, or 78 or an RNA equivalent or a DNA/RNA chimeric thereof. In some embodiments, the SARS-CoV-2 region 2 promoter primer consists essentially of the nucleotide sequence of SEQ ID NO: 63, 64, 65, 66, 67, 68, 69, 70, or 78 or an RNA equivalent or a DNA/RNA chimeric thereof. In some embodiments, the SARS-CoV-2 region 2 promoter primer consists of the nucleotide sequence of SEQ ID NO: 63, 64, 65, 66, 67, 68, 69, 70, or 78 or an RNA equivalent or a DNA/RNA chimeric thereof. In some embodiments, the SARS-CoV-2 region 2 promoter primer comprises a nucleotide sequence having no more than 0, 1, 2, 3, 4, or 5 mismatches from the nucleotide sequence of SEQ ID NO: 63, 64, 65, 66, 67, 68, 69, 70, or 78 or an RNA equivalent or a DNA/RNA chimeric thereof. In some embodiments, the SARS-CoV-2 region 2 promoter primer comprises a nucleotide sequence having at least 80%, at least 85% at least 90%, or at least 95% homology to the nucleotide sequence of SEQ ID NO: 63, 64, 65, 66, 67, 68, 69, 70, or 78 or an RNA equivalent or a DNA/RNA chimeric thereof. A SARS-CoV-2 region 2 promoter primer can have 1 or more modified nucleotides or nucleotide analogs. In some embodiments, two different SARS-CoV-2 region 2 promoter primers are used in a TMA reaction.

In some embodiments, a SARS-CoV-2 region 2 probe comprises a target hybridizing region 25-29 nucleobases in length, wherein the target hybridizing region is contained within SEQ ID NO: 54 or a RNA equivalent, a DNA/RNA chimeric, and/or a complement thereof and contains the nucleotide sequence of SEQ ID NO: 55, 56, or 115 or a DNA equivalent, a DNA/RNA chimeric, and/or a complement thereof. In some embodiments, a SARS-CoV-2 region 2 probe comprises a target hybridizing region 25-29 nucleobases in length, wherein the target hybridizing region is contained within SEQ ID NO: 54 or a RNA equivalent, a DNA/RNA chimeric, and/or a complement thereof and contains a nucleotide sequence differing by no more than 0, 1, 2, 3, 4, or 5 nucleotides from the nucleotide sequence of SEQ ID NO: 55, 56, or 115 or a RNA equivalent, a DNA/RNA chimeric, and/or a complement thereof. In some embodiments, the SARS-CoV-2 region 2 probe comprises the nucleotide sequence of SEQ ID NO: 17, 43-48, 105, 106, 109, or 110 or a DNA or RNA equivalent, a DNA/RNA chimeric, and/or a complement thereof. In some embodiments, the SARS-CoV-2 region 2 probe consists essentially of the nucleotide sequence of SEQ ID NO: 17, 43-48, 105, 106, 109, or 110 or a DNA or RNA equivalent, a DNA/RNA chimeric, and/or a complement thereof. In some embodiments, the SARS-CoV-2 region 2 probe consists of the nucleotide sequence of SEQ ID NO: 17, 43-48, 105, 106, 109, or 110 or a DNA or RNA equivalent, a DNA/RNA chimeric, and/or a complement thereof. In some embodiments, the SARS-CoV-2 region 2 probe comprises a nucleotide sequence having no more than 0, 1, 2, 3, 4, or 5 mismatches from the nucleotide sequence of SEQ ID NO: 17, 43-48, 105, 106, 109, or 110 or a DNA or RNA equivalent, a DNA/RNA chimeric, and/or a complement thereof. In some embodiments, the SARS-CoV-2 region 2 probe comprises a nucleotide sequence having at least 80%, at least 85% at least 90%, or at least 95% homology to the nucleotide sequence of SEQ ID NO: 17, 43-48, 105, 106, 109, or 110 or a DNA or RNA equivalent, a DNA/RNA chimeric, and/or a complement thereof. A SARS-CoV-2 region 2 probe can have 1 or more modified nucleotides or nucleotide analogs.

In some embodiments, a SARS-CoV-2 region 2 probe comprises a SARS-CoV-2 region 2 torch. In some embodiments, the SARS-CoV-2 region 2 torch comprises the nucleotide sequence of SEQ ID NO: 105 or 106 or a DNA equivalent, a DNA/RNA chimeric, and/or a complement thereof. In some embodiments, the SARS-CoV-2 region 2 torch consists essentially of the nucleotide sequence of SEQ ID NO: 105 or 106 or a DNA equivalent, a DNA/RNA chimeric, and/or a complement thereof. In some embodiments, the SARS-CoV-2 region 2 torch consists of the nucleotide sequence of SEQ ID NO: 105 or 106 or a DNA equivalent, a DNA/RNA chimeric, and/or a complement thereof. In some embodiments, the SARS-CoV-2 region 2 torch comprises a nucleotide sequence having no more than 0, 1, 2, 3, 4, or 5 mismatches from the nucleotide sequence of SEQ ID NO: 105 or 106 or a DNA equivalent, a DNA/RNA chimeric, and/or a complement thereof. In some embodiments, the SARS-CoV-2 region 2 torch comprises a nucleotide sequence having at least 80%, at least 85% at least 90%, or at least 95% homology to the nucleotide sequence of SEQ ID NO: 105 or 106 or a DNA equivalent, a DNA/RNA chimeric, and/or a complement thereof. A SARS-CoV-2 region 2 torch can have 1 or more modified nucleotides or nucleotide analogs.

A SARS-CoV-2 region 2 primer set includes at least one first primer and at least 1 second primer as described above. In some embodiments, a SARS-CoV-2 region 2 primer set includes at least one first primer and at least 2 different second primers.

In some embodiments, the methods further comprise purifying the SARS-CoV-2 target nucleic acid from other components in the sample, *e.g.,* before an amplification, such as before, concurrent with, or after a capture step. Such purification may include methods of separating and/or concentrating organisms contained in a sample from other sample components, or removing or degrading non-nucleic acid sample components, *e.g.,* protein, carbohydrate, salt, lipid, *etc.* In particular embodiments, a target nucleic acid is captured specifically or non-specifically and separated from other sample components. Non-specific target capture methods may involve selective precipitation of nucleic acids from a substantially aqueous mixture, adherence of nucleic acids to a support that is washed to remove other sample components, or other means of physically separating nucleic acids from a mixture that contains SARS-CoV-2 nucleic acid and other sample components.

Target capture typically occurs in a solution phase mixture that contains one or more target capture oligonucleotides (TCO) that hybridize to the SARS-CoV-2 target sequence under hybridizing conditions. In some embodiments, a TCO comprises an immobilized capture probe-binding region, In some embodiments comprising a TCO having an immobilized capture probe-binding region, the SARS-CoV-2-target:TCO complex is captured by adjusting the hybridization conditions so that the immobilized capture probe-binding region hybridizes to an immobilized probe. In some embodiments, the immobilized probe is bound to a particulate solid support, such as paramagnetic beads. Selective and non-specific target capture methods are also described, *e.g.,* in US Patent No. 6,110,678 and International Patent Application Pub. No. WO 2008/016988, each incorporated by reference herein. Exemplary SARS-CoV-2-specific target capture oligonucleotides are listed in Table 39, *infra,* and are further set forth in the Embodiments section, *supra,* and Examples 10 and 11, *infra.*

Isolation can follow capture, where, for example, the complex on the solid support is separated from other sample components. Isolation can be accomplished by any appropriate technique, *e.g.,* washing a support associated with the SARS-CoV-2 target-sequence one or more times (*e.g.,* two or three times) to remove other sample components and/or unbound oligomer. In embodiments using a particulate solid support, such as paramagnetic beads, particles associated with the SARS-CoV-2 target may be suspended in a washing solution and retrieved from the washing solution, in some embodiments by using magnetic attraction. To limit the number of handling steps, the SARS-CoV-2 target nucleic acid may be amplified by simply mixing the target sequence in the complex on the support with amplification oligomers and proceeding with amplification steps.

Sample preparation may also include pooling a plurality of samples into a single pooled batch. Preferably for pooling, an aliquot of each sample is pooled into the larger batch. The larger batch of pooled samples can be from a plurality of samples wherein the plurality is from 2 to about 200 individual samples.

### SARS-CoV-2 target capture oligonucleotides

In some embodiments, a SARS-CoV-2 target capture oligonucleotide comprises a target hybridizing region 21-24 nucleobases in length, wherein the target hybridizing region comprises the nucleotide sequence of SEQ ID NO: 97, 98, 99, 100, 101, or 104 or a DNA equivalent, a DNA/RNA chimeric, and/or complement thereof. In some embodiments, a SARS-CoV-2 target capture oligonucleotide comprises a target hybridizing region 21-24 nucleobases in length, wherein the target hybridizing region comprises a nucleotide sequence differing by no more than 0, 1, 2, 3, 4, or 5 nucleotides from the nucleotide sequence of SEQ ID NO: 97, 98, 99, 100, 101, or 104 or a DNA equivalent, a DNA/RNA chimeric, and/or complement thereof. In some embodiments, the SARS-CoV-2 target capture oligonucleotide comprises the nucleotide sequence of SEQ ID NO: 97, 98, 99, 100, 101, or 104 or a DNA equivalent or a DNA/RNA chimeric thereof. In some embodiments, the target hybridizing region of the SARS-CoV-2 target capture oligonucleotide consists essentially of the nucleotide sequence of SEQ ID NO: 97, 98, 99, 100, 101, or 104 or a DNA equivalent or a DNA/RNA chimeric thereof. In some embodiments, the target hybridizing region of the SARS-CoV-2 target capture oligonucleotide consists of the nucleotide sequence of SEQ ID NO: 97, 98, 99, 100, 101, or 104 or a DNA equivalent or a DNA/RNA chimeric thereof. In some embodiments, the SARS-CoV-2 target capture oligonucleotide comprises a nucleotide sequence having no more than 0, 1, 2, 3, 4, or 5 mismatches from the nucleotide sequence of SEQ ID NO: 228, 97, 98, 99, 100, 101, or 104 or a DNA equivalent or a DNA/RNA chimeric thereof. In some embodiments, the SARS-CoV-2 target capture oligonucleotide comprises a nucleotide sequence having at least 80%, at least 85% at least 90%, or at least 95% homology to the nucleotide sequence of SEQ ID NO: 97, 98, 99, 100, 101, or 104 or a DNA equivalent or a DNA/RNA chimeric thereof. A SARS-CoV-2 target capture oligonucleotide can have 1 or more modified nucleotides or nucleotide analogs.

In some embodiments, a SARS-CoV-2 region 1 target capture oligonucleotide is linked to a TₙAₘ sequence, wherein n in an integer from 0 to 3 and m in an integer from 14 to 50. In some embodiments, n is 3 and m is 30. In some embodiments, an influenza target capture oligonucleotide comprises the nucleotide sequence of SEQ ID NO: 91, 92, 93, 94, 95, or 96 or a DNA equivalent or a DNA/RNA chimeric thereof. In some embodiments, an influenza target capture oligonucleotide consists essentially of the nucleotide sequence of SEQ ID NO: 91, 92, 93, 94, 95, or 96 or a DNA equivalent or a DNA/RNA chimeric thereof. In some embodiments, an influenza target capture oligonucleotide consists of the nucleotide sequence of SEQ ID NO: 91, 92, 93, 94, 95, or 96 or a DNA equivalent or a DNA/RNA chimeric thereof. In some embodiments, an influenza target capture oligonucleotide comprises a nucleotide sequence having no more than 0, 1, 2, 3, 4, or 5 mismatches from the nucleotide sequence of SEQ ID NO: 91, 92, 93, 94, 95, or 96 or a DNA equivalent or a DNA/RNA chimeric thereof. In some embodiments, an influenza target capture oligonucleotide comprises a nucleotide sequence having at least 80%, at least 85% at least 90%, or at least 95% homology to the nucleotide sequence of SEQ ID NO: 91, 92, 93, 94, 95, or 96 or a DNA equivalent or a DNA/RNA chimeric thereof. A SARS-CoV-2 region 1 promoter primer can have 1 or more modified nucleotides or nucleotide analogs.

In some embodiments, the compositions, formulations, or kits contain one or more positive controls. The positive control can be a nucleic acid containing a target sequence for SARS-CoV-2 region 1, SARS-CoV-2 region 2, SARS-CoV-2 region 3, influenza A region 1, influenza A region 2, influenza B region 1, or influenza B region 2. The positive control can be, but is not limited to DNA, RNA, plasmid, or an *in vitro* transcript

A detection step may be performed using any of a variety of known techniques to detect a signal specifically associated with an amplified target sequence, such as, *e.g.,* by hybridizing the amplification product with a labeled detection probe and detecting a signal resulting from the labeled probe (including from label released from the probe following hybridization in some embodiments). In some embodiments, the labeled probe comprises a second moiety, such as a quencher or other moiety that interacts with the label, such as a fluorophore, as discussed above. The detection step may also provide additional information on the amplified sequence, such as, *e.g.,* all or a portion of its nucleic acid base sequence. Detection may be performed after the amplification reaction is completed or may be performed simultaneously with amplifying the target region, *e.g.,* in real time. In some embodiments, amplified product is detected near or at the end of the amplification step. In embodiments, a linear detection probe is used to provide a signal to indicate hybridization of the probe to the amplified product. One example of such detection uses a luminescentally labeled probe that hybridizes to target nucleic acid. The luminescent label is then hydrolyzed from non-hybridized probe. Detection is performed by chemiluminescence using a luminometer. (*See, e.g.,* International Patent Application Pub. No. WO 89/002476, incorporated by reference herein). In some embodiments, the detection is done in real time. In some embodiments, the detection probe a hairpin probe. A hairpin probe can be, but is not limited to, a molecular beacon, molecular torch, or hybridization switch probe that is labeled with a reporter moiety that is detected when the probe binds to amplified product (*e.g.,* a dual-labeled hairpin probe comprising both a fluorescent label and a quenching moiety). In some embodiments, a hairpin probe is used for real-time detection. In some embodiments, the detection probe is a linear oligomer such as, *e.g.,* an oligomer labeled with both a fluorophore and a quenching moiety (*e.g.,* a TaqMan probe). Such probes may comprise target-hybridizing sequences and non-target-hybridizing sequences. Various forms of such probes have been described previously (*see, e.g.,* US Patent Nos. 5,210,015; 5,487,972; 5,118,801; 5,312,728; 5,925,517; 6,150,097; 6,849,412; 6,835,542; 6,534,274; and 6,361,945; and US Patent Application Pub. Nos. 20060068417A1 and 20060194240A1; each incorporated by reference herein). In some embodiments, a hairpin probe is used for real-time detection. In some embodiments, a linear oligomer is used for real-time detection. Exemplary SARS-CoV-2-specific detection probe oligomers are listed in Table *39, infra,* and are further set forth in the Embodiments section, *supra,* and Examples 2-13, *infra* (including, *e.g.,* their use in combination with first and second SARS-CoV-2-specific amplification oligomers for detection of SARS-CoV-2 target nucleic acid).

In some embodiments, detection is performed at time intervals. Detection can be done by measuring fluorescence at regular time intervals. Time intervals can be, but are not limited to: 1-60 sec, 1-120 sec, 1-180 sec, 1-240 sec, or 1-300 sec. In some embodiments, the time interval is 5, 10, 15, 20, 25, 30, 35, 40, 45, 50, 55, or 60 sec. For detection performed at regular time intervals, each interval is referred to as a cycle. Detection can be performed for 20-240 cycles, 30-210 cycles, 40-180 cycles, 50-150 cycles, or 60-120 cycles. For example, detection every 30 sec for 60 minutes constitutes 120 cycles. Detection may occur at the beginning or end of a cycle. Detection can also be performed continuously.

### Multiplex amplification and/or detection

A "multiplex" amplification reaction, such as an isothermal amplification reaction, is characterized in that two or more different amplification products, or amplicons, are generated by means of using two or more amplification oligomer combinations in the same amplification reaction. A multiplex amplification reaction includes two or more amplification oligomer combinations (*e.g.,* two or more first (NT7) primer and second (promoter) primers sets) for amplifying two target sequences. A multiplex amplification system comprises a composition, formulations, reaction mix, or kit for performing a multiplex amplification and/or detection reaction. In addition to amplification oligomer combinations, a multiplex amplification system can further comprise two or more probes for detecting the corresponding amplicons, and/or two or more TCOs.

In some embodiments, a multiplex amplification system for amplifying and/or detecting the presence or absence of SARS-CoV-2 comprises two or more amplification oligomer combinations selected from the group consisting of:
(a) a first amplification oligomer combination for amplifying a target sequence in a first region (region 1) of SARS-CoV-2 ;
(b) a second amplification oligomer combination for amplifying a target sequence in a second region (region 2) of SARS-CoV-2;
(c) a control amplification oligomer combination for amplifying a target sequence in control target nucleic acid; and
(d) one or more amplification oligomer combinations for amplifying one more target sequence in one or more non-SARS-CoV-2 microorganisms (*e.g.,* non-SARS-CoV-2 pathogens).
Any of the above multiplex amplification systems can further comprise probes for detecting amplicons generated by the amplification oligomer combinations and/or one or more TCO for capturing a SARS-CoV-2 target nucleic acid or internal control target nucleic acid.

The probes utilized in a multiplex amplification system can be labeled such that any one probe species (or probe for detecting a species) can be distinguished from other probe species (or probes for detecting other species) in a multiplex detection assay. In some embodiments, a probe for detecting one or both SARS-CoV-2 amplicons can be distinguished from a probe for detecting an internal control amplicon. In multiplex amplification reactions having amplification oligomer combinations for amplifying two different regions of SARS-CoV-2 target nucleic acid, the probes for detecting the SARS-CoV-2 amplicons can utilize the same or different labels. The labels can be, but are not limited to, fluorophores, and fluorophore/quencher combinations (*i.e.,* FRET hybridization probes as described in Matthews and Kricka, Analytical Biochemistry, vol. 169 (1988), pp: 1-25)).

Compositions, formulations, kits, and methods for detection of the SARS-CoV-2 nucleic acid may optionally include oligonucleotides for amplification and/or detection of a non-SARS-CoV-2 internal control (IC) nucleic acid. The control is amplified and detected in the same assay reaction mixtures or a parallel assay reaction mixture by using amplification and detection oligomers specific for the IC sequence. IC nucleic acid sequences can be, *e.g.,* a DNA plasmid, an RNA template sequence (*e.g.,* an *in vitro* transcript), or a synthetic nucleic acid that is spiked into a sample. Alternatively, the IC nucleic acid sequence may be a cellular component, which may be from exogenous cellular sources or endogenous cellular sources relative to the specimen. In these instances, an internal control nucleic acid is co-amplified with the SARS-CoV-2 nucleic acid in the amplification reaction mixtures. The internal control amplification product and the SARS-CoV-2 target sequence amplification product can be detected independently.

In certain embodiments, amplification and detection of a signal from an amplified IC sequence demonstrates that the assay reagents, equipment, conditions, and performance of assay steps were functioning and properly used in the assay if no signal is obtained for the intended target SARS-CoV-2 nucleic acid (*e.g.,* samples that test negative for SARS-CoV-2). An IC may also be used as an internal calibrator for the assay when a quantitative result is desired, *i.e.,* the signal obtained from the IC amplification and detection is used to set a parameter used in an algorithm for quantitating the amount of SARS-CoV-2 nucleic acid in a sample based on the signal obtained for an amplified SARS-CoV-2 target sequence. ICs are also useful for monitoring the integrity of one or more steps in an assay. The primers and probe for the IC target sequence are configured and synthesized by using any well-known method provided that the primers and probe function for amplification of the IC target sequence and detection of the amplified IC sequence using substantially the same assay conditions used to amplify and detect the SARS-CoV-2 target sequence. In some embodiments that include a target capture-based purification step, a target capture probe specific for the IC target is included in the assay in the target capture step so that the IC is treated in the assay in a manner analogous to that for the intended SARS-CoV-2 analyte in all of the assay steps.

Described are formulations and kits for determining the presence or absence of SARS-CoV-2 in a sample. In some embodiments, a formulation or kit comprises at least one primer set (amplification oligomer combination) for amplifying a target sequence in SARS-CoV-2. In some embodiments, a formulation or kit comprises at least two primer sets (amplification oligomer combinations) for amplifying two different target sequences in SARS-CoV-2. Any of the formulations or compositions described herein may be provided as an aqueous solution. In some embodiments, a formulation is an aqueous formulation comprising (1) at least two SARS-CoV-2-specific amplification oligomers for amplification of a SARS-CoV-2 target region as described herein and (2) an organic buffer. In some embodiments, a formulation is an aqueous formulation comprising (1) at least four SARS-CoV-2-specific amplification oligomers for amplification of two different SARS-CoV-2 target regions as described herein and (2) an organic buffer. In some embodiments, a formulation or kit further comprises a probe for detecting an amplicon generated by the amplification oligomers. A kit or an aqueous formulation for amplification of a SARS-CoV-2 nucleic acid may include one or more additional components such as, *e.g.,* a DNA polymerase enzyme, a reverse transcriptase enzyme, or a detection probe oligomer. In some embodiments, a formulation is an aqueous formulation comprising (1) a SARS-CoV-2-specific detection probe oligomer as described herein and (2) an organic buffer. A kit or an aqueous formulation comprising one or more detection probe oligomers may include one or more additional components such as, *e.g.,* a surfactant, a DNA polymerase enzyme, a reverse transcriptase enzyme, or at least one amplification oligomer. Particularly suitable surfactants include, for example, polyethylene glycol mono [4-(1,1,3,3-tetramethylbutyl) phenyl] ether and polyoxyethylene sorbitan fatty acid esters (*e.g.,* polysorbate 20, polysorbate 40, or polysorbate 60). In some embodiments, a surfactant in an aqueous detection probe formulation is a non-linear surfactant (*i.e.,* a surfactant having a branched chain structure) such as, for example, a polyoxyethylene sorbitan fatty acid ester (*e.g.,* polysorbate 20, polysorbate 40, or polysorbate 60) or digitonin. A kit or an aqueous formulation as above for amplification or detection of SARS-CoV-2 nucleic acid may further include a bulking agent such as, *e.g.,* trehalose, raffinose, or a combination thereof. In some embodiments, an aqueous formulation as above contains at inorganic salt such as, *e.g.,* magnesium, potassium, or sodium; in some such variations, the concentration of the inorganic salt is 4 mM or less. A particularly suitable organic buffer for an aqueous formulation as above is Tris (2-amino-2-(hydroxymethyl)-1,3-propanediol). In some embodiments, a kit may further contain one or more of: a Target Capture Reagent (TCR), an amplification (AMP) reagent, and a promoter reagent. A kit may further contain one or more of: buffer, enzyme reagent, RNA polymerase, dNTPs, NTPs, Sample Transport Medium, Target Capture Wash Solution, Target Enhancer Reagent, and a reconstitution reagent.

Any of the described aqueous formulations may be aliquoted into, *e.g.,* vials, ampules, multi-well plates, or other containers and dried (*e.g.,* lyophilized) according to procedures known in the art. The dried formulations may be used for long term storage. The dried product typically appears as a powder or cake. The containers are then typically sealed. In some embodiments, the dried formulation aliquots are transferred to wells of a multi-well plate and the multi-well plate is then sealed Methods of preparing such dried formulations from the aqueous formulation, as well as the dried formulations prepared by such methods, are additional aspects of the instant disclosure. In some embodiments, there is provided a dried formulation that enables reconstitution into an aqueous formulation as described herein. Dried formulations for amplification or detection of a SARS-CoV-2 nucleic acid typically contain, in addition to one or more amplification oligomers and/or detection probes as described herein, a bulking agent such as, *e.g.,* trehalose, raffinose, or a combination thereof. In some embodiments, a formulation further comprises an inorganic salt. In some embodiments, the percent mass of the inorganic salt to the mass of the dried formulation is 0.249% or less, 0.222% or less, or 0.195% or less. Methods of preparing an aqueous formulation from a lyophilized formulation as described herein are also encompassed by the instant disclosure; such methods generally include dissolving the dried formulation in a suitable diluent (*e.g.,* an organic buffer or water) to provide a reconstituted formulation.

Also provided are reaction mixtures for determining the presence or absence of a SARS-CoV-2 target nucleic acid in a sample. A reaction mixture in accordance with the present disclosure includes one or both of (1) an oligomer combination as described herein for amplification of a SARS-CoV-2 target nucleic acid and (2) one or more detection probe oligomers as described herein for determining the presence or absence of a SARS-CoV-2 amplification product. The reaction mixture may further include one or more additional components such as, for example, a TCO or a non-specific capture probe, *e.g.,* poly-(k) capture probes as described in US 2013/0209992, which is incorporated herein by reference. For an amplification reaction mixture, the reaction mixture will typically include other reagents suitable for performing *in vitro* amplification such as, *e.g.,* buffers, salt solutions, appropriate nucleotide triphosphates (*e.g.,* dATP, dCTP, dGTP, and dTTP; and/or ATP, CTP, GTP and UTP), and/or enzymes (*e.g.,* a thermostable DNA polymerase, or reverse transcriptase and/or RNA polymerase), and will typically include test sample components, in which a SARS-CoV-2 target nucleic acid may or may not be present. A reaction mixture may include amplification oligomers for only one target region of a SARS-CoV-2 genome, or it may include amplification oligomers for multiple SARS-CoV-2 target regions. In addition, for a reaction mixture that includes a detection probe together with an amplification oligomer combination, selection of amplification oligomers and detection probe oligomers for a reaction mixture are linked by a common target region (*i.e.,* the reaction mixture will include a probe that binds to a sequence amplifiable by an amplification oligomer combination of the reaction mixture). In some embodiments, a reaction mixture comprises an aqueous formulation as described above. In some embodiments, a reaction mixture is reconstituted with water, a reconstitution reagent, or an organic buffer from a dried formulation as described above. In some embodiments, a reaction mixture comprises a TCO and a primer. In some embodiments, the reaction mixture comprises a TCO, a primer set, and a probe.

Also provided herein are kits for practicing the methods as described herein. A kit in accordance with the present disclosure includes one or both of (1) an oligomer combination as described herein for amplification of a SARS-CoV-2 target nucleic acid and (2) one or more detection probe oligomers as described herein for determining the presence or absence of a SARS-CoV-2 amplification product. A kit may further include several optional components such as, for example, a TCO or a capture probe, *e.g.,* poly-(k) capture probe as described in US 2013/0209992. Other reagents that may be present in the kits include reagents suitable for performing *in vitro* amplification such as, *e.g.,* buffers, salt solutions, appropriate nucleotide triphosphates (*e.g.,* dATP, dCTP, dGTP, dTTP; and/or ATP, CTP, GTP and UTP), and/or enzymes (*e.g.,* a thermostable DNA polymerase, or a reverse transcriptase and/or RNA polymerase). Oligomers as described herein may be packaged in a variety of different embodiments, and those skilled in the art will appreciate that the disclosure embraces many different kit configurations. For example, a kit may include amplification oligomers for only one target region of a SARS-CoV-2 genome, or it may include amplification oligomers for multiple SARS-CoV-2 target regions. In addition, for a kit that includes a detection probe together with an amplification oligomer combination, selection of amplification oligomers and detection probe oligomers for a kit are linked by a common target region (*i.e.,* the kit will include a probe that binds to a sequence amplifiable by an amplification oligomer combination of the kit). In some embodiments, the kit further includes a set of instructions for practicing methods in accordance with the present disclosure, where the instructions may be associated with a package insert and/or the packaging of the kit or the components thereof.

The compositions, kits, formulations, reaction mixtures, and methods are further illustrated by the following non-limiting examples.

### EXAMPLES

### Example 1: Real-Time PCR Amplification and Detection of Coronavirus Using Different Combinations of Primers and Probe

This example describes screening experiments testing primer and probe combinations for the real-time PCR amplification and detection of coronavirus. Reactions are generally prepared and performed as presented herein and as follows.

Several primer and probe mixtures (PPR mixes) were prepared in a microfuge tube to include a forward primer, a reverse primer, and a dual labeled hairpin detection probe. The internal control PPR mix comprises 0.625 µM of each primer and 0.5 µM of the probe, while the coronavirus PPR mixes contain 1.25 µM of each of the primers and the probe. These PPR mixes also contain 150 mM of KCl, 10 mM MgCl₂, and were brought to final volume using 10 mM TRIS. The IC detection probe was labeled with Quasar 705 and Black Hole Quencher 2 and each coronavirus ("CoV") detection probe was labeled with FAM and Black Hole Quencher 1 (all available from BioSearch Technologies, Inc., Novato, CA or Glen Research, Inc., Sterling, VA).

An equal volume of the internal control PPR mix (275 µL) was added to each of the coronavirus mixtures (275 µL) to provide 1.25× PPR mixes (550 µL total volume). Amplification and detection reactions were prepared by combining 20 µL of each PPR mix and 5 µL of a target nucleic acid (e.g., a synthetic target nucleic acid or a purified virus) eluate with a 1.25X master mix containing dNTPs dUTP, Taq polymerase, reverse transcriptase and RNase inhibitor. Each of the amplification and detection mixtures was then overlaid with oil. A synthetic coronavirus target nucleic acid was prepared from a stock concentration to provide a concentration of 1,000 copies per reaction in a 5 µL aliquot (200 copies/µL). Dilutions were made into a sample transport media (containing lithium lauryl sulfate (LLS), EDTA, and sodium phosphate). Amplification and detection reactions were set up at 12 reactions per condition; 6 reactions positive for the target nucleic acid and 6 reactions negative for the target nucleic acid. Negative reactions include sample transport media without the coronavirus target nucleic acid. The reactions were performed in real-time using a Panther Fusion system (available from Hologic, Inc., Marlborough MA) by thermal cycling. The data was analyzed using the My Access Tool on the Panther Fusion System and JMP chart analysis.

The resulting amplification curves are evaluated for differences in Ct and RFU signal for the positive samples, and background RFU for the negative samples.

### Example 2: Linearity and Sensitivity Testing of SARS-CoV-2 Primer & Probe Mixes

The individual and combined linearity and sensitivity tests were conducted with *in vitro* transcripts (IVTs suspended in sample transport media (STM) and supplemented with 1,000 HeLa cells/mL). These experiments were carried out in two stages. In the first stage, IVTs (SEQ ID NOs:2 & 3) at 1,000 copies/reaction were tested with the individual primer and probe sets (see Table 1), as well as the combined oligo sets to gather information about their individual and combined performance characteristics of these primer & probe combinations. In the second stage, the linearity and the sensitivity of the combined oligo sets were tested with 7 concentration levels of the IVTs (SEQ ID NOs:2 & 3) ranging from 1×10⁷(1E+7) copies/reaction to 10 copies/reaction.

Two SARS-CoV-2 primer and probe (PPR) mixes were prepared as generally described in Example 1, above. These SARS-CoV-2 PPR mixes included the indicated primers and probes comprising analog nucleotides and varied placement of fluorophores/quenchers, (see Table 1). Quasar 705 (Q705); CalRed 610, Black Hole Quencher 1 (BHQ-1); and Black Hole Quencher 2 (BHQ-2) are available from Biosearch Technologies, Inc., Novato, CA. 5MeC is available from Sigma-Aldrich Corp., St. Louis, MO. Propyne dU is available from Glen Research, Sterling, VA. Positive reactions were set-up as follows: (i) SARS-CoV-2 PPR Mix 1 and IC PPR Mix; (ii) SARS-CoV-2 PPR Mix 5 and IC PPR Mix; and (iii) SARS-CoV-2 PPR Mix 1, SARS-CoV-2 PPR Mix 5, and IC PPR Mix. The negative reaction contained only the IC PPR Mix. Each of the positive reactions and the negative reaction were run in multiples of 6 reactions for stage 1 and multiples of 12 reactions for stage 2.

**Table 1. SARS-CoV-2 Primer & Probe Mixture Sequences.**

| PPR Mix | [Forward Primer | Reverse Primer | \|Detection Probe |
|---|---|---|---|
| Internal Control (IC) PPR Mix | SEQ ID NO:4 | SEQ ID NO:5 | SEQ ID NO:9 |
| | | | 5' Q705 |
| | | | 3'BHQ-2 |
| SARS-CoV-2 PPR Mix 1 | SEQ ID NO:12 | SEQ ID NO:13 | SEQ ID NO:14 |
| | | | 5' CalRed 610 |
| | 5MeC at residues 1 & 9 | | 5MeC at residues 3, 7, 11, 12, 18, 21, & 23 |
| | | | 3' BHQ-2 |
| SARS-CoV-2 PPR Mix 5 | SEQ ID NO:15 | SEQ ID NO:16 | SEQ ID NO:17 |
| | | Propyne dU at residues 5, 10, & 16. | 5' CalRed 610 |
| | | | 5MeC at residues 3, 4, 7, 10, 16, 22, 25, & 27 |
| | | 5MeC at residues 13, 14, 21, & 23 | |
| | | | 3' BHQ-2 |

Table 2 shows the results summary for the individual and combined primer/probe mixtures. The IC reaction showed 100% detection with all tested conditions. No issues with the combined oligo sets were observed. The linearity and the sensitivity data for the combined PPR mixes listed in (iii), above, are summarized in Table 3 and FIG. 1. These results show that 100% detection of both IVTs was seen down to 100 cp/rxn. PCR efficiency was high with an R2 value >0.99 and slope at -3.33. The IC exhibited 100% detection in all dilutions.

**Table 2. Results Summary for Individual and Combined Oligo Sets (Stage 1).**

| Oligo Sets | Reactivity | IVT conc. (cp/rxn) | Ave. Ct | StdDev Ct | Ave. RFU | SteDev RFU | Ave. Tslope | StdDev Tslope |
|---|---|---|---|---|---|---|---|---|
| SARS2 PPR Mix1 | 6/6 | 10,000 | 27.85 | 0.13 | 112,915 | 1,783 | 715 | 174 |
| SARS2 PPR Mix5 | 6/6 | 10,000 | 27.66 | 0.11 | 63,339 | 971 | 649 | 52 |
| SARS2 PPR Mixes 1&5 | 6/6 | 10,000 | 26.81 | 0.09 | 84,401 | 1,034 | 577 | 24 |

**Table 3. IVT Sensitivity Results for the Combined Oligo Sets (Stage 2).**

| IVT conc. (cp/rxn) | Reactivity | Ave. Ct | StdDev Ct | Ave. RFU | SteDev RFU | Ave. Tslope | StdDev Tslope |
|---|---|---|---|---|---|---|---|
| 10,000,000 | 11/12 | 16.13 | 0.09 | 88435 | 2581 | 971 | 142 |
| 1000000 | 12/12 | 19.87 | 0.22 | 85128 | 4271 | 810 | 214 |
| 100000 | 12/12 | 22.59 | 0.14 | 84935 | 2914 | 670 | 47 |
| 10000 | 12/12 | 26.16 | 0.28 | 81974 | 2284 | 782 | 176 |
| 1000 | 12/12 | 29.60 | 0.41 | 74777 | 4026 | 750 | 43 |
| 100 | 12/12 | 32.45 | 0.26 | 62556 | 2939 | 751 | 89 |
| 10 | 12/12 | 36.48 | 0.92 | 29578 | 13083 | 711 | 156 |

### Example 3. Virus Sensitivity experiments in the presence of HeLa cells

A specific virus strain was tested to show sensitivity and linearity of the combined PPR mix containing each of SARS-CoV-2 PPR Mix 1, SARS-CoV-2 PPR Mix 5, and IC PPR Mix from Table 1. Negative reaction was IC PPR Mix only. The virus strain was SARS-Related Coronavirus 2, Isolate USA-WA1/2020 (obtained through BEI Resources, NIAID, NIH, NR-52281). Stock concentration of the virus was 2.8×10⁵ TCID₅₀ after 6 days at 37°C and 5% CO₂. Stock virus was spiked into STM containing 10,000 HeLa cells/mL and diluted to six working concentrations ranging from 14 to 1.4×10⁻⁴ TCID₅₀/mL (Table 4). Positive and negative reactions were run in triplicate. Results for the virus linearity and sensitivity test are shown in Table 4, along with corresponding linearity plot in FIG. 2. These results show that 100% detection (3/3 replicates) was observed for viral strains down to a concentration of 1.4×10⁻³ TCID₅₀/mL. IC was detected under all the tested conditions.

**Table 4. Virus Sensitivity Results for the Combined Oligo Sets.**

| Virus conc. (cp/rxn) | Reactivity | Ave. Ct | StdDev Ct | Ave. RFU | StdDev RFU | Ave. Tslope | StdDev Tslope |
|---|---|---|---|---|---|---|---|
| 14 | 3/3 | 25.20 | 0.08 | 78060 | 1708 | 869 | 61 |
| 1.4 | 3/3 | 27.76 | 0.08 | 78937 | 765 | 579 | 28 |
| 1.4E-1 | 3/3 | 31.40 | 0.21 | 65578 | 604 | 726 | 99 |
| 1.4E-2 | 3/3 | 34.51 | 0.62 | 50532 | 6808 | 565 | 19 |
| 1.4E-3 | 3/3 | 37.23 | 0.99 | 22957 | 12130 | 826 | 76 |
| 1.4E-4 | 0/3 | N/A | N/A | -2 | 24 | N/A | N/A |

### Example 4: Specificity and Interference Experiments (Panels and Specific MERS/SARS Testing)

Cross-reactivity of the combined PPR Mixes was evaluated in 53 microorganisms commonly found in reparatory track infections. These microorganisms were grouped into 16 panels as listed in Table 5 and tested using the combined PPR mix containing each of SARS-CoV-2 PPR Mix 1, SARS-CoV-2 PPR Mix 5, and IC PPR Mix from Table 1. Stock concentrations of each organism were spiked into sample transport media to form a final concentration organism sample. The final concentration of each organism was then pooled into one of 16 different panels, as described in Table 5. 360 µL of each panel was extracted, and 5 µL of the 360 µL eluates was used in the final PCR reaction. 1 extraction and 3 PCR replicates were performed for each panel. IVTs (SEQ ID NOs:2 & 3) at a concentration of 10,000 copies/reaction was used as the positive control. All panels tested were negative for the SARS-CoV-2 using the combined PPR Mixes. Positive control reactions were positive and negative control reactions were negative using the combined PPR Mixes. The specificity results are summarized in Table 6. Interference was also evaluated simultaneously with the specificity experiment. Reactivity for the corresponding IVTs at the concentration of 10,000 copies/reaction was evaluated in the presence of the panels described in Table 5. The results are summarized in Table 6. The IVTs are detected in all the tested panels. None of the microorganisms tested was detected by the SARS-CoV-2 PPR mixes (*i.e.,* the SARS-CoV-2 PPR mixes did not cross-react with any of the challenge organisms). Similarly, none of the microorganism interfered with detection of SARS-CoV-2 target nucleic acid by the PPR mixes.

**Table 5. Summary of the Microorganisms Tested for Specificity and Interference.**

| Panel | Organism | Manufacturer | Strain/ Cell Line | ATTC/PN# | Units | Stock Conc. | Final Conc. |
|---|---|---|---|---|---|---|---|
| 1 | Adenovirus Culture Fluid | ZeptoMetrix | Type 1 | 0810050CF | TCID50/mL | 1.02E+08 | 1.00E+06 |
| | Adenovirus Culture Fluid | ZeptoMetrix | Type 4 | 0810070CF | TCID50/mL | 2.19E+06 | 1.00E+04 |
| | Adenovirus Culture Fluid | ZeptoMetrix | Type 7A | 0810021CF | TCID50/mL | 9.55E+06 | 1.00E+05 |
| | Mumps Virus Culture Fluid | ZeptoMetrix | Isolate 1 | 0810079CF | TCID50/mL | 1.05E+06 | 1.00E+04 |
| 2 | Measles Virus Culture Fluid | ZeptoMetrix | NA | 0810025CF | TCID50/mL | 2.45E+05 | 1.00E+03 |
| | Corynebacterium diphtheriae | ZeptoMetrix | Z116 | CFU/mL | CFU/mL | 4.00E+09 | 1.00E+06 |
| | Cytomegalovirus (CMV) Culture Fluid | ZeptoMetrix | AD-169 | 0810003CF | TCID50/mL | 2.19E+06 | 1.00E+04 |
| | Epstein-Barr Virus (EBV) Culture Fluid | ZeptoMetrix | B95-8 | 0810008CF | cp/mL | 7.70E+07 | 1.00E+06 |
| 3 | Varicella Zoster Virus (VZV) Culture Fluid | ZeptoMetrix | Strain 82 | 0810167CF | cp/mL | 2.03E+10 | 1.00E+08 |
| | Varicella Zoster Virus (VZV) Culture Fluid | ZeptoMetrix | Strain 82 | 0810167CF | cp/mL | 1.00E+08 | 1.00E+06 |
| | Herpes Simplex Virus Type 1 (HSV-1) Culture Fluid | ZeptoMetrix | MacIntyre | 0810005CF | TCID50/mL | 3.80E+06 | 1.00E+04 |
| | Human Herpes Virus Type 6B (HHV-6B) Culture Fluid | ZeptoMetrix | Z29 | 0810072CF | cp/mL | 4.22E+08 | 1.00E+06 |
| | Human Herpes Virus Type 7 (HHV-7) Culture Fluid | ZeptoMetrix | SB | 0810071CF | TCID50/mL | 1.15E+07 | 1.00E+05 |
| 4 | Human Metapneumovirus (hMPV) 20 Type A2 Culture Fluid | ZeptoMetrix | IA14-2003 | 0810163CF | TCID50/mL | 4.07E+07 | 1.00E+04 |
| | Human Metapneumovirus (hMPV) 3 Type B1 Culture Fluid | ZeptoMetrix | Peru2-220 | 0810156CF | TCID50/mL | 3.16E+06 | 1.00E+04 |
| | Human Metapneumovirus (hMPV) 4 Type B2 Culture Fluid | ZeptoMetrix | Perul-2002 | 0810157CF | TCID50/mL | 5.37E+08 | 1.00E+06 |
| | Human Metapneumovirus (hMPV) 9 Type A1 Culture Fluid | ZeptoMetrix | IA3-2002 | 0810160CF | TCID50/mL | 1.58E+09 | 1.00E+06 |
| 5 | Influenza A H1N1 Virus Culture Fluid | ZeptoMetrix | Taiwan/42/0 6 | 0810247CF | TCID50/mL | 1.17E+05 | 1.00E+03 |
| | Influenza B Culture Fluid | ZeptoMetrix | B/Florida/04/ 06 | 0810255CF | TCID50/mL | 1.41E+05 | 1.00E+03 |
| | Respiratory Syncytial Virus Type A (RSV-A) Culture Fluid | ZeptoMetrix | 2006 Isolate | 0810040ACF | TCID50/mL | 1.00E+06 | 1.00E+03 |
| | Respiratory Syncytial Virus Type B (RSV-B) Culture Fluid | ZeptoMetrix | CH93(18)-18 | 0810040CF | TCID50/mL | 7.24E+05 | 1.00E+03 |
| 6 | Parainfluenza Virus Culture Fluid | ZeptoMetrix | Type 1 | 0810014CF | TCID50/mL | 5.01E+05 | 1.00E+03 |
| | Parainfluenza Virus Culture Fluid | ZeptoMetrix | Type 2 | 0810015CF | TCID50/mL | 2.45E+05 | 1.00E+03 |
| | Parainfluenza Virus Culture Fluid | ZeptoMetrix | Type 3 | 0810016CF | TCID50/mL | 5.89E+07 | 1.00E+05 |
| | Parainfluenza Virus Culture Fluid | ZeptoMetrix | Type 4A | 0810060CF | TCID50/mL | 5.01E+05 | 1.00E+03 |
| 7 | Rhinovirus Culture Fluid | ZeptoMetrix | Type B14 | 0810284CF | TCID50/mL | 1.51E+06 | 1.00E+04 |
| | Rhinovirus Culture Fluid | ZeptoMetrix | Type 1A | 0810012CFN | TCID50/mL | 4.17E+05 | 1.00E+03 |
| | Enterovirus Type 68 Culture Fluid | ZeptoMetrix | 2007 Isolate | 0810237CF | TCID50/mL | 2.19E+06 | 1.00E+04 |
| | Enterovirus Type 71 Culture Fluid | ZeptoMetrix | 200E Isolate | 0810236CF | TCID50/mL | 8.51E+07 | 1.00E+05 |
| 8 | E. coli | Hologic | GP1914 | 43653 | CFU/mL | 1.00E+08 | 1.00E+06 |
| | H. influenzae | Hologic | GP1915 | 51654 | CFU/mL | 1.00E+08 | 1.00E+06 |
| | Mycobacterium tuberculosis | ZeptoMetrix | H37Ra-1 | 801660 | TCID50/mL | 6.08E+07 | 1.00E+06 |
| | L. plantarum | Hologic | GP1916 | 8014 | CFU/mL | 1.00E+08 | 1.00E+06 |
| 9 | Legionella pneumophila | Hologic | GP0004 | 33152 | CFU/mL | 1.00E+08 | 1.00E+06 |
| | Bordetella pertussis | Hologic | GP0267 | 8467 | CFU/mL | 1.00E+09 | 1.00E+06 |
| | Mycoplasma pneumoniae | Hologic | GP0100 | 29342 | CFU/mL | 1.00E+08 | 1.00E+06 |
| | Moraxella catarrhalis | Hologic | GP0119 | 25238 | CFU/mL | 1.00E+08 | 1.00E+06 |
| 10 | N. meningiditis | Hologic | GP0755 | 13077 | CFU/mL | 1.00E+09 | 1.00E+06 |
| | N. mucosa | Hologic | GP0190 | 19696 | CFU/mL | 1.00E+08 | 1.00E+06 |
| | P. aeruginosa | Hologic | GP1917 | 19142 | CFU/mL | 1.00E+08 | 1.00E+06 |
| | S. aureus | Hologic | GP0018 | 12600 | CFU/mL | 1.00E+08 | 1.00E+06 |
| 11 | S. epidermidis | Hologic | GP1918 | 35984 | CFU/mL | 1.00E+08 | 1.00E+06 |
| | S. pneumoniae | Hologic | GP1919 | 49619 | CFU/mL | 1.00E+08 | 1.00E+06 |
| | S. pyogenes | Hologic | GP1920 | 19615 | CFU/mL | 1.00E+08 | 1.00E+06 |
| | S. salivarius | Hologic | GP0816 | 13419 | CFU/mL | 1.00E+08 | 1.00E+06 |
| 12 | Pneumocystis jirovecii (PJP)/ P. Carinii | Hologic | S0385 | S0385 | N/A | 1.00E+08 | 1.00E+06 |
| | Chlamydia pneumonia | ATCC; VR-1356 | 2023 | 70019109 | IFU/mL | 4.00E+07 | 1.00E+05 |
| 13 | Human coronavirus 229E | ATCC | N/A | VR-740 | TCID50/mL | 8.90E+06 | 1.00E+05 |
| | Human coronavirus OC43 | ZeptoMetrix | HCT-8 | 0810024CF | TCID50/mL | 1.26E+06 | 1.00E+04 |
| | Human coronavirus NL63 | ZeptoMetrix | Vero | 0810228CF | TCID50/mL | 5.62E+04 | 1.00E+03 |
| 14 | MERS-coronavirus | BEI Resources | EMC/2012 | NR-50549 | TCID50/mL | 8.90E+05 | 1.00E+04 |
| 15 | SARS-coronavirus (IVT) | Hologic | N/A | N/A | Copies/mL | 2.89E+09 | 1.00E+06 |
| 16 | HKU1 (IVT) | Hologic | N/A | N/A | Copies/mL | 2.89E+09 | 1.00E+06 |

**Table 6. Summary of the Specificity and Interference Results.**

| Panels | Specificity | Interference | | | | | | |
|---|---|---|---|---|---|---|---|---|
| | Reactivity | Reactivity | Ave. Ct | StdDev Ct | Ave. RFU | SteDev RFU | Ave. Tslope | StdDev Tslope |
| 1 | 0/3 | 3/3 | 29.97 | 0.13 | 77759 | 1396 | 712 | 246 |
| 2 | 0/3 | 3/3 | 28.82 | 0.09 | 84682 | 643 | 602 | 32 |
| 3 | 0/3 | 3/3 | 29.43 | 0.12 | 78162 | 603 | 713 | 54 |
| 4 | 0/3 | 3/3 | 29.71 | 0.21 | 79299 | 3423 | 604 | 60 |
| 5 | 0/3 | 3/3 | 29.10 | 0.04 | 81778 | 3052 | 913 | 23 |
| 6 | 0/3 | 3/3 | 29.07 | 0.04 | 80113 | 2386 | 971 | 22 |
| 7 | 0/3 | 3/3 | 29.65 | 0.16 | 76887 | 1324 | 626 | 59 |
| 8 | 0/3 | 3/3 | 30.08 | 0.10 | 74341 | 868 | 765 | 220 |
| 9 | 0/3 | 3/3 | 29.22 | 0.09 | 79175 | 2256 | 850 | 79 |
| 10 | 0/3 | 3/3 | 29.09 | 0.04 | 81210 | 2960 | 940 | 27 |
| 11 | 0/3 | 3/3 | 29.01 | 0.13 | 83299 | 3919 | 652 | 198 |
| 12 | 0/3 | 3/3 | 29.79 | 0.24 | 77604 | 2803 | 737 | 207 |
| 13 | 0/3 | 3/3 | 29.82 | 0.16 | 80463 | 2466 | 570 | 49 |
| 14 | 0/3 | 3/3 | 34.59 | 0.45 | 20679 | 5560 | 781 | 140 |
| 15 | 0/3 | 3/3 | 34.04 | 0.31 | 31426 | 679 | 604 | 120 |
| 16 | 0/3 | 3/3 | 33.97 | 0.17 | 29011 | 2160 | 805 | 191 |
| Positive Control | 3/3 | 3/3 | 29.22 | 0.11 | 81078 | 2585 | 835 | 77 |

### Example 5: Isothermal Amplification and Real-Time Detection of SARS-CoV-2 Using Different Combinations of Primers and Probes.

This example describes screening experiments testing several primer and probe combinations for the real-time isothermal amplification and detection of SARS-CoV-2. Reactions were generally prepared and performed as presented herein and as follows.

Sample, lysis, and target capture reagents commonly used with the isothermal assays include the following. Sample transport reagent: 110 mM lithium lauryl sulfate (LLS), 15 mM NaH₂PO₄, 15 mM Na₂HPO₄, 1mM EDTA, 1 mM EGTA, pH 6.7. Lysis buffer: 790 mM HEPES, 230 mM succinic acid, 10% (w/v) LLS, and 680 mM LiOH monohydrate. Target Capture Reagent (TCR): 250 mM HEPES, 1.88 M LiCl, 310 mM LiOH, 100 mM EDTA, pH 6.4, and 250 µg/ml of paramagnetic particles (0.7-1.05 micron particles, Sera-MagTM MG-CM) with (dT)14 oligomers covalently bound thereto. Wash Solution: 10 mM HEPES, 150 mM NaCl, 6.5 mM NaOH, 1 mM EDTA, 0.3% (v/v) ethanol, 0.02% (w/v) methylparaben, 0.01% (w/v) propylparaben, and 0.1% (w/v) sodium lauryl sulfate, pH 7.5. Amplification reagents commonly used with isothermal assays include the following. Amplification Reagent: 125 mM HEPES, 26.7 mM rATP, 33.3 mM rGTP, 5 mM each of rCTP and UTP, 1.33 mM each of dATP, dCTP, dGTP and dTTP, 8% (w/v) trehalose, pH 7.7, to which primers and probes may be added. Real-Time Amplification reagent: 11.61 mM Tris base, 14.94 mM Tris-HCl, 28.5 mM MgCl₂, 23.30 mM KCl, 3.3% Glycerol, 0.02% PRO CLIN 300, 0.05 mM Zinc Acetate Dihydrate, 0.76 mM each of dATP, dCTP, dGTP, and dTTP, 6.50 mM each ATP, CTP, and GTP, 7.50 mM UTP, to which primers and probes may be added. Enzyme reagents commonly used with isothermal assays include the following. Enzyme Reagent: 57.46 mM HEPES, 49.58 mM N-Acetyl-L-Cysteine, 0.98 mM EDTA free acid, 0.039 mM EDTA Disodium Dihydrate, 0.10 v/v TRITON X-100, 49.61 mM KCl, 0.20 v/v Glycerol, 0.03 w/v Trehalose Dihydrate and, per reaction, about 90 U/µl of MMLV reverse transcriptase (RT) and about 20 U/µl of T7 RNA polymerase per reaction (where 1 U of RT incorporates 1 nmol of dTTP in 10 min at 37° C using 200-400 µM oligo-dT-primed poly A-template, and 1 U of T7 RNA polymerase incorporates 1 nmol of ATP into RNA in 1 hr at 37° C using a T7 promoter in DNA template). Reagents commonly used with AE-labeled detection probe reactions include the following. Probe Reagent for Acridinium Ester (AE)-labeled probes: a solution of (a) 100 mM Li-succinate, 3% (w/v) LLS, 10 mM mercaptoethanesulfonate (MES), and 3% (w/v) polyvinylpyrrolidone, or (b) 100 mM Li-succinate, 0.1% (w/v) LLS, and 10 mM MES. Hybridization Reagent: (C-type) 100 mM succinic acid, 2% (w/v) LLS, 100 mM LiOH, 15 mM aldrithiol-2, 1.2 M LiCl, 20 mM EDTA, and 3.0% (v/v) ethanol, pH 4.7. Selection Reagent: 600 mM boric acid, 182.5 mM NaOH, 1% (v/v) octoxynol (TRITON^{®} X-100), pH 8.5 to 9.2, to hydrolyze AE labels on unbound oligomers. Detection Reagents for AE labels are Detect Reagent I: 1 mM nitric acid and 32 mM H₂O₂ and Detect Reagent II: 1.5 M NaOH. (*see also,* US Pat. Nos. 5,283,174, 5,656,744, 5,658,737, 5,888,779, 6,110,678, and 10,196,674 describing the compositions and methods of target capture, isothermal amplification and detection reactions).

Oligo screening experiments for amplification oligos and dual-labeled, hairpin detection probes were performed on an OEM platform (Stratagene Mx3000) using the real-time TMA format. Briefly, Amplification reagent containing T7 promoter primer/non-T7 primer/detection probe combinations were added to a multi-well plate for a total volume of 30 µl per well. Each well was then spiked with 10 µl (for a total reaction volume of 40 µl) of an *in vitro* transcript target nucleic acid (SEQ ID NOs:2 and/or 3) at 1×10⁷ copies of target nucleic acid per reaction and incubated for 5 minutes at 60°C using the Mx3000. After the 5-min incubation, the samples were all transferred to a Thermomixer heating block set at 4°C and incubated for 5 min. The enzyme reagent (10 µl) was then added to each reaction well and incubated for an additional minute with mixing at 1,200 RPM. Following the mixing step, the samples were transferred back to the Mx3000 and a real-time amplification and detection reaction was performed for 120 min taking fluorescent emission reads (Fam/Hex channels) every 30 seconds. Fluorescence emission reflects the dual-labeled hairpin detection probes binding to target amplicon resulting in dye separation from quencher. Fluorescence curve profiles were analyzed for amplification of target.

In an initial set of experiments, sixty combinations of T7 promoter primer, non-T7 primer and dual labeled detection probe oligonucleotide were tested in a real-time TMA amplification and detection reaction as described directly above. Table 7 discloses the sequence and concentration of the various T7 promoter primers tested. Table 8 discloses the sequence and concentration of the various non-T7 primers tested. Table 9 discloses the sequence and concentration of the various dual-labeled hairpin detection probes (torches). These reaction mixtures were each tested singly, and the performance of each combination was recorded in Table 10, below, as TTime. Combination conditions are presented using the Rxn Component IDs in the following order: T7 promoter primer, non-T7 primer, Torch.

**Table 7. T7 Promoter Primers.**

| Rxn Component ID | SEQ ID NO | Stock Oligo Concentration | Final Concentration |
|---|---|---|---|
| 1 | 62 | 35.93 pm/µL | 0.2 pm/µL |
| 2 | 61 | 17.24 pm/µL | 0.2 pm/µL |
| 3 | 60 | 53.16 pm/µL | 0.2 pm/µL |
| 4 | 59 | 46.51 pm/µL | 0.2 pm/µL |
| 5 | 58 | 72.11 pm/µL | 0.2 pm/µL |
| 6 | 57 | 47.63 pm/µL | 0.2 pm/µL |

**Table 8: Non-T7 Primers.**

| Rxn Component ID | SEQ ID NO | Stock Oligo Concentration | Final Concentration |
|---|---|---|---|
| a | 12 | 221.25 pm/µL | 0.4 pm/µL |
| b | 21 | 227.75 pm/µL | 0.4 pm/µL |
| c | 18 | 223.75 pm/µL | 0.4 pm/µL |
| d | 19 | 212.75 pm/µL | 0.4 pm/µL |
| e | 20 | 216.75 pm/µL | 0.4 pm/µL |

**Table 9: Dual-Labeled Hairpin Detection Probes.**

| Rxn Component ID | SEQ ID NO * | Stock Oligo Concentration | Final Concentration |
|---|---|---|---|
| 1 | 104 | 89 pm/µL | 0.6 pm/µL |
| 2 | 103 | 126 pm/µL | 0.6 pm/µL |

| | | | |
|---|---|---|---|
| * The probes were labeled with the fluorophore FAM and the quencher Dabcyl. | | | |

**Table 10: TTimes for each Combination Condition. Combination condition is labeled according to: T7 promoter primer of Table 7 (first digit), Non-T7 Primer of Table 8 (letter), and Probe of Table 9 (final digit).**

| Combination Condition | TTime | Combination Condition | TTime |
|---|---|---|---|
| 1a1 | 9.82 | 1a2 | 15.60 |
| 2a1 | 9.10 | 2a2 | 13.69 |
| 3a1 | 9.20 | 3a2 | 12.16 |
| 4a1 | 15.29 | 4a2 | 18.54 |
| 5a1 | **5.81** | 5a2 | **9.29** |
| 6a1 | **5.38** | 6a2 | **9.49** |
| 1b1 | 9.90 | 1b2 | 16.19 |
| 2b1 | 7.72 | 2b2 | 12.12 |
| 3b1 | 7.32 | 3b2 | 12.54 |
| 4b1 | 6.43 | 4b2 | 10.51 |
| 5b1 | **3.85** | 5b2 | **6.73** |
| 6b1 | **3.79** | 6b2 | **6.12** |
| 1c1 | 16.49 | 1c2 | 15.98 |
| 2c1 | 12.50 | 2c2 | 12.28 |
| 3c1 | 13.88 | 3c2 | 14.12 |
| 4c1 | 10.48 | 4c2 | 10.95 |
| 5c1 | **6.76** | 5c2 | **7.19** |
| 6c1 | **6.14** | 6c2 | **6.22** |
| 1d1 | 16.34 | 1d2 | 15.9 |
| 2d1 | 12.10 | 2d2 | 12.66 |
| 3d1 | 12.90 | 3d2 | 12.47 |
| 4d1 | 10.28 | 4d2 | 9.86 |
| 5d1 | **5.94** | 5d2 | **6.33** |
| 6d1 | **6.28** | 6d2 | **6.31** |
| 1e1 | 14.98 | 1e2 | 19.26 |
| 2e1 | 12.33 | 2e2 | 12.28 |
| 3e1 | 13.29 | 3e2 | 12.43 |
| 4e1 | 10.81 | 4e2 | 10.94 |
| 5e1 | **7.37** | 5e2 | **6.56** |
| 6e1 | **6.09** | 6e2 | **6.17** |

In this experiment, combinations including SEQ ID NO:57 or SEQ ID NO:58 showed a consistently fast TTime. Combinations using either SEQ ID NO:57 or 58 as a T7 promoter provider and SEQ ID NO:12 an a non-T7 primer showed slower TTimes compared combinations containing these same T7 promoter primers. All combinations provided acceptable performance.

### Example 6: Analytical Sensitivity of a Number of Primer and Probe Combinations

In this next example, oligonucleotide combinations each containing one of several T7 promoter primers (Table 11), a single non-T7 primer (Table 12) and a single torch (Table 13) were tested against a dilution of target nucleic acids to measure the sensitivity of these combinations. Reaction mixtures were set up as generally described for Example 5, above, and the reactions were run in triplicate. Target nucleic acids were tested at a dilution from 6.7 log copies down to 1.7 log copies.

**Table 11. T7 Promoter Primer.**

| Rxn Component ID | SEQ ID NO | Stock Oligo Concentration | Final Concentration |
|---|---|---|---|
| 2 | 61 | 17.24 pm/µL | 0.2 pm/µL |
| 4 | 59 | 46.51 pm/µL | 0.2 pm/µL |
| 5 | 58 | 72.11 pm/µL | 0.2 pm/µL |
| 6 | 57 | 47.63 pm/µL | 0.2 pm/µL |

**Table 12. Non-T7 Primer.**

| Rxn Component ID | SEQ ID NO | Stock Oligo Concentration | Final Concentration |
|---|---|---|---|
| b | 21 | 227.75 pm/µL | 0.4 pm/µL |

**Table 13. Torch.**

| Rxn Component ID | SEQ ID NO * | Stock Oligo Concentration | Final Concentration |
|---|---|---|---|
| 1 | 104 | 89 pm/µL | 0.15 pm/µL |

| | | | |
|---|---|---|---|
| * The probe was labeled with FAM as a fluorophore and Dabcyl as a quencher | | | |

Combination conditions and test results are presented in Table 14, below, using the Rxn Component IDs format presented in and described for Table 10.

**Table 14. TTimes for Each Combination Condition.**

| log Copies | 2b1 | 4b1 | 5b1 | 6b1 |
|---|---|---|---|---|
| 0.0 | nd | nd | nd | nd |
| 0.0 | nd | nd | nd | nd |
| 0.0 | nd | nd | nd | nd |
| 1.7 | nd | 24.78 | 13.04 | 13.24 |
| 1.7 | nd | nd | 14.26 | 13.51 |
| 1.7 | nd | 23.94 | 17.00 | 12.88 |
| 2.7 | 23.39 | 19.96 | 12.68 | 11.87 |
| 2.7 | nd | 19.82 | 11.83 | 12.37 |
| 2.7 | nd | 19.05 | 12.42 | 11.47 |
| 3.7 | 21.56 | 17.06 | 10.28 | 10.73 |
| 3.7 | 21.40 | 18.02 | 10.22 | 10.78 |
| 3.7 | 21.10 | 18.37 | 11.23 | 12.86 |
| 4.7 | 16.32 | 14.45 | 9.81 | 9.26 |
| 4.7 | 16.32 | 15.13 | 9.45 | 9.08 |
| 4.7 | 16.27 | 15.48 | 9.59 | 9.30 |
| 5.7 | 14.18 | 12.50 | 8.30 | 7.85 |
| 5.7 | 13.59 | 12.42 | 7.88 | 7.72 |
| 5.7 | 13.75 | 12.42 | 8.14 | 7.78 |
| 6.7 | 15.04 | 11.40 | 6.82 | 6.72 |
| 6.7 | 12.86 | 11.45 | 6.70 | 6.67 |
| 6.7 | 12.61 | 11.13 | 7.06 | 6.65 |

| | | | | |
|---|---|---|---|---|
| nd represents assay conditions that provided no readable TTime | | | | |

In this experiment, all combinations showed good sensitivity, with combinations 5b1 and 6b1 showing good sensitivity down to 1.70 log copies of target nucleic acid per reaction. In a separate experiment (not shown) combination 6b1 was run set up as described in this example but was run on the Panther instrument. In the separate experiment, combination 6b1 showed sensitivity down to 5 copies of target nucleic acid per reaction.

In a further sensitivity experiment, a number of combinations containing 0.2 pm/µL of SEQ ID NO:57 as a promoter primer, 0.15 pm/µL of SEQ ID NO:104 as a torch and 0.4 pm/µL of one of SEQ ID Nos:12, 21, 18, or 20 as non-T7 primers were further tested for sensitivity. The reactions were set up as generally described above, and each condition was run in triplicate. The Combination Condition indicators from Table 10 are used in the data table (Table 15 shows average TTimes for the triplicate reactions).

**Table 15. Average TTimes for Sensitivity Experiments.**

| log copies | 6a1 | 6b1 | 6c1 | 6e1 |
|---|---|---|---|---|
| 0 | nd | nd | nd | nd |
| 1.69 | 15.17 | 14.29 | nd | 14.57 |
| 2.69 | 11.92 | 11.60 | nd | 11.79 |
| 3.69 | 11.22 | 9.97 | 18.63 | 11.29 |
| 4.69 | 9.52 | 9.39 | 14.92 | 9.00 |
| 5.69 | 7.72 | 7.49 | 12.80 | 7.54 |
| 6.69 | 6.36 | 6.66 | 11.55 | 6.74 |

| | | | | |
|---|---|---|---|---|
| nd represents assay conditions that provided no readable TTime | | | | |

These data show that each of combinations 6a1, 6b1, 6c1, and 6e1 have good sensitivity, with some showing detection as low as at least 1.7 log copies per reaction.

### Example 7: Isothermal Amplification and Real-Time Detection of SARS-CoV-2 Using Different Combinations of Primers and Probes.

Sixty additional combinations of T7 promoter primer, non-T7 primer and dual labeled detection probe oligonucleotide were tested in a real-time TMA amplification and detection reaction as described in Example 5. Table 16 discloses the sequence and concentration of the various T7 promoter primers tested. Table 17 discloses the sequence and concentration of the various non-T7 primers tested. And Table 18 discloses the sequence and concentration of the various dual-labeled hairpin detection probes (torches). These reaction mixtures were each tested singly, and the performance of each combination was recorded in Table 19 as TTime. Combination conditions are presented using the Rxn Component IDs in the following order: T7 promoter primer, non-T7 primer, Torch.

**Table 16. T7 Promoter Primers.**

| Rxn Component ID | SEQ ID NO: | Stock Oligo Concentration | Final Concentration |
|---|---|---|---|
| 1 | 70 | 60.68 pm/µL | 0.2 pm/µL |
| 2 | 78 | 58.93 pm/µL | 0.2 pm/µL |
| 3 | 64 | 61.12 pm/µL | 0.2 pm/µL |
| 4 | 63 | 48.79 pm/µL | 0.2 pm/µL |
| 5 | 66 | 47.00 pm/µL | 0.2 pm/µL |
| 6 | 65 | 70.00 pm/µL | 0.2 pm/µL |

**Table 17. Non-T7 Primers.**

| Rxn Component ID | SEQ ID NO: | Stock Oligo Concentration | Final Concentration |
|---|---|---|---|
| a | 22 | 225.75 pm/µL | 0.4 pm/µL |
| b | 15 | 88.25 pm/µL | 0.4 pm/µL |
| c | 23 | 205.75 pm/µL | 0.4 pm/µL |
| d | 24 | 197.00 pm/µL | 0.4 pm/µL |
| e | 25 | 149.00 pm/µL | 0.4 pm/µL |

**Table 18. Dual-Labeled Hairpin Detection Probes.**

| Rxn Component ID | SEQ ID NO:* | Stock Oligo Concentration | Final Concentration |
|---|---|---|---|
| 1 | 106 | 84.31 pm/µL | 0.6 pm/µL |
| 2 | 105 | 87.16 pm/µL | 0.6 pm/µL |

| | | | |
|---|---|---|---|
| * The probes were labeled with the fluorophore FAM and the quencher Dabcyl. | | | |

Each of the T7 promoter primers, non-T7 primers and Torch components from the above Tables 16-18 were combined and tested. Combination conditions are presented in Table 19 using the Rxn Component IDs in the following order: T7 promoter primer, non-T7 primer, Torch.

**Table 19. TTimes for each Combination Condition. Combination condition is labeled according to: T7 promoter primer of Table 16 (first digit), Non-T7 Primer of Table 17 (letter), and Probe of Table 18 (final digit).**

| Combination Condition | TTime | Combination Condition | TTime |
|---|---|---|---|
| 1a1 | **8.92** | 1a2 | **9.27** |
| 2a1 | **7.07** | 2a2 | **7.66** |
| 3a1 | nd | 3a2 | nd |
| 4a1 | 12.29 | 4a2 | 11.99 |
| 5a1 | nd | 5a2 | nd |
| 6a1 | 11.49 | 6a2 | 11.50 |
| 1b1 | **8.84** | 1b2 | **8.67** |
| 2b1 | **6.89** | 2b2 | **6.98** |
| 3b1 | nd | 3b2 | nd |
| 4b1 | nd | 4b2 | nd |
| 5b1 | nd | 5b2 | nd |
| 6b1 | 12.88 | 6b2 | 10.74 |
| 1c1 | **8.38** | 1c2 | **8.11** |
| 2c1 | **6.22** | 2c2 | **6.53** |
| 3c1 | nd | 3c2 | * |
| 4c1 | 11.46 | 4c2 | 11.30 |
| 5c1 | nd | 5c2 | nd |
| 6c1 | nd | 6c2 | nd |
| 1d1 | **7.74** | 1d2 | **8.23** |
| 2d1 | **6.21** | 2d2 | **6.90** |
| 3d1 | nd | 3d2 | nd |
| 4d1 | 11.96 | 4d2 | 12.63 |
| 5d1 | nd | 5d2 | nd |
| 6d1 | nd | 6d2 | nd |
| 1e1 | **7.73** | 1e2 | **8.19** |
| 2e1 | **6.35** | 2e2 | **6.97** |
| 3e1 | nd | 3e2 | nd |
| 4e1 | 11.64 | 4e2 | 12.44 |
| 5e1 | nd | 5e2 | nd |
| 6e1 | 12.49 | 6e2 | 12.07 |

| | | | |
|---|---|---|---|
| nd represents assay conditions that provided no readable TTime * The TTime for Combination Condition 3c2 was very low and had a linear rather than sigmoidal slope, likely indicating a false positive result. | | | |

In this experiment, combinations including SEQ ID NO:70, SEQ ID NO:78 or SEQ ID NO:24 showed a consistently fast TTime. Except for Combination Condition 3c2, all the combinations presenting a TTime provided good performance.

### Example 8: Analytical Sensitivity of Several Primer and Probe Combinations

Oligonucleotide combinations each containing one of several T7 promoter primers (Table 20), a single non-T7 primer (Table 21) and a single detection probe (Table 22) were tested against a dilution of target nucleic acids to measure the sensitivity of these combinations. Reaction mixtures were set up as generally described for Example 5, and the reactions run in triplicate.

**Table 20. T7 Promoter Primers.**

| Rxn Component ID | SEQ ID NO | Stock Oligo Concentration | Final Concentration |
|---|---|---|---|
| 1 | 70 | 60.68 pm/µL | 0.2 pm/µL |
| 2 | 78 | 58.93 pm/µL | 0.2 pm/µL |
| 4 | 63 | 48.79 pm/µL | 0.2 pm/µL |
| 6 | 65 | 70.00 pm/µL | 0.2 pm/µL |

**Table 21. Non-T7 Primer.**

| Rxn Component ID | SEQ ID NO | Stock Oligo Concentration | Final Concentration |
|---|---|---|---|
| d | 24 | 197.00 pm/µL | 0.4 pm/µL |

**Table 22. Torch.**

| Rxn Component ID | SEQ ID NO* | Stock Oligo Concentration | Final Concentration |
|---|---|---|---|
| 1 | 106 | 84.31 pm/µL | 0.6 pm/µL |

| | | | |
|---|---|---|---|
| *The probe was labeled with FAM as a fluorophore and Dabcyl as a quencher | | | |

Combination conditions and test results are presented Table 23 using the Rxn Component IDs format presented in a described for Table 19.

**Table 23. TTimes for Sensitivity Experiments.**

| log Copies | 1d1 | 2d1 | 3d1 or 4d1 | 6d1 |
|---|---|---|---|---|
| 0.0 | nd | nd | nd | nd |
| 0.0 | nd | nd | nd | nd |
| 0.0 | nd | nd | nd | nd |
| 1.7 | nd | nd | nd | nd |
| 1.7 | nd | nd | nd | nd |
| 1.7 | nd | nd | nd | nd |
| 2.7 | 18.69 | 13.00 | nd | nd |
| 2.7 | 18.56 | 13.26 | nd | nd |
| 2.7 | 19.30 | nd | nd | nd |
| 3.7 | 15.14 | 10.89 | 19.81 | nd |
| 3.7 | 15.08 | 10.03 | 20.33 | nd |
| 3.7 | 15.49 | 10.31 | 20.07 | nd |
| 4.7 | 12.10 | 8.72 | 15.87 | nd |
| 4.7 | 12.40 | 8.73 | 15.29 | nd |
| 4.7 | 13.33 | 8.73 | 15.19 | nd |
| 5.7 | 9.51 | 7.31 | 12.80 | nd |
| 5.7 | 8.94 | 7.13 | 12.61 | nd |
| 5.7 | 9.53 | 7.14 | 13.62 | nd |
| 6.7 | 8.06 | 6.29 | 11.03 | nd |
| 6.7 | 8.03 | 6.06 | 10.38 | nd |
| 6.7 | 8.42 | 6.13 | 10.56 | nd |

| | | | | |
|---|---|---|---|---|
| nd represents assay conditions that provided no readable TTime | | | | |

In this experiment, combinations 1d1, 2d1, and 4d1 showed good sensitivity to 3.7 log copies of target nucleic acid per reaction, with combinations 1d1 and 2d1 showing good sensitivity down to 2.7 log copies per reaction. Combination 6d1 was not reactive in this experiment.

In a further sensitivity experiment, a number of combinations containing 0.2 pm/µL of SEQ ID NO:78 as a promoter primer, 0.15 pm/µL of SEQ ID NO:106 as a torch and 0.4 pm/µL of one of SEQ ID Nos:15, 23, 24, or 25 as non-T7 primers were further tested for sensitivity. The reactions were set up as generally described directly above, and each combination was run in triplicate. The Combination Condition indicators from Table 19 are used in the data tables (Table 24: showing average TTimes for the triplicate reactions).

**Table 24. Average TTimes for Sensitivity Experiments.**

| log copies | 2b1 | 2c1 | 2d1 | 2e1 |
|---|---|---|---|---|
| 0 | nd | nd | nd | nd |
| 1.69 | nd | nd | nd | nd |
| 2.69 | nd | 12.81 | 12.96 | 14.50 |
| 3.69 | 11.68 | 10.36 | 10.55 | 10.82 |
| 4.69 | 9.25 | 8.72 | 8.92 | 8.92 |
| 5.69 | 7.49 | 7.16 | 7.28 | 7.44 |
| 6.69 | 6.21 | 6.05 | 6.01 | 6.18 |

| | | | | |
|---|---|---|---|---|
| nd represents assay conditions that provided no readable TTime | | | | |

These data show these combinations to have good sensitivity, with some showing detection as low as at least 2.7 log copies per reaction.

### Example 9: Multiplex Formulation with Primer and Probe Combinations Targeting Two Regions of SARS-CoV-2.

Several oligomer combinations were prepared in a reaction mixture for performing multiplex, real-time amplification and detection reactions targeting two regions of a coronavirus target nucleic acid. These reaction mixtures included T7 promoter primers having the sequences shown in SEQ ID NO:57 and SEQ ID NO:70, the non-T7 primers having the sequences shown in SEQ ID NO:21 and SEQ ID NO:24, and the detection probes having the sequences shown in SEQ ID NO:104 (labeled with FAM as a fluorophore and Dabcyl as a quencher) and SEQ ID NO:106 (labeled with HEX as a fluorophore and Dabcyl as a quencher). In each of the various combinations, the T7 promoter primer of SEQ ID NO:70 was included in a reaction mixture at either 0.1 pm/µL, 0.2 pm/µL, or 0.3pm/µL, while the other oligomer components were provided at the same concentration (0.2 pm/µL for SEQ ID NO:57, 0.4 pm/µL. for SEQ ID NO:21, 0.4 pm/µL for SEQ ID NO:24, 0.15 pm/µL for SEQ ID NO:104, and 0.15 pm/µL for SEQ ID NO:106). The reactions were otherwise set up and run as generally described in Example 5. Results are shown in Table 25 with the different combinations referenced by the concentration of SEQ ID NO:70.

**Table 25. TTime Data for Multiplex Reactions.**

| log copies | 0.1 pm/µL SEQ ID NO:70 | | 0.2 pm/µL SEQ ID NO:70 | | 0.3 pm/µL SEQ ID NO:70 | |
|---|---|---|---|---|---|---|
| | Region 1 FAM Channel | Region 2 HEX Channel | Region 1 FAM Channel | Region 2 HEX Channel | Region 1 FAM Channel | Region 2 HEX Channel |
| 0.00 | nd | nd | nd | nd | nd | nd |
| 0.00 | nd | nd | nd | nd | nd | nd |
| 0.00 | nd | nd | nd | nd | nd | nd |
| 1.69 | 16.25 | nd | 16.01 | nd | nd | nd |
| 1.69 | 16.84 | nd | 15.23 | nd | nd | nd |
| 1.69 | 16.81 | nd | 14.83 | nd | nd | nd |
| 2.69 | 12.10 | nd | 13.31 | nd | 14.63 | nd |
| 2.69 | 12.45 | nd | 12.65 | nd | 14.18 | nd |
| 2.69 | 12.25 | nd | 13.41 | nd | 13.88 | nd |
| 3.69 | 10.90 | nd | 10.57 | 18.25 | 11.29 | 17.94 |
| 3.69 | 10.49 | nd | 10.65 | 18.31 | 10.69 | 16.46 |
| 3.69 | 10.46 | nd | 10.98 | 18.21 | 10.63 | 17.17 |
| 4.69 | 9.10 | 16.24 | 9.67 | 14.33 | 9.13 | 12.13 |
| 4.69 | 9.43 | 15.11 | 9.14 | 13.42 | 8.93 | 12.21 |
| 4.69 | 9.77 | 17.67 | 9.45 | 13.95 | 9.71 | 13.19 |
| 5.69 | 7.76 | 9.24 | 7.78 | 10.09 | 8.25 | 9.80 |
| 5.69 | 7.59 | 12.44 | 8.19 | 10.46 | 8.20 | 9.75 |
| 5.69 | 7.74 | 12.67 | 8.24 | 10.55 | 8.18 | 9.72 |
| 6.69 | 6.93 | 8.05 | 6.74 | 8.00 | 7.20 | 7.84 |
| 6.69 | 6.71 | 9.72 | 6.78 | 7.97 | 6.73 | 7.24 |
| 6.69 | 6.78 | 9.82 | 6.82 | 8.11 | 7.34 | 7.95 |

| | | | | | | |
|---|---|---|---|---|---|---|
| nd represents assay conditions that provided no readable TTime | | | | | | |

In this example, the multiplex combinations having 0.2 pm/µL of SEQ ID NO:70 showed the most robust performance with detection down to 1.7 log copies in the FAM channel and down to 3.7 log copies in the HEX channel. All combinations performed well, providing consistent TTimes for the triplicate conditions.

### Example 10: Analytical Sensitivity Testing using Target Capture Oligonucleotide, Primer and Probe Combinations in Singleplex and in Multiplex Reactions.

Two oligomer combinations were prepared for performing singleplexed and multiplexed, target capture, amplification and end-point detection reactions targeting two regions of a coronavirus target nucleic acid. The two regions of the coronavirus target nucleic acid are represented by IVTs having sequences shown in SEQ ID NOs:2 & 3. A stock concentration of SEQ ID NO:2 (2.4×10¹⁵ copies/mL) and a stock concentration of SEQ ID NO:3 (1.5×10¹⁵ copies/mL) were each serially diluted to 300, 100, 30, 10, 3, and 1 copies/mL using sample transport media as the diluent. Negative control was sample transport media without IVT. The target capture, primer, and probe oligonucleotides are shown in Table 26. Detection probe oligos were labeled with acridinium ester (AE). The negative control was added to 5 wells of a reaction plate. Each dilution of each IVT was also added to the reaction plate in multiples of 5 for the singleplex reactions. Combined dilution of the two IVTs were added to the reaction place in multiples of 5 for the multiplex reactions. Each reaction was performed at 100 µL total volume. A target capture reaction was then performed, and the captured samples were eluted into separate wells for isothermal amplification and detection. The assay was performed using a Panther instrument from Hologic, Inc. Results are shown in Table 27.

**Table 26: Coronavirus Target Capture Oligonucleotides, T7 Promoter Primers, Non-T7 Primers, and Detection Probes.**

| Oligo Type | SEQ ID NO: | Stock Oligo Conc | Final Conc |
|---|---|---|---|
| TCO | 92 | 588.00 mg/L | 0.5 mg/L |
| T7 | 57 | 47.63 pm/µL | 0.2 pm/µL |
| Non-T7 | 21 | 227.75 pm/µL | 0.4 pm/µL |
| Probe | 36 | 20419941 RLU/µL | 6700 RLU/µL |
| TCO | 96 | 908.00 mg/L | 0.5 mg/L |
| T7 | 78 | 58.93 pm/µL | 0.2 pm/µL |
| Non-T7 | 24 | 197.00 pm/µL | 0.4 pm/µL |
| Probe | 45 | 3548884 RLU/µL | 5700 RLU/µL |

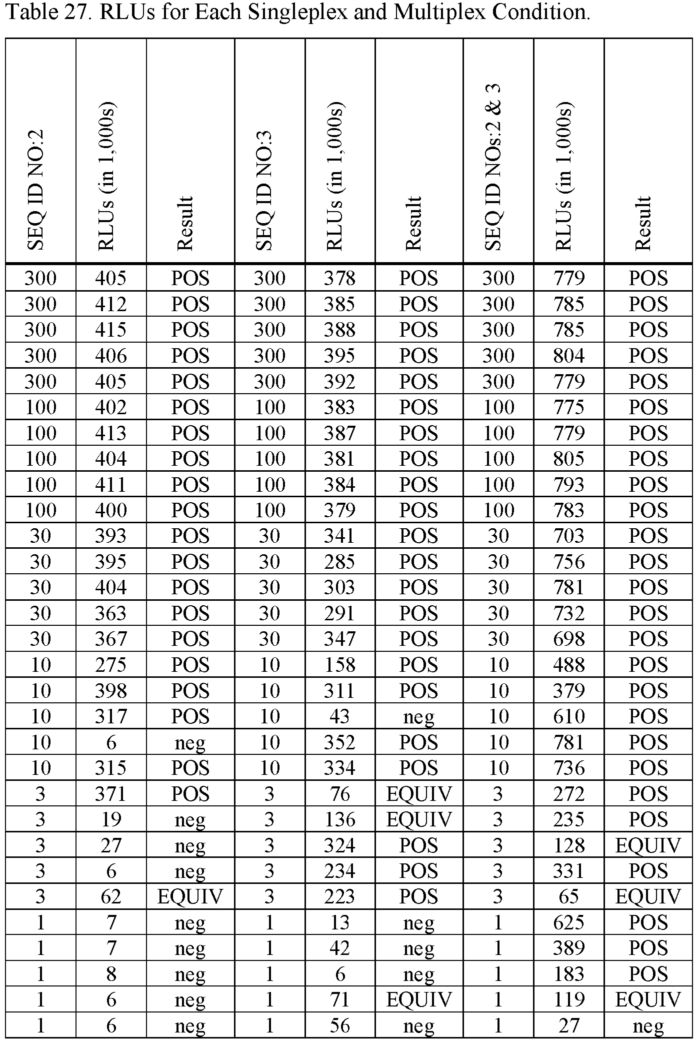

Results in this experiment showed comparable sensitivity for both regions of the coronavirus target nucleic acids with 100% positivity at 30 copies/mL for singleplex and multiplex conditions; 80% positivity for the singleplex conditions and 100% positivity for the multiplex condition at 10 copies/mL, and 60% positivity for one of the singleplex conditions at 3 copies/mL and for the multiplex condition at 1 copy/mL. The negative reaction wells showed no false positives. Reaction curves showed good kinetics.

### Example 11: Microbial Cross-Reactivity and Interference Testing

The purpose of this experiment was to demonstrate that the Table 28 primer and probe combinations do not cross-react with genetically closely related or commonly encountered microorganisms. Additionally, this experiment demonstrated that evaluated microorganisms do not interfere with the Table 28 primer and probe combinations' capabilities to detect SARS-CoV-2. A variety of microorganisms listed in Table 29 were tested in the presence and absence of SARS-CoV-2 inactivated cultured virus (BEI Resources, Manassas, VA; cat no NR-52281) spiked at 0.03 TCID50/mL (3× LoD) in pooled clinical nasopharyngeal (NP) swabs processed in a 1:1.56 ratio of STM. Stock concentrations of the microorganisms listed in Table 29 were diluted in sample transport media to achieve a target concentration (see Table 30). The assay was set-up and performed as generally described in Example 10.

**Table 28. Primers and Probes.**

| Oligo Type | SEQ ID NO: |
|---|---|
| T7 Primer | 57 |
| Non-T7 Primer | 21 |
| Detection Probe | 36 |
| T7 Primer | 78 |
| Non-T7 Primer | 24 |
| Detection Probe | 45 |

**Table 29. Microorganisms (Unless otherwise indicated, microorganisms were from samples collected at Hologic, Inc. or were represented by in vitro transcripts synthesized by Hologic, Inc.)**

| Microorganisms from the same genetic family | | Circulating Microorganisms | |
|---|---|---|---|
| Human coronavirus 229E | ATCC, Manassas, VA; cat no VR-740 | Adenovirus Type 1 | ZeptoMetrix, Buffalo, NY; cat no 0810050CF |
| Human coronavirus OC43 | ZeptoMetrix, Buffalo, NY; cat no 0810024CF (strain HCT-8) | Human Metapneumovirus (hMPV) | ZeptoMetrix, Buffalo, NY; cat no 0810160CF (strain IA3-2002) |
| Human coronavirus HKU1 | | Parainfluenza virus Types 1-4 | ZeptoMetrix, Buffalo, NY; cat nos 0810014CF, 0810015CF, 0810016CF, & 0810060CF |
| Human coronavirus NL63 | ZeptoMetrix, Buffalo, NY; cat no 0810228CF (Vero strain) | Influenza A | ZeptoMetrix, Buffalo, NY; cat no 0810514CF (Norway/466/2014) |
| SARS-coronavirus | | Influenza B | ZeptoMetrix, Buffalo, NY; cat no 08100253CF (Florida/04/06) |
| MERS-coronavirus | BEI Resources, Manassas, VA; EMC/1012 isolate | Enterovirus (e.g., EV68) | ZeptoMetrix, Buffalo, NY; cat no 0810236CF (200E Isolate) |
| | | Respiratory syncytial virus | ZeptoMetrix, Buffalo, NY; cat no 0810040ACF (2006 isolate) |
| | | Rhinovirus Type B14 | ZeptoMetrix, Buffalo, NY; cat no 0810023CFN |
| | | *Chlamydia pneumonia* | ATCC, Manassas, VA; cat no 70019109 (strain 2023) |
| | | *Haemophilus influenzae* | |
| | | *Legionella pneumophila* | |
| | | *Mycobacterium tuberculosis* | ZeptoMetrix, Buffalo, NY; cat no. 801660 (strain H37Ra-1) |
| | | *Streptococcus pneumonia* | |
| | | *Streptococcus pyogenes* | |
| | | *Bordetella pertussis* | |
| | | *Mycoplasma pneumoniae* | |
| | | *Pneumocystis jirovecii (PJP)* | |
| | | *Candida albicans* | |
| | | *Pseudomonas aeruginosa* | |
| | | *Staphylococcus epidermidis* | |
| | | *Streptococcus salivarius* | |
| | | 30 clinical NP swab specimens collected from individuals and testing negative for SARS-CoV-2 to represent the diverse microbial flora in the human respiratory tract | |

**Table 30. Reaction Concentrations for Microorganisms Tested.**

| Microbe | Units | Target concentration |
|---|---|---|
| Human coronavirus 229E | TCID50/mL | 1.00E+05 |
| Human coronavirus OC43 | TCID50/mL | 1.00E+05 |
| Human coronavirus HKU1 | c/mL | 1.00E+06 |
| Human coronavirus NL63 | TCID50/mL | 5.00E+02 |
| SARS-coronavirus | c/mL | 1.00E+06 |
| MERS-coronavirus | TCID50/mL | 1.00E+04 |
| Adenovirus Type 1 | TCID50/mL | 1.00E+05 |
| Human Metapneumovirus (hMPV) | TCID50/mL | 1.00E+06 |
| Parainfluenza virus Type 1 | TCID50/mL | 1.00E+05 |
| Parainfluenza virus Type 2 | TCID50/mL | 1.00E+05 |
| Parainfluenza virus Type 3 | TCID50/mL | 1.00E+05 |
| Parainfluenza virus Type 4 | TCID50/mL | 1.00E+03 |
| Influenza A (H3N2) | TCID50/mL | 1.00E+05 |
| Influenza B | TCID50/mL | 2.00E+03 |
| Enterovirus (e.g., EV68) | TCID50/mL | 1.00E+05 |
| Respiratory syncytial virus | TCID50/mL | 1.00E+05 |
| Rhinovirus | TCID50/mL | 1.00E+04 |
| *Chlamydia pneumonia* | IFU/mL | 1.00E+06 |
| *Haemophilus influenzae* | CFU/mL | 1.00E+06 |
| *Legionella pneumophila* | CFU/mL | 1.00E+06 |
| *Mycobacterium tuberculosis* | TCID50/mL | 1.00E+06 |
| *Streptococus pneumonia* | CFU/mL | 1.00E+06 |
| *Streptococcus pyrogenes* | CFU/mL | 1.00E+06 |
| *Bordetella pertussis* | CFU/mL | 1.00E+06 |
| *Mycoplasma pneumoniae* | CFU/mL | 1.00E+06 |
| *Pneumocystis jirovecii (PJP)* | | 1.00E+06 |

The assay was set-up and performed as generally described in Example 10. Briefly for a first region of the SARS-CoV-2 target nucleic acid, a target capture reagent was prepared to contain a target capture oligonucleotide (SEQ ID NO:92 at 0.666 mg/L), an amplification reagent was prepared to contain a T7 primer (SEQ ID NO:57 at 0.1 pmol/µL) and a non-T7 primer (SEQ ID NO:21 at 0.2 pmol/µL); and a detection hybridization reagent containing an AE labeled detection probe (SEQ ID NO:36 at 5E3 RLU/µL). Similarly, for a second region of the SARS-CoV-2 target nucleic acid, a target capture oligonucleotide (SEQ ID NO:96 at 0.666 mg/L), an amplification reagent was prepared to contain a T7 primer (SEQ ID NO:78 at 0.1 pmol/µL) and a non-T7 primer (SEQ ID NO:24 at 0.2 pmol/µL); and a detection hybridization reagent containing an AE labeled detection probe (SEQ ID NO:45 at 5E3 RLU/µL). A nucleic acid internal control system was also prepared to contain a target capture oligonucleotide (SEQ ID NO:10 at 0.5 mg/L in Target Capture Reagent), a T7 primer (SEQ ID NO:6 at 0.2 pmol/µL in an amplification reagent), a non-T7 primer (SEQ ID NO:7 at 0.4 pmol/µL in an amplification reagent) and an AE labeled detection probe oligonucleotide (SEQ ID NO:8 at 4E3 GLU/µL). Target signal was distinguished from internal control signal using flasher/glower detection probes, wherein signal from any target in reaction lights off quickly and with a higher intensity than is measured for the slower, longer lasting, and less intense glower signal of the internal control probe. All samples were run in triplicate on a Panther instrument (Hologic, Inc.). Results are presented in Table 31.

**Table 31. Cross-Reactivity and Interference Testing Results.**

| Microorganism | SARS-CoV-2 Unspiked | | | | SARS-CoV-2 Spiked | | | |
|---|---|---|---|---|---|---|---|---|
| | N Tested | N Valid | N Detected | % Detected | N Tested | N Valid | N Detected | % Detected |
| No organism Control | 3 | 3 | 0 | 0 | 3 | 3 | 3 | 100 |
| Human coronavirus 229E | 3 | 3 | 0 | 0 | 3 | 3 | 3 | 100 |
| Human coronavirus OC43 | 3 | 3 | 0 | 0 | 3 | 3 | 3 | 100 |
| Human coronavirus HKU1 | 3 | 3 | 0 | 0 | 3 | 3 | 3 | 100 |
| Human coronavirus NL63 | 3 | 3 | 0 | 0 | 3 | 3 | 3 | 100 |
| SARS-coronavirus | 3 | 3 | 0 | 0 | 3 | 3 | 3 | 100 |
| MERS-coronavirus | 3 | 3 | 0 | 0 | 3 | 3 | 3 | 100 |
| Adenovirus Type 1 | 3 | 3 | 0 | 0 | 3 | 3 | 3 | 100 |
| Human Metapneumovirus | 3 | 3 | 0 | 0 | 3 | 3 | 3 | 100 |
| Parainfluenza virus 1 | 3 | 3 | 0 | 0 | 3 | 3 | 3 | 100 |
| Parainfluenza virus 2 | 3 | 3 | 0 | 0 | 3 | 3 | 3 | 100 |
| Parainfluenza virus 3 | 3 | 3 | 0 | 0 | 3 | 3 | 3 | 100 |
| Parainfluenza virus 4 | 3 | 3 | 0 | 0 | 3 | 3 | 3 | 100 |
| Influenza A (H3N2) | 3 | 3 | 0 | 0 | 3 | 3 | 3 | 100 |
| Influenza B | 3 | 3 | 0 | 0 | 3 | 3 | 3 | 100 |
| Enterovirus (e.g., EV68) | 3 | 3 | 0 | 0 | 3 | 3 | 3 | 100 |
| Respiratory syncytial virus | 3 | 3 | 0 | 0 | 3 | 3 | 3 | 100 |
| Rhinovirus | 3 | 3 | 0 | 0 | 3 | 3 | 3 | 100 |
| *Chlamydia pneumonia* | 3 | 3 | 0 | 0 | 3 | 3 | 3 | 100 |
| *Haemophilus influenzae* | 3 | 3 | 0 | 0 | 3 | 3 | 3 | 100 |
| *Legionella pneumophila* | 3 | 3 | 0 | 0 | 3 | 3 | 3 | 100 |
| *Mycobacterium tuberculosis* | 3 | 3 | 0 | 0 | 3 | 3 | 3 | 100 |
| *Streptococcus pneumonia* | 3 | 3 | 0 | 0 | 3 | 3 | 3 | 100 |
| *Streptococcus pyogenes* | 3 | 3 | 0 | 0 | 3 | 3 | 3 | 100 |
| *Bordetella pertussis* | 3 | 3 | 0 | 0 | 3 | 3 | 3 | 100 |
| *Mycoplasma pneumoniae* | 3 | 3 | 0 | 0 | 3 | 3 | 3 | 100 |
| *Pneumocystis jirovecii (PJP)* | 3 | 3 | 0 | 0 | 3 | 3 | 3 | 100 |
| *Candida albicans* | 3 | 3 | 0 | 0 | 3 | 3 | 3 | 100 |
| *Pseudomonas aeruginosa* | 3 | 3 | 0 | 0 | 3 | 3 | 3 | 100 |
| *Staphylococcus epidermidis* | 3 | 3 | 0 | 0 | 3 | 3 | 3 | 100 |
| *Streptococcus salivarius* | 3 | 3 | 0 | 0 | 3 | 3 | 3 | 100 |
| Negative clinical NP swab specimens | 90 | 90 | 0 | 0 | 90 | 90 | 90 | 100 |

This example showed negative results for all challenge microorganism reactions that were void of a SARS-CoV-2 target nucleic acid (all internal controls showed positive in these reactions). This example also showed 100% positivity for detecting SARS-CoV-2 when assayed in the presence of these challenge organisms. Thus, the primers and probes do not cross-react with genetically closely related microorganisms and commonly encountered nasopharyngeal microorganisms. Also, the genetically closely related microorganisms and commonly encountered nasopharyngeal microorganisms do not interfere with detection of SARS-CoV-2 target nucleic acid using the primers and probes.

### Example 12: Clinical Performance of a PCR Assay and an Isothermal Assay

The clinical performances of a PCR assay and an isothermal assay were determined using a panel of remnant clinical specimens. For the experiment, 105 remnant clinical nasopharyngeal specimens were collected from US patients with signs and symptoms of respiratory infection. The PCR assay was set up and generally performed as described in Example 2. The isothermal assay was set up and performed as generally described in Example 11. The PCR primers and probes were SEQ ID NOs:12-17 (the PCR internal control being SEQ ID NOs:4, 5, and 9). The isothermal primers and probes were SEQ ID NOs:21, 24, 36, 45, 57, and 78 (the isothermal internal controls being SEQ ID NOs:6, 7, 8, & 10). The assays were performed on a Panther Fusion instrument, 1 replicate per remnant clinical sample per assay. Results are shown in Table 32.

**Table 32. Clinical Performance.**

| | | SARS-CoV-2 PCR Result | | |
|---|---|---|---|---|
| | | Positive | Negative | Total |
| SARS-CoV-2 TMA Result | Positive | 50 | 1 | 51 |
| | Negative | 0 | 54 | 54 |
| | Total | 50 | 55 | 105 |

These assays showed an overall agreement of 99.0%, a positive agreement of 100 %, and a negative agreement of 98.2% on these 105 clinical remnant samples.

### Example 13: Clinical Performance of a PCR Assay and an Isothermal Assay on Pooled Clinical Samples

Each of a PCR assay and an isothermal assay were tested for their abilities to detect as few as 1 positive clinical sample in a pool of five samples. 325 individual nasopharyngeal (NP) clinical samples were collected using a nasopharyngeal swab, a nasal swab or an oropharyngeal swab and placed in 3 ml of a viral transport medium or universal transport medium according to manufacturer's instructions. These clinical samples were individually assayed to determine the presence or absence of SARS CoV-2 in each sample. Using the individual testing results, samples were pooled to make 45 positive pooled samples and 20 negative pooled samples. Positive pooled samples were one positive individual sample combined with 4 negative individual samples. Negative pooled samples were a combination of 5 negative individual samples. 100 µL of each of 5 individual samples were combined into 780 µL of sample transportation media for a pooled sample volume of 1.280 mL. One replicate from each positive and negative pool was tested in a PCR assay and one replicate from each positive and negative pool was tested in an isothermal assay. The assays were performed as generally described above and using the oligonucleotides disclosed in Tables 1 and 26, and internal control oligonucleotides at SEQ ID NOs:4-10. Assays were performed using a Panther Fusion instrument. Results were presented as Ct for the PCR reactions and kRLU for the isothermal amplification assays and are summarized in Tables 33-34.

**Table 33. PCR Assay Ct Summary Statistics.**

| | | | | PCR Results | | | | | |
|---|---|---|---|---|---|---|---|---|---|
| Type | Type | N Tested | N Analyzed | Average CoV Ct | StDev CoV Ct | CV CoV Ct | Average IC Ct | StDev IC Ct | CV IC Ct |
| Negative | Pools | 20 | 20 | - | - | - | 30.49 | 0.44 | 1.4% |
| | Individual | 280 | 279* | - | - | - | 30.38 | 0.39 | 1.3% |
| Positive | Pools | 45 | 43** | 28.47 | 4.48 | 15.7% | 30.28 | 0.27 | 0.9% |
| | Individual | 45 | 44*** | 26.61 | 4.66 | 17.5% | 30.47 | 0.56 | 1.8% |

| | | | | | | | | | |
|---|---|---|---|---|---|---|---|---|---|
| *1 false positive removed from calculations (same sample gave a false positive isothermal assay result) **2 expected positive pools gave negative results in the PCR assay. The IC results from these pools were excluded from calculations **1 invalid excluded from calculations | | | | | | | | | |

**Table 34. Isothermal Assay kRLU Summary Statistics.**

| | | | | Aptima Results | | |
|---|---|---|---|---|---|---|
| Type | Type | N Tested | N Analyzed | Average kRLU | StDev kRLU | CV kRLU |
| Negative | Pools | 20 | 20 | 273 | 7 | 2.5% |
| | Individual | 280 | 270* | 270 | 11 | 4.0% |
| Positive | Pools | 45 | 45 | 1,137 | 99 | 8.7% |
| | Individual | 45 | 45 | 1,147 | 68 | 5.9% |

| | | | | | | |
|---|---|---|---|---|---|---|
| *1 false positive excluded from calculations (same sample gave a false positive PCR result) *9 invalids excluded from calculations | | | | | | |

Pooled results were analyzed against expected results to calculate percent agreement. Results were analyzed to show assay performance in populations with different low positive prevalence. Low positive prevalence was determined by collecting 31,000 individual test results and determining Ct distribution for these results. The Ct results were divided into quartiles and, based on the Ct distribution, low positive was determined as samples having a Ct value greater than 34 (11.1% of the total positive samples). Table 35 and Table 36 show the positive percent agreement (PPA) and negative percent agreement (NPA) of an isothermal amplification assay and a PCR assay, respectively, in a population with 11.1% low positive prevalence (5/45). The isothermal amplification assay had a PPA of 100% (92.1-100) and the PCR assay had a PPA of 95.6% (85.2-98.8). Both assays had an NPA of 100% (83.9-100).

**Table 35. Isothermal PPA/NPA for 11.1% Low Positive Prevalence.**

| | | Expected Result | | Total | | Agreement | 95% C.I. |
|---|---|---|---|---|---|---|---|
| | | Pos | Neg | | | | |
| Pooled Result | Pos | 45 | 0 | 45 | Positive Percent Agreement | 100.0% | (92.1% - 100.0%) |
| | Neg | 0 | 20 | 20 | Negative Percent Agreement | 100.0% | (83.9% - 100.0%) |
| Total | | 45 | 20 | 65 | Overall Agreement | 100.0% | (94.4% - 100.0%) |

**Table 36. PCR PPA/NPA for 11.1% Low Positive Prevalence.**

| | | Expected Result | | Total | | Agreement | 95% C.I. |
|---|---|---|---|---|---|---|---|
| | | Pos | Neg | | | | |
| Pooled Result | Pos | 43 | 0 | 43 | Positive Percent Agreement | 95.6% | (85.2% - 98.8%) |
| | Neg | 2 | 20 | 22 | Negative Percent Agreement | 100.0% | (83.9% - 100%) |
| Total | | 45 | 20 | 65 | Overall Agreement | 96.9% | (89.5% - 99.2%) |

Table 37 and Table 38 show the PPA and NPA of the isothermal amplification assay and the PCR assay, respectively, in a population with 25% low positive prevalence (5/20). The Isothermal assay had a PPA of 100% (83.9-100) and the PCR assay had a PPA of 90.0% (69.9-97.2). Both assays had an NPA of 100% (83.9-100).

**Table 37: Isothermal PPA/NPA for 25% Low Positive Prevalence.**

| | | Expected Result | | Total | | Agreement | 95% C.I. |
|---|---|---|---|---|---|---|---|
| | | Pos | Neg | | | | |
| Pooled Result | Pos | 20 | 0 | 20 | Positive Percent Agreement | 100.0% | (83.9% - 100%) |
| | Neg | 0 | 20 | 20 | Negative Percent Agreement | 100.0% | (83.9% - 100%) |
| Total | | 20 | 20 | 40 | Overall Agreement | 100.0% | (91.2% - 100%) |

**Table 38. PCR PPA/NPA for 25% Low Positive Prevalence.**

| | | Expected Result | | Total | | Agreement | 95% C.I. |
|---|---|---|---|---|---|---|---|
| | | Pos | Neg | | | | |
| Pooled Result | Pos | 18 | 0 | 18 | Positive Percent Agreement | 90.0% | (69.9% - 97.2%) |
| | Neg | 2 | 20 | 22 | Negative Percent Agreement | 100.0% | (83.9% - 100%) |
| Total | | 20 | 20 | 40 | Overall Agreement | 95.0% | (83.5% - 98.6%) |

Both the isothermal assay and the PCR assay showed a greater than 90% PPA to expected results pools from individual samples obtained in populations having as high as 25% low positivity prevalence. Additionally, both assays showed at least 99% NPA.

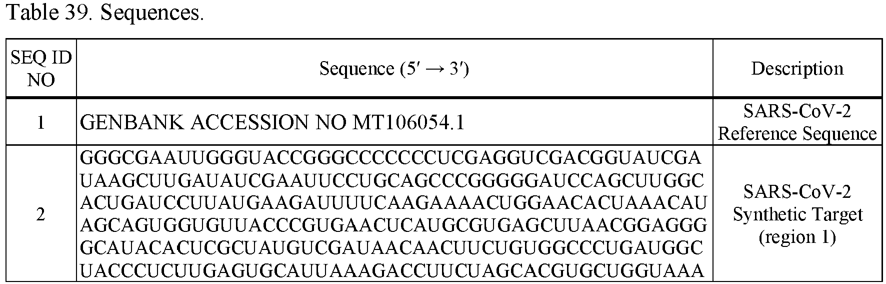

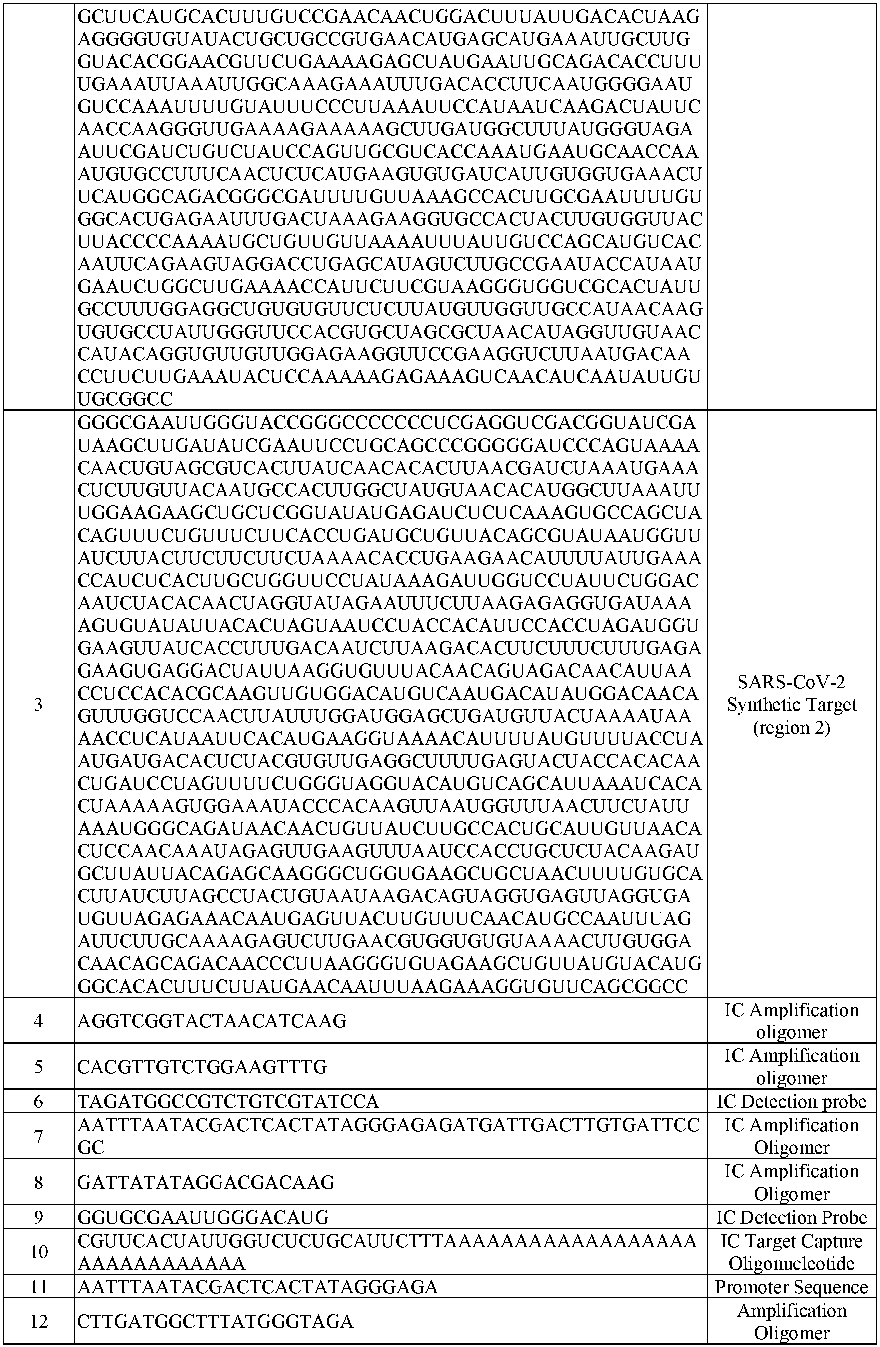

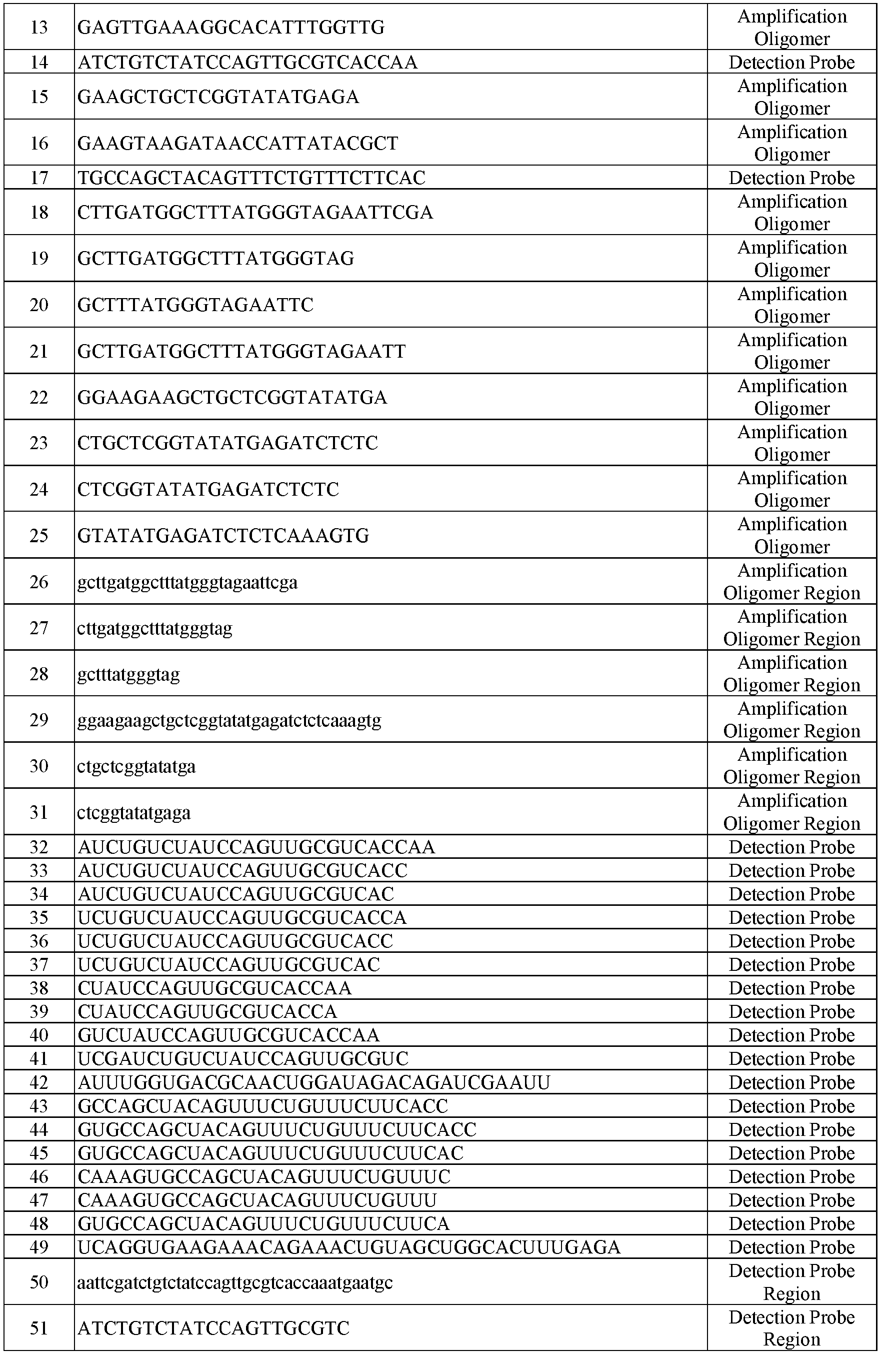

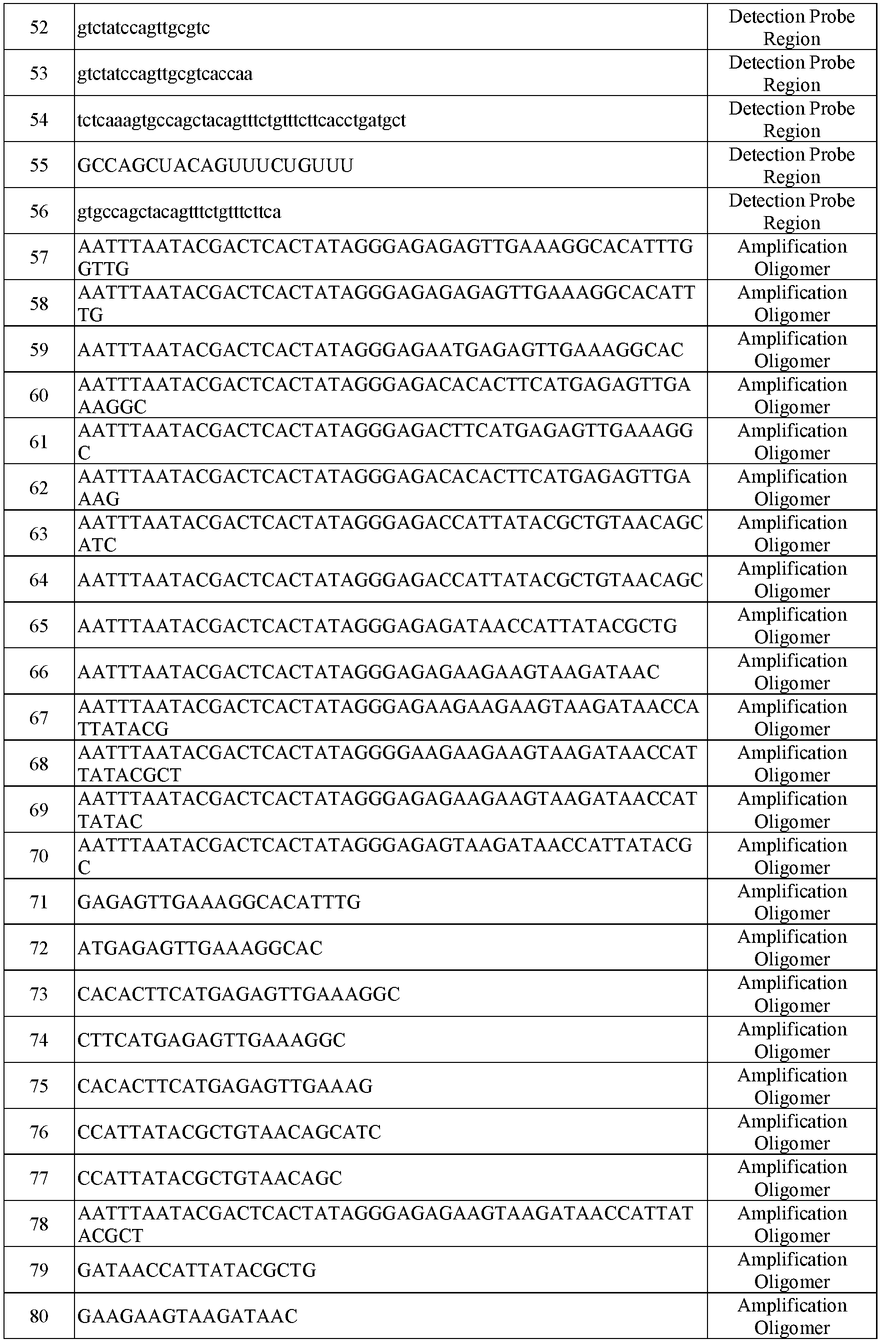

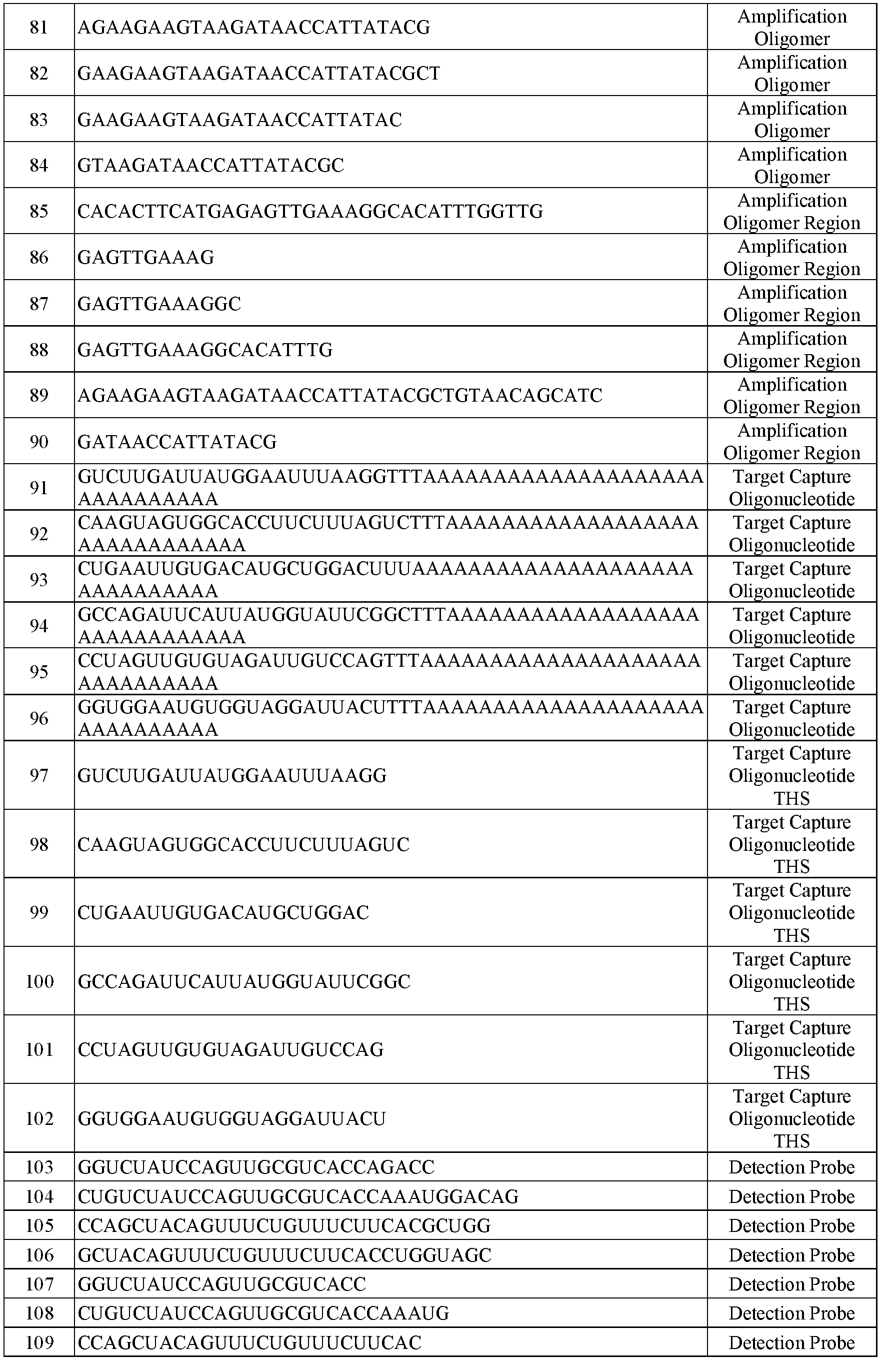

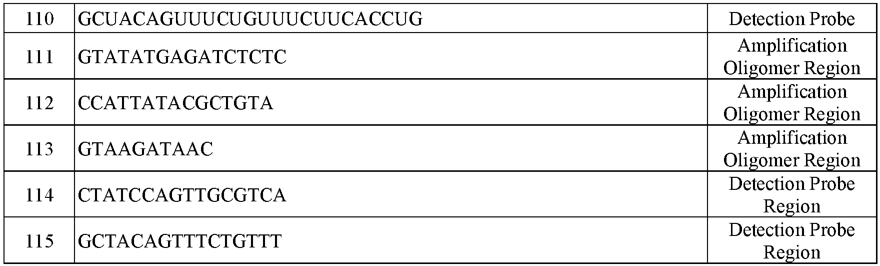

From the foregoing, it will be appreciated that, although specific embodiments have been described herein for purposes of illustration, various modifications may be made without deviating from the spirit and scope of the disclosure. All publications, patents, and patent applications cited herein are hereby incorporated by reference in their entireties for all purposes.
The further aspects of the present disclosure are set forth in the following numbered paragraphs:
1. A composition or kit for determining the presence or absence of SARS-CoV-2 in a sample, said composition or kit comprising:
   (a) a first amplification oligomer combination comprising first and second SARS-CoV-2-specific amplification oligomers capable of amplifying a target region of a SARS-CoV-2 target nucleic acid,
      (i) wherein the first SARS-CoV-2-specific amplification oligomer comprises a first SARS-CoV-2-specific target-hybridizing sequence 18 to 27 contiguous nucleotides in length, wherein the target-hybridizing sequence is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
      (ii) wherein the second SARS-CoV-2-specific amplification oligomer comprises a second SARS-CoV-2-specific target-hybridizing sequence 18 to 23 contiguous nucleotides in length, wherein the target-hybridizing sequence is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88; and
   (b) a second amplification oligomer combination comprising first and second SARS-CoV-2-specific amplification oligomers capable of amplifying a target region of a SARS-CoV-2 target nucleic acid,
      (i) wherein the first SARS-CoV-2-specific amplification oligomer comprises a first SARS-CoV-2-specific target-hybridizing sequence 20 to 23 contiguous nucleotides in length, wherein the target-hybridizing sequence is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
      (ii) wherein the second SARS-CoV-2-specific amplification oligomer comprises a second SARS-CoV-2-specific target-hybridizing sequence 16 to 27 contiguous nucleotides in length, wherein the target-hybridizing sequence is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113.
2. The composition or kit of paragraph 1, wherein the first SARS-CoV-2-specific target-hybridizing sequence of (a)(i) contains a nucleotide sequence differing by no more than 0, 1, 2, 3, 4, or 5 nucleotides from the nucleotide sequence of SEQ ID NO: 12, SEQ ID NO: 18, SEQ ID NO: 19, SEQ ID NO:20, or SEQ ID NO:21, or an RNA equivalent or a DNA/RNA chimeric thereof and including from 0-7 nucleotide analogs, and/or wherein the second SARS-CoV-2-specific amplification oligomer of (a)(ii) contains a nucleotide sequence differing by no more than 0, 1, 2, 3, 4, or 5 nucleotides from the nucleotide sequence of SEQ ID NO: 13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.
3. The composition or kit of paragraph 2, wherein the first SARS-CoV-2-specific target-hybridizing sequence of (a)(i) contains the nucleotide sequence of SEQ ID NO: 12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence of (a)(ii) the nucleotide sequence of SEQ ID NO: 13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.
4. The composition or kit of any one of paragraphs 1-3, wherein the first SARS-CoV-2-specific target-hybridizing sequence of (b)(i) contains a nucleotide sequence differing by no more than 0, 1, 2, 3, 4, or 5 nucleotides from the nucleotide sequence of SEQ ID NO: 15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence of (b)(ii) contains a nucleotide sequence differing by no more than 0, 1, 2, 3, 4, or 5 nucleotides from the nucleotide sequence of SEQ ID NO: 16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
5. The composition or kit of paragraph 4, wherein the first SARS-CoV-2-specific target-hybridizing sequence of (b)(i) contains the nucleotide sequence of SEQ ID NO: 15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence of (b)(ii) contains the nucleotide sequence of SEQ ID NO: 16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
6. A composition or kit for determining the presence or absence of SARS-CoV-2 in a sample, said composition or kit comprising an amplification oligomer combination comprising first and second SARS-CoV-2-specific amplification oligomers capable of amplifying a target region of a SARS-CoV-2 target nucleic acid,
   (a) wherein the first SARS-CoV-2-specific amplification oligomer comprises a target-hybridizing sequence 18 to 27 contiguous nucleotides in length, wherein the target-hybridizing sequence is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
   (b) wherein the second SARS-CoV-2-specific amplification oligomer comprises a second SARS-CoV-2-specific target-hybridizing sequence 18 to 23 contiguous nucleotides in length, wherein the target-hybridizing sequence is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88.
7. The composition or kit of paragraph 6, wherein the first SARS-CoV-2-specific target-hybridizing sequence contains a nucleotide sequence differing by no more than 0, 1, 2, 3, 4, or 5 nucleotides from the nucleotide sequence of SEQ ID NO: 12, SEQ ID NO: 18, SEQ ID NO: 19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence contains a nucleotide sequence differing by no more than 0, 1, 2, 3, 4, or 5 nucleotides from the nucleotide sequence of SEQ ID NO: 13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.
8. The composition or kit of paragraph 7, wherein the first SARS-CoV-2-specific target-hybridizing sequence contains the nucleotide sequence of SEQ ID NO: 12, SEQ ID NO: 18, SEQ ID NO: 19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence contains the nucleotide sequence of SEQ ID NO: 13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.
9. A composition or kit for determining the presence or absence of SARS-CoV-2 in a sample, said composition or kit comprising an amplification oligomer combination comprising first and second SARS-CoV-2-specific amplification oligomers capable of amplifying a target region of a SARS-CoV-2 target nucleic acid,
   (a) wherein the first SARS-CoV-2-specific amplification oligomer comprises a first SARS-CoV-2-specific target-hybridizing sequence that is from 20 to 23 contiguous nucleotides in length, wherein the target-hybridizing sequence is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
   (b) wherein the second SARS-CoV-2-specific amplification oligomer comprises a second SARS-CoV-2-specific target-hybridizing sequence 16 to 27 contiguous nucleotides in length, wherein the target-hybridizing sequence is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113.
10. The composition or kit of paragraph 9, wherein the first SARS-CoV-2-specific target-hybridizing sequence contains a nucleotide sequence differing by no more than 0, 1, 2, 3, 4, or 5 nucleotides from the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence contains a nucleotide sequence differing by no more than 0, 1, 2, 3, 4, or 5 nucleotides from the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
11. The composition or kit of paragraph 10, wherein the first SARS-CoV-2-specific target-hybridizing sequence contains the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence contains the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
12. A composition or kit comprising a SARS-CoV-2-specific-detection probe oligomer comprising a SARS-CoV-2-specific detection probe target-hybridizing sequence that (i) is from 19 to 27 contiguous nucleotides in length, is contained within SEQ ID NO:50, and contains a nucleotide sequence differing by no more than 0, 1, 2, 3, 4, or 5 nucleotides from the nucleotide sequence of SEQ ID NO:51, SEQ ID NO:52, SEQ ID NO:53, or SEQ ID NO:114; or (ii) is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO:115.
13. The composition or kit of paragraph 12, wherein the SARS-CoV-2-specific detection probe target hybridizing sequence contains a nucleotide sequence differing by no more than 0, 1, 2, 3, 4, or 5 nucleotides from the nucleotide sequence of SEQ ID NO:14, SEQ ID NO:17, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:44, SEQ ID NO:45, SEQ ID NO:46, SEQ ID NO:47, SEQ ID NO:48, SEQ ID NO:107, SEQ ID NO:108, SEQ ID NO:109 or SEQ ID NO:110, including a DNA equivalent, an RNA equivalent, or a DNA/RNA chimeric thereof and including from 0-7 nucleotide analogs.
14. The composition or kit of paragraph 13, wherein the SARS-CoV-2-specific detection probe target hybridizing sequence contains the nucleotide sequence of SEQ ID NO:14, SEQ ID NO:17, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:44, SEQ ID NO:45, SEQ ID NO:46, SEQ ID NO:47, SEQ ID NO:48, SEQ ID NO:107, SEQ ID NO:108, SEQ ID NO:109 or SEQ ID NO:110, including a DNA equivalent, an RNA equivalent, or a DNA/RNA chimeric thereof and including from 0-7 nucleotide analogs
15. A formulation for amplifying a SARS-CoV-2 nucleic acid in a sample, the formulation comprising:
   (a) a first amplification oligomer combination comprising first and second SARS-CoV-2-specific amplification oligomers capable of amplifying a target region of a SARS-CoV-2 target nucleic acid,
      (i) wherein the first SARS-CoV-2-specific amplification oligomer comprises a first SARS-CoV-2-specific target-hybridizing sequence 18 to 27 contiguous nucleotides in length, wherein the target-hybridizing sequence is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
      (ii) wherein the second SARS-CoV-2-specific amplification oligomer comprises a second SARS-CoV-2-specific target-hybridizing sequence 18 to 23 contiguous nucleotides in length, wherein the target-hybridizing sequence is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88;
   (b) a second amplification oligomer combination comprising first and second SARS-CoV-2-specific amplification oligomers capable of amplifying a target region of a SARS-CoV-2 target nucleic acid,
      (i) wherein the first SARS-CoV-2-specific amplification oligomer comprises a first SARS-CoV-2-specific target-hybridizing sequence 20 to 23 contiguous nucleotides in length, wherein the target-hybridizing sequence is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
      (ii) wherein the second SARS-CoV-2-specific amplification oligomer comprises a second SARS-CoV-2-specific target-hybridizing sequence 16 to 27 contiguous nucleotides in length, wherein the target-hybridizing sequence is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113; and
   (c) a buffer.
16. The formulation of paragraph 15, wherein the first SARS-CoV-2-specific target-hybridizing sequence of (a)(i) is SEQ ID NO: 12, SEQ ID NO: 18, SEQ ID NO: 19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence of (a)(ii) is SEQ ID NO: 13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.
17. The formulation of paragraph 15 or 16, wherein the first SARS-CoV-2-specific target-hybridizing sequence of (b)(i) is SEQ ID NO: 15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence of (b)(ii) is SEQ ID NO: 16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
18. The formulation of any one of paragraphs 15 to 17, wherein the formulation is a dried composition.
19. A formulation for amplifying a SARS-CoV-2 nucleic acid in a sample, the formulation comprising:
   (a) an amplification oligomer combination comprising first and second SARS-CoV-2-specific amplification oligomers capable of amplifying a target region of a SARS-CoV-2 target nucleic acid, wherein the first SARS-CoV-2-specific amplification oligomer comprises a first SARS-CoV-2-specific target-hybridizing sequence 18 to 27 contiguous nucleotides in length, wherein the target-hybridizing sequence is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and wherein the second SARS-CoV-2-specific amplification oligomer comprises a second SARS-CoV-2-specific target-hybridizing sequence 18 to 23 contiguous nucleotides in length, wherein the target-hybridizing sequence is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88; and
   (b) a buffer.
20. The formulation of paragraph 19, wherein the first SARS-CoV-2-specific target-hybridizing sequence is SEQ ID NO:12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence is SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.
21. The formulation of paragraph 19 or 20, wherein the formulation is a dried composition.
22. A formulation for amplifying a SARS-CoV-2 nucleic acid in a sample, the formulation comprising:
   (a) an amplification oligomer combination comprising first and second SARS-CoV-2-specific amplification oligomers capable of amplifying a target region of a SARS-CoV-2 target nucleic acid, wherein the first SARS-CoV-2-specific amplification oligomer comprises a first SARS-CoV-2-specific target-hybridizing sequence 20 to 23 contiguous nucleotides in length, wherein the target-hybridizing sequence is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and wherein the second SARS-CoV-2-specific amplification oligomer comprises a second SARS-CoV-2-specific target-hybridizing sequence 16 to 27 contiguous nucleotides in length, wherein the target-hybridizing sequence is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113; and
   (b) a buffer.
23. The formulation of paragraph 22, wherein the first SARS-CoV-2-specific target-hybridizing sequence is SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence is SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
24. The formulation of paragraph 22 or 23, wherein the formulation is a dried composition.
25. A formulation for detecting SARS-CoV-2 in a sample, the formulation comprising:
   (a) a buffer; and
   (b) a SARS-CoV-2-specific-detection probe oligomer comprising a SARS-CoV-2-specific detection probe target-hybridizing sequence that
      (i) is from 19 to 27 contiguous nucleotides in length, is contained within SEQ ID NO:50, and contains a nucleotide sequence differing by no more than 0, 1, 2, 3, 4, or 5 nucleotides from the nucleotide sequence of SEQ ID NO:51, SEQ ID NO:52, SEQ ID NO:53, or SEQ ID NO:114; or
      (ii) is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO:115.
26. The formulation of paragraph 25, wherein the SARS-CoV-2-specific detection probe target hybridizing sequence is selected from the group consisting of: SEQ ID NO:14, SEQ ID NO:17, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:44, SEQ ID NO:45, SEQ ID NO:46, SEQ ID NO:47, SEQ ID NO:48, SEQ ID NO:107, SEQ ID NO:108, SEQ ID NO:109 and SEQ ID NO:110, including a DNA equivalent, an RNA equivalent, or a DNA/RNA chimeric thereof and including from 0-7 nucleotide analogs.
27. The formulation of paragraph 25 or 26, wherein the formulation is a dried composition.
28. A kit comprising a dried composition as in any one of paragraphs 18, 21, 24, and 27.
29. The kit of paragraph 28, further comprising a reconstitution reagent, wherein the dried composition is in a first vial within the kit and the reconstitution reagent is in a second vial within the kit.
30. A method for preparing an aqueous reaction mixture for determining the presence or absence of a SARS-CoV-2 in a sample, the method comprising the step of combining a dried composition as described in any one of embodiments 18, 21, 24, and 27 with a reconstitution reagent to make an aqueous reaction mixture.
31. A method for determining the presence or absence of SARS-CoV-2 in a sample, the method comprising:
   (a) contacting a sample with at least a first amplification oligomer combination and a second amplification oligomer combination, wherein;
      (i) the first amplification oligomer combination comprises first and second SARS-CoV-2-specific amplification oligomers capable of amplifying a first target region of a SARS-CoV-2 target nucleic acid,
         (1) wherein the first SARS-CoV-2-specific amplification oligomer comprises a first SARS-CoV-2-specific target-hybridizing sequence 18 to 27 contiguous nucleotides in length, wherein the target-hybridizing sequence is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
         (2) wherein the second SARS-CoV-2-specific amplification oligomer comprises a second SARS-CoV-2-specific target-hybridizing sequence 18 to 23 contiguous nucleotides in length, wherein the target-hybridizing sequence is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88; and
      (ii) a second amplification oligomer combination comprising first and second SARS-CoV-2-specific amplification oligomers capable of amplifying a second target region of a SARS-CoV-2 target nucleic acid,
         (1) wherein the first SARS-CoV-2-specific amplification oligomer comprises a first SARS-CoV-2-specific target-hybridizing sequence 20 to 23 contiguous nucleotides in length, wherein the target-hybridizing sequence is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
         (2) wherein the second SARS-CoV-2-specific amplification oligomer comprises a second SARS-CoV-2-specific target-hybridizing sequence 16 to 27 contiguous nucleotides in length, wherein the target-hybridizing sequence is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113;
   (b) incubating the sample in conditions suitable for *in vitro* nucleic acid amplification, wherein any SARS-CoV-2 target nucleic acid, if present in the sample, is used as a template for generating one or more amplicons corresponding to at least one of the first or second SARS-CoV-2 target regions; and
   (c) detecting the presence or absence of the one or more amplicons, thereby determining the presence or absence of SARS-CoV-2 in the sample.
32. The method of paragraph 31, wherein the first SARS-CoV-2-specific target-hybridizing sequence of (a)(i) contains a nucleotide sequence differing by no more than 0, 1, 2, 3, 4, or 5 nucleotides from the nucleotide sequence of SEQ ID NO:12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence of (a)(ii) contains a nucleotide sequence differing by no more than 0, 1, 2, 3, 4, or 5 nucleotides from the nucleotide sequence of SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.
33. The method of paragraph 32, wherein the first SARS-CoV-2-specific target-hybridizing sequence of (a)(i) contains the nucleotide sequence of SEQ ID NO:12, SEQ ID NO:18, SEQ ID NO:19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence of (a)(ii) contains the nucleotide sequence of SEQ ID NO:13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.
34. The method of any one of paragraphs 31 to 33, wherein the first SARS-CoV-2-specific target-hybridizing sequence of (b)(i) contains a nucleotide sequence differing by no more than 0, 1, 2, 3, 4, or 5 nucleotides from the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence of (b)(ii) contains a nucleotide sequence differing by no more than 0, 1, 2, 3, 4, or 5 nucleotides from the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
35. The method of paragraph 34, wherein the first SARS-CoV-2-specific target-hybridizing sequence of (b)(i) contains the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence of (b)(ii) contains the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
36. A method for determining the presence or absence of SARS-CoV-2 in a sample, the method comprising:
   (a) contacting a sample with an amplification oligomer combination comprising first and second SARS-CoV-2-specific amplification oligomers capable of amplifying a target region of a SARS-CoV-2 target nucleic acid,
      (i) wherein the first SARS-CoV-2-specific amplification oligomer comprises a first SARS-CoV-2-specific target-hybridizing sequence 18 to 27 contiguous nucleotides in length, wherein the target-hybridizing sequence is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
      (ii) wherein the second SARS-CoV-2-specific amplification oligomer comprises a second SARS-CoV-2-specific target-hybridizing sequence 18 to 23 contiguous nucleotides in length, wherein the target-hybridizing sequence is contained within SEQ ID NO:85, and contains a nucleotide sequence differing by no more than 0, 1, 2, 3, 4, or 5 nucleotides from the nucleotide sequence of SEQ ID NO:86, SEQ ID NO:87 or SEQ ID NO:88;
   (b) incubating the sample in conditions suitable for *in vitro* nucleic acid amplification, wherein any SARS-CoV-2 target nucleic acid, if present in the sample, is used as a template for generating an amplicon corresponding to the SARS-CoV-2 target region; and
   (c) detecting the presence or absence of the amplicon, thereby determining the presence or absence of SARS-CoV-2 in the sample.
37. The method of paragraph 36, wherein the first SARS-CoV-2-specific target-hybridizing sequence contains a nucleotide sequence differing by no more than 0, 1, 2, 3, 4, or 5 nucleotides from the nucleotide sequence of SEQ ID NO: 12, SEQ ID NO: 18, SEQ ID NO: 19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence contains a nucleotide sequence differing by no more than 0, 1, 2, 3, 4, or 5 nucleotides from the nucleotide sequence of SEQ ID NO: 13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs.
38. The method of paragraph 37, wherein the first SARS-CoV-2-specific target-hybridizing sequence contains the nucleotide sequence of SEQ ID NO: 12, SEQ ID NO: 18, SEQ ID NO: 19, SEQ ID NO:20, or SEQ ID NO:21, or an RNA equivalent or a DNA/RNA chimeric thereof and including from 0-7 nucleotide analogs, and/or wherein the second SARS-CoV-2-specific target-hybridizing sequence contains the nucleotide sequence of SEQ ID NO: 13, SEQ ID NO:71, SEQ ID NO:72, SEQ ID NO:73, SEQ ID NO:74, or SEQ ID NO:75, or an RNA equivalent or a DNA/RNA chimeric thereof and including from 0-7 nucleotide analogs
39. A method for determining the presence or absence of SARS-CoV-2 in a sample, the method comprising:
   (a) contacting a sample with an amplification oligomer combination comprising first and second SARS-CoV-2-specific amplification oligomers capable of amplifying a target region of a SARS-CoV-2 target nucleic acid,
      (i) wherein the first SARS-CoV-2-specific amplification oligomer comprises a first SARS-CoV-2-specific target-hybridizing sequence 20 to 23 contiguous nucleotides in length, wherein the target-hybridizing sequence is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
      (ii) wherein the second SARS-CoV-2-specific amplification oligomer comprises a second SARS-CoV-2-specific target-hybridizing sequence 16 to 27 contiguous nucleotides in length, wherein the target-hybridizing sequence is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113;
   (b) incubating the sample in conditions suitable for *in vitro* nucleic acid amplification, wherein any SARS-CoV-2 target nucleic acid, if present in the sample, is used as a template for generating an amplicon corresponding to the SARS-CoV-2 target region; and
   (c) detecting the presence or absence of the amplicon, thereby determining the presence or absence of SARS-CoV-2 in the sample.
40. The method of paragraph 39, wherein the first SARS-CoV-2-specific target-hybridizing sequence contains a nucleotide sequence differing by no more than 0, 1, 2, 3, 4, or 5 nucleotides from the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence contains a nucleotide sequence differing by no more than 0, 1, 2, 3, 4, or 5 nucleotides from the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
41. The method of paragraph 40, wherein the first SARS-CoV-2-specific target-hybridizing sequence contains the nucleotide sequence of SEQ ID NO:15, SEQ ID NO:22, SEQ ID NO:23, SEQ ID NO:24 or SEQ ID NO:25, or an RNA equivalent or a DNA/RNA chimeric thereof and including from 0-7 nucleotide analogs, and/or wherein the second SARS-CoV-2-specific target-hybridizing sequence contains the nucleotide sequence of SEQ ID NO:16, SEQ ID NO:76, SEQ ID NO:77, SEQ ID NO:79, SEQ ID NO:80, SEQ ID NO:81, SEQ ID NO:82, SEQ ID NO:83 or SEQ ID NO:84, or an RNA equivalent or a DNA/RNA chimeric thereof and including from 0-7 nucleotide analogs.
42. A multiplex method for determining the presence or absence of SARS-CoV-2 and at least one other pathogen in a sample, wherein the presence or absence of SARS-CoV-2 is determined using the method of any one of paragraphs 31 to 38.
43. The multiplex method of paragraph 42, wherein the method determines the presence or absence of SARS-CoV-2 and one or more pathogens selected from the group consisting of influenza A, influenza B, respiratory syncytial virus A, respiratory syncytial virus B, parainfluenza virus 1, parainfluenza virus 2, parainfluenza virus 3, parainfluenza virus 4, adenovirus, metapneumovirus, rhino virus, coronavirus 229E, coronavirus NL63, coronavirus HKU1, coronavirus OC43, SARS-CoV (SARS), and MERS-CoV.
44. The method of any one of paragraphs 31-41, wherein detecting the presence or absence of the amplicon comprises contacting the sample with a SARS-CoV-2-specific-detection probe oligomer comprising a SARS-CoV-2-specific detection probe target-hybridizing sequence that (i) is from 19 to 27 contiguous nucleotides in length, is contained within SEQ ID NO:50, and contains a nucleotide sequence differing by no more than 0, 1, 2, 3, 4, or 5 nucleotides from the nucleotide sequence of SEQ ID NO:51, SEQ ID NO:52, SEQ ID NO:53, or SEQ ID NO:114; or (ii) is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO:115.

## Claims

1. A composition or kit for determining the presence or absence of SARS-CoV-2 in a sample, said composition or kit comprising:
(a) a first amplification oligomer combination comprising first and second SARS-CoV-2 region 1-specific amplification oligomers capable of amplifying a first target region of a SARS-CoV-2 target nucleic acid,
(i) wherein the first SARS-CoV-2 region 1-specific amplification oligomer comprises a first SARS-CoV-2 region 1-specific target-hybridizing sequence that is from 18 to 27 contiguous nucleotides in length, is contained within SEQ ID NO:26, and contains a nucleotide sequence differing by no more than 0, 1, 2, 3, 4, or 5 nucleotides from the nucleotide sequence of SEQ ID NO:27 or SEQ ID NO:28, and
(ii) wherein the second SARS-CoV-2 region 1-specific amplification oligomer comprises a second SARS-CoV-2 region 1-specific target-hybridizing sequence that is from 18 to 23 contiguous nucleotides in length, is contained within SEQ ID NO:85, and contains a nucleotide sequence differing by no more than 0, 1, 2, 3, 4, or 5 nucleotides from the nucleotide sequence of SEQ ID NO:86, SEQ ID NO:87, or SEQ ID NO:88, or
(b) a second amplification oligomer combination comprising first and second SARS-CoV-2 region 2-specific amplification oligomers capable of amplifying a second target region of a SARS-CoV-2 target nucleic acid,
(i) wherein the first SARS-CoV-2 region 2-specific amplification oligomer comprises a first SARS-CoV-2 region 2-specific target-hybridizing sequence that is from 20 to 23 contiguous nucleotides in length, is contained within SEQ ID NO:29, and contains a nucleotide sequence differing by no more than 0, 1, 2, 3, 4, or 5 nucleotides from the nucleotide sequence of SEQ ID NO:30, SEQ ID NO:31, or SEQ ID NO:111, and
(ii) wherein the second SARS-CoV-2 region 2-specific amplification oligomer comprises a second SARS-CoV-2 region 2-specific target-hybridizing sequence that is from 16 to 27 contiguous nucleotides in length, is contained within SEQ ID NO:89, and contains a nucleotide sequence differing by no more than 0, 1, 2, 3, 4, or 5 nucleotides from the nucleotide sequence of SEQ ID NO:90, SEQ ID NO:112, or SEQ ID NO:113.

2. The composition or kit according to claim 1, further comprising:
(i) a SARS-CoV-2 region 1-specific detection probe oligomer, wherein the detection probe oligomer comprises a SARS-CoV-2 region 1-specific detection probe target-hybridizing sequence that is from 19 to 27 contiguous nucleotides in length, is contained within SEQ ID NO:50, and contains a nucleotide sequence differing by no more than 0, 1, 2, 3, 4, or 5 nucleotides from the nucleotide sequence of SEQ ID NO:51, SEQ ID NO:52, SEQ ID NO:54, or SEQ ID NO: 114; or
(ii) a SARS-CoV-2 region 2-specific detection probe oligomer, wherein the detection probe oligomer comprises a SARS-CoV-2 region 2-specific detection probe target-hybridizing sequence that is from 25 to 29 contiguous nucleotides in length, is contained within SEQ ID NO:54, and contains a nucleotide sequence differing by no more than 0, 1, 2, 3, 4, or 5 nucleotides from the nucleotide sequence of SEQ ID NO:55, SEQ ID NO:56, or SEQ ID NO: 115.

3. The composition or kit of any one of claims 1 to 2, wherein the second SARS-CoV-2 region 1-specific target-hybridizing sequence and the second SARS-CoV-2 region 2-specific target-hybridizing sequence of (a)(ii) or (b)(ii) is joined at its 5' end to a promoter sequence.

4. The composition or kit of claim 3, wherein the promoter sequence is substantially identical to SEQ ID NO: 11.

5. The composition or kit of any one of claims 2 to 4, wherein the first and/or second SARS-CoV-2 region 1-specific detection probe oligomer and the SARS-CoV-2 region 2-specific detection probe oligomer further comprises a detectable label.

6. The composition or kit of claim 5, wherein the detectable label is a fluorescent or chemiluminescent label.

7. The composition or kit of claim 5, wherein the detectable label is a fluorescent label and the first and/or second SARS-CoV-2 region 1-specific detection probe oligomer and the SARS-CoV-2 region 2-specific detection probe oligomer further comprises a non-fluorescent quencher.

8. The composition or kit of any one of claims 2 to 7, wherein the first and/or second SARS-CoV-2 region 1-specific detection probe oligomer and the SARS-CoV-2 region 2-specific detection probe oligomer further comprises a self-complementary sequence joined to the SARS-CoV-2 region 1-specific detection probe target-hybridizing sequence and the SARS-CoV-2 region 2-specific detection probe target-hybridizing sequence, optionally wherein the self-complementary sequence is joined to the 5' end of the SARS-CoV-2 region 1-specific detection probe target-hybridizing sequence and the SARS-CoV-2 region 2-specific detection probe target-hybridizing sequence.

9. The composition or kit of claim 8, wherein the self-complementary sequence is joined to the SARS-CoV-2 region 1-specific detection probe target-hybridizing sequence and the SARS-CoV-2 region 2-specific detection probe target-hybridizing sequence via a non-nucleotide linker.

10. The composition or kit of any one of claims 2 to 9, wherein the first and/or second SARS-CoV-2 region 1-specific detection probe oligomer and the SARS-CoV-2 region 2-specific detection probe oligomer is a molecular beacon or a molecular torch.

11. The composition or kit of claim 1, further comprising a target capture oligonucleotide comprising: (i) a target hybridizing sequence containing or consisting of a nucleotide sequence differing by no more than 0, 1, 2, 3, 4, or 5 nucleotides from the nucleotide sequence of SEQ ID NO:97, SEQ ID NO:98, SEQ ID NO:99, SEQ ID NO:100, SEQ ID NO:101, or SEQ ID NO:102, including a DNA equivalent, an RNA equivalent, or a DNA/RNA chimeric thereof and including from 0-24 nucleotide analogs, and (ii) a solid support binding region that is d(T)_{0-3d}(A)₁₄₋₃₀.

12. The composition or kit of claim 11, wherein the target capture oligonucleotide comprises or consists of a nucleotide sequence differing by no more than 0, 1, 2, 3, 4, or 5 nucleotides from the nucleotide sequence of SEQ ID NO:91, SEQ ID NO:92, SEQ ID NO:93, SEQ ID NO:94, SEQ ID NO:95, or SEQ ID NO:96.

13. A method for determining the presence or absence of SARS-CoV-2 in a sample, the method comprising:
(a) contacting a sample with at least a first amplification oligomer combination and a second amplification oligomer combination according to claim 1;
(b) incubating the sample in conditions suitable for *in vitro* nucleic acid amplification, wherein any SARS-CoV-2 target nucleic acid, if present in the sample, is used as a template for generating one or more amplicons corresponding to at least one of the first or second SARS-CoV-2 target regions; and
(c) detecting the presence or absence of the one or more amplicons, thereby determining the presence or absence of SARS-CoV-2 in the sample.

14. A multiplex method for determining the presence or absence of SARS-CoV-2 and at least one other pathogen in a sample, wherein the presence or absence of SARS-CoV-2 is determined using the method of claim 13.

15. The multiplex method of claim 14, wherein the method determines the presence or absence of SARS-CoV-2 and one or more pathogens selected from the group consisting of influenza A, influenza B, respiratory syncytial virus A, respiratory syncytial virus B, parainfluenza virus 1, parainfluenza virus 2, parainfluenza virus 3, parainfluenza virus 4, adenovirus, metapneumovirus, rhino virus, coronavirus 229E, coronavirus NL63, coronavirus HKU1, coronavirus OC43, SARS-CoV (SARS), and MERS-CoV.
